# EUROPEAN PATENT SPECIFICATION

(11) **EP 3 927 817 B1**
(45) Date of publication and mention of the grant of the patent: **20.08.2025**
(21) Application number: 20704547.7
(22) Date of filing: 18.02.2020
(51) Int. Cl.: C12N 9/06, A61K 38/44, C12N 15/53

(54) **NEW RECOMBINANT DIAMINE OXIDASE AND ITS USE FOR THE TREATMENT OF DISEASES CHARACTERIZED BY EXCESS HISTAMINE**
NEUE REKOMBINANTE DIAMINOXIDASE UND DEREN VERWENDUNG ZUR BEHANDLUNG VON DURCH ÜBERSCHÜSSIGES HISTAMIN CHARAKTERISIERTEN KRANKHEITEN
NOUVEL OXYDASE DIAMINE RECOMBINANT ET SON UTILISATION POUR LE TRAITEMENT DE MALADIES CARACTÉRISÉES PAR UN EXCÈS D'HISTAMINE

(30) Priority: 19.02.2019 EP 19157932
(43) Date of publication of application: 29.12.2021
(73) Proprietor: Medizinische Universität Wien, 1090 Wien (AT); Universität für Bodenkultur Wien, 1180 Wien (AT)
(72) Inventor: BÖHM, Thomas, 1130 Wien (AT); JILMA, Bernd, 1090 Wien (AT); BORTH, Nicole, 1180 Wien (AT); GLUDOVACZ, Elisabeth, 1200 Wien (AT)
(74) Representative: Loidl, Manuela Bettina
(86) International application number: PCT/EP2020/054197
(87) International publication number: WO 2020/169577

(56) References cited:
- WO-A1-2012/028891
- WO-A2-02/43745
- AARON P. MCGRATH ET AL: "Structure and Inhibition of Human Diamine Oxidase", BIOCHEMISTRY, vol. 48, no. 41, 20 October 2009 (2009-10-20), pages 9810 - 9822, XP055596327, ISSN: 0006-2960, DOI: 10.1021/bi9014192
- DU-CUNY L ET AL: "Computational modeling of novel inhibitors targeting the Akt pleckstrin homology domain", BIOORGANIC & MEDICINAL CHEMISTRY, vol. 17, no. 19, 1 October 2009 (2009-10-01), pages 6983 - 6992, XP026601546, ISSN: 0968-0896, [retrieved on 20090819], DOI: 10.1016/J.BMC.2009.08.022
- BRADLEY O. ELMORE ET AL: "Human kidney diamine oxidase: heterologous expression, purification, and characterization", JBIC, JOURNAL OF BIOLOGICAL AND INORGANIC CHEMISTRY, vol. 7, no. 6, 13 February 2002 (2002-02-13), pages 565 - 579, XP055106416, ISSN: 0949-8257, DOI: 10.1007/s00775-001-0331-1
- NOVOTNY W F ET AL: "Diamine oxidase is the amiloride-binding protein and is inhibited by amiloride analogues", JOURNAL OF BIOLOGICAL CHEMISTRY, AMERICAN SOCIETY FOR BIOCHEMISTRY AND MOLECULAR BIOLOGY, US, vol. 269, no. 13, 1 January 1994 (1994-01-01), pages 9921 - 9925, XP002194296, ISSN: 0021-9258
- DATABASE UniProt [online] 16 November 2011 (2011-11-16), "RecName: Full=Amine oxidase {ECO:0000256|RuleBase:RU000672}; EC=1.4.3.- {ECO:0000256|RuleBase:RU000672};", XP002792064, retrieved from EBI accession no. UNIPROT:G3QJ02 Database accession no. G3QJ02
- DATABASE UniProt [online] 1 May 2000 (2000-05-01), "RecName: Full=Amine oxidase {ECO:0000256|RuleBase:RU000672}; EC=1.4.3.- {ECO:0000256|RuleBase:RU000672};", XP002792065, retrieved from EBI accession no. UNIPROT:Q9SXW5 Database accession no. Q9SXW5

## Description

### FIELD OF THE INVENTION

The present invention refers to a recombinant diamine oxidase (DAO) variant comprising any one of SEQ ID NOs:2 to 8 or a sequence having at least 99% sequence identity with any one of SEQ ID NOs:2 to 8, with decreased glycosaminoglycan binding affinity compared to the respective wild type human DAO, wherein said DAO comprises at least one amino acid modification of any one of amino acids at positions 568-575 of the glycosaminoglycan (GAG) binding domain with reference to the numbering of SEQ ID NO:1.

The present invention also refers to the use of the DAO variant in the treatment of a condition associated with excess histamine, specifically in the treatment of chronic allergic diseases and/or in the treatment of high risk pregnancy, more specifically in the treatment of anaphylaxis, anaphylactic shock, chronic urticaria, acute urticaria, asthma, hay fever, allergic rhinitis, allergic conjunctivitis, histamine intoxication, headache, atopic dermatitis inflammatory diseases, mastocytosis, mast cell activation syndrome (MCAS), pre-eclampsia, hyperemesis gravidarum, pre-term labor, peptic ulcers, acid reflux, pruritus, and sepsis.

### BACKGROUND OF THE INVENTION

Histamine (2-(1*H*-Imidazol-4-yl)ethanamine) is an organic compound involved in local immune responses, regulating physiological function in the gut and acting as a neurotransmitter for the brain, spinal cord, and uterus. Histamine is involved in the inflammatory response and has a central role as a mediator of itching. As part of an immune response to foreign pathogens, histamine is produced by basophils and by mast cells found in nearby connective tissues. It is stored in inactive form in the metachromatic granules of the mast cells and basophilic leukocytes, where it is available for an immediate release. Histamine can also be freshly synthesized by neutrophils and macrophages during inflammation. After its release, histamine is a powerful physiological and pathological mediator binding to four receptors (H₁ - H₄) expressed on many different cells in the body. Binding of histamine to its receptors is very specific. After binding many downstream activities are induced. Acute allergic reactions such as hay fever, runny nose, itchy eyes or in more severe cases asthma with breathing problems; acute and chronic urticaria and hypersensitivity reactions, also called anaphylaxis for example caused by medications especially antibiotics or radio contrast agents, by a peanut or wasp allergy etc., are mediated by tissue mast cells and basophils in the blood releasing several mediators of which histamine is one of the most active ones. Histamine-induced symptoms include anaphylaxis, a hypersensitivity reaction with drop in blood pressure, fainting and breathing problems among other symptoms, bronchospasm, flushing (reddening of the skin), pruritus (itchiness), tachycardia (high pulse rate), syncope (fainting), hypotension (low blood pressure), epigastric and abdominal pain, nausea, vomiting, diarrhea, fatigue, memory loss, depression and headache among others. Excess histamine (>10 ng/ml; symptoms start at 1 to 3 ng/ml) in the blood is life-threatening. Histamine concentration in blood of approximately 100 ng/ml can result in cardiac arrest. Specifically during pregnancy, hyperhistaminemia can lead to specific gestational complications such as preeclampsia, spontaneous abortion, preterm labour and hyperemesis gravidarum (Brew O. and Sullivan M.H.F., J.Reprod.lmmunol., 72(2006), 94-107, Maintz L. et al., Human Reproduction Update, 14,5, 2008, 485-495).

Mast cells and basophils are cells of the immune system with many different functions. Nevertheless, mast cells and histamine play an important role in many diseases like mast cell activation syndromes, MCAS, i.e. inability to tolerate histamine in red wine or cheese or other foods; in literature often described as histamine intolerance, atopic dermatitis (also called neurodermitis, a skin disease), mastocytosis (increased number of mast cells in the skin and/or internal organs like the bone marrow or liver or spleen), peptic ulcers (damage of the mucosa in the stomach or duodenum caused by excessive histamine followed by acid release), acid reflux, headache, pruritus and possibly even sepsis among other diseases. Histamine can be also newly synthesized by histidine decarboxylase induced in for example neutrophils and macrophages under certain disease conditions.

Moreover, histamine may also get into the body from the outside, by inhaling, or orally, e.g. by ingesting histamine-containing foodstuffs, such as cheese, wine, canned fish and sauerkraut. Histamine can also be produced by bacteria of the microbiome.

Anti-histamines opposing the activity of histamine receptors H₁ and H₂ have been available for several decades and they are assumed to be efficacious in treating symptoms such as runny nose and itching skin and eyes. Anti-histamines are one of the most frequently prescribed medications worldwide. Nevertheless, thorough data analysis has clearly shown that the efficacy of anti-histamines is limited under several pathological conditions. For example, about 25% of chronic urticaria patients are antihistamine resistant and suffer from a low quality of life. Treatment of hypersensitivity reactions with anti-histamines is widely practiced but high quality efficacy data are missing and in some documents a lack of efficacy is stated. Anaphylaxis guidelines do not necessarily recommend the use of histamine H₁ receptor blocker for the treatment because the evidence of benefit is not clear. The use of histamine H₂ receptors antagonists in anaphylaxis is discouraged because it can worsen the symptoms.

This limited efficacy of anti-histamines is not surprising. Several studies have shown that anti-histamines can only block the effects of 2- to 3-fold increased histamine concentrations, but are much less efficacious when histamine concentrations increase beyond that. During anaphylaxis or hypersensitivity reactions histamine concentrations in the circulation can be increased more than 100-fold in normal people and even more in mastocytosis patients. Mastocytosis patients have continuously 5- to 15-fold increased levels of histamine and its metabolites under steady-state, non-anaphylactic conditions. The body cannot degrade the excessive histamine fast enough and the development of various symptoms is a logical consequence.

In the mammalian organism, histamine is degraded by two enzymes: diamine oxidase (diamine oxidase, DAO, EC 1.4.3.6) and histamine-N-methyltransferase (HNMT or short NMT, EC 2.1.1.8). NMT catalyzes the N-methylation to N-methyl-histamine.

The structure and inhibition of human DAO is reviewed in McGrath AP et al. (Biochemistry, 2009, 48(41), 9810-9822). DAO catalyzes the oxidative deamination of histamine to imidazole acetaldehyde. DAO was originally identified as the enzyme that cleared exogenous histamine from minced lung and liver samples and was therefore termed histaminase (Best CH., J. Physiol., 1929, 67, 256-263). Subsequently, a protein identified as diamine oxidase was termed amiloride-binding protein, and was incorrectly implicated with the amiloride-sensitive Na⁺ channel. Some databases still designate AOC1 as ABP1. That DAO and ABP1 were in fact the same protein was later noted by the original authors (Novotny WF et al., J.Biol.Chem., 1994, 269, 9921-9925) who also reported the cloning of the human gene, corresponding to a 751 amino acid residues protein. Over-expression of recombinant human DAO (hDAO) has been achieved in insect cells (Elmore BO. et al., J.Biol.Inorg.Chem., 2002, 7, 565-579). Whilst it is further reported by Elmore et al. that DAO contains a heparin binding consensus sequence (residues 568-575), it could not be derived from this structure that the heparin-binding domain really binds heparin. A 12-mer heparin molecule is ~5 nm (50 Å) long and the diameter of the circular heparin binding domain in DAO is about 25 Å. The molecular weight of a heparin 12-mer = 284 * 12 = 3408 Da. LMWH does not strongly bind to DAO but has an average molecular weight of 5000 Da (18 sugar units). Only HMWH binds to DAO with an average molecular weight of 15000 Da but this corresponds to 15000/284 = 52 sugar units in a row.

The availability of large amounts of recombinant hDAO enabled the identification of its preferred substrates in vitro, showing a distinct preference for diamines. In particular two atypical diamines, histamine and 1-methyl histamine are excellent substrates. Each contains an imidazole group in place of one of the primary amines present in typical diamine substrates like spermidine, putrescine or cadaverine. hDAO is the frontline enzyme for degradation of exogenous histamine and reduced levels of DAO have been shown to be directly correlated with histamine intolerance (Maintz L. et al., Am.J.Clin.Nutr., 2007, 85, 1185-1196). Reduced DAO activities have been found in multiple heterogenous complications of pregnancy such as diabetes, threatened and missed abortion and trophoblastic disorders (Maintz L., et al., 2008).

In the gastro-intestinal tract DAO degrades histamine from the diet to avoid and to protect the body from increased concentrations in the blood. Nevertheless, except during pregnancy, DAO antigen and consequently activity is low or even absent in plasma (Boehm T. et al., Clinical Biochemistry 50, 2017, 444-451). During pregnancy DAO activity is increased more than 100-fold.

In WO 02/43745 the systemic use of DAO of plant origin for the treatment of histamine-mediate diseases is disclosed. The administration of enzymes directly isolated from plants, however, is a great problem because of the frequent occurrence of allergens in plants, primarily in view of the fact that the leguminous plants disclosed in WO 02/43745 have a high allergenic potential.

WO2006/003213A1 describes specific administration formulations of animal derived or recombinantly produced DAO.

The expression and purification of recombinant wild-type (wt) human (rh) DAO in CHO cells was published by Gludovacz E. et al. (J Biotechnol. 2016, 227:120-130). Nevertheless, in rats the alpha-distribution half-life of wt rhDAO was less than 10 minutes and comprised more than 80% of the injected protein amount. The beta-elimination half-life was about 3 hours. The scaling factor for biologics to extrapolate from rat to human is about 4 to 8 and therefore the PK profile of recombinant wt DAO is not suitable for preclinical and clinical development. Three glycosylation sites of DAO had been mutated, but glycan mutations did not improve the PK profile. The effects of glycan mutations on expression and activity of DAO have been published by Gludovacz E. et al. (J.Biol.Chem. 2018, 293(3), 1070-1087).

DATABASE UniProt Accession No. G3QJ02, 2011, XP-002792064 discloses diamine oxidase sequence from Gorilla gorilla.

DATABASE UniProt Accession No. Q9SXW5, 2000 discloses diamine oxidase sequence from Pisum sativum.

WO 2012/028891A1 reports histaminase from vegetable origin. Because released histamine cannot be blocked or inactivated efficiently by anti-histamines, new treatment and prophylaxis approaches for rapid inactivation of excess histamine would be of significant benefit to patients suffering from high circulatory histamine concentrations. Many patients suffer for hours from increased histamine concentrations. The half-life of histamine is even increased during anaphylaxis and hypotension due to reduced kidney filtration and blood flow.

Thus there is a high and unmet demand for improved treatment of conditions associated with excess histamine.

### SUMMARY OF THE INVENTION

Under pathological conditions excess histamine cannot be efficiently antagonized by currently available anti-histamines. The increased histamine levels cause annoying, severe, debilitating, life-threatening symptoms and occasionally death.

It is an objective of the present invention to provide an improved regimen for removal of excess histamine and treatment and prevention of histamine-induced diseases and conditions.

The objective is solved by the present invention by the provision of a recombinant modified DAO to increase the concentration of active DAO within the body of an individual to thereby assist in, or enable, respectively, the degradation of histamine.

Administration of recombinant human DAO as described herein can degrade excess histamine under acute, subacute, subchronic, chronic and prophylactic conditions, thereby significantly benefiting patients suffering or going to suffer from several diseases, where anti-histamines are not efficacious enough to degrade the excess histamine concentrations. This establishes an entirely new treatment option for subjects suffering from excess histamine.

According to the invention, a recombinant human diamine oxidase (DAO) variant comprising any one of SEQ ID NOs:2 to 8 or a sequence having at least 99% sequence identity with any one of SEQ ID NOs:2 to 8, with decreased glycosaminoglycan (GAG) binding affinity compared to the GAG binding affinity of the respective wild type human DAO is provided, wherein said DAO comprises at least one amino acid modification of any one of amino acids at positions 568-575 of the glycosaminoglycan (GAG) binding domain with reference to the numbering of SEQ ID NO:1.

Specifically, the GAG binding domain is a heparin/heparan sulfate binding domain.

Specifically, the amino acid modification results in decreased GAG binding affinity of the DAO variant while its enzymatic activity towards histamine is preserved.

According to a specific embodiment, the at least one amino acid modification in the GAG binding domain of the DAO variant is an amino acid substitution, deletion, insertion or coupling with a chemical moiety.

According to a specific embodiment of the invention, the recombinant DAO variant of the invention comprises 2, 3, 4, 5, 6, 7, or 8 amino acid modifications in the GAG binding domain. Specifically, said amino acid residues are substituted by other amino acid residues, specifically arginine or lysine is substituted by serine or threonine.

According to an alternative embodiment, the recombinant DAO variant comprises a GAG binding domain of amino acid sequence X₁FX₂X₃X₄LPX₅, wherein
X₁ can be by any amino acid, specifically it is A or S, more specifically it is S;
X₂ can be by any amino acid, specifically it is K;
X₃ can be by any amino acid, specifically it is A or T, more specifically it is T;
X₄ can be by any amino acid, specifically it is K; and
X₅ can be by any amino acid, specifically it is K or T, more specifically it is T.

In a further specific embodiment, the recombinant DAO variant comprises the amino acid sequence selected from the group consisting of SFKAKLPK (SEQ ID NO:33), AFKAKLPT (SEQ ID NO:34), AFKTKLPK (SEQ ID NO:35), SFKTKLPK (SEQ ID NO:36), AFKTKLPT (SEQ ID NO:37), SFKAKLPK (SEQ ID NO:38).

According to a specific embodiment, the recombinant DAO variant as disclosed herein, further comprises at least one modification of the solvent accessible cysteine at amino acid position 123 (cys123) with reference to the numbering of SEQ ID NO:1, specifically the modification of the cysteine is an amino acid substitution, deletion or conjugation with a chemical moiety.

In a preferred embodiment, cys123, according to the numbering of SEQ ID NO:1 of DAO, is substituted by alanine (cys123ala, C123A).

The DAO variant of the invention specifically shows reduced clearance from plasma. The invention specifically provides a recombinant DAO variant which has significantly increased plasma half-life compared to wild type DAO, specifically said half-life is increased at least 1.5 fold, specifically at least 2 fold compared to wild type DAO.

In an alternative or further embodiment, the DAO variant has an at least 10-fold increased AUC compared to wild type DAO.

According to an embodiment provided herein, internalization of the recombinant DAO variant by endothelial cells is at least 10%, 25%, 50%, 60%, 70%, 80%, specifically 90% reduced compared to wild type DAO.

According to a further embodiment, the GAG binding affinity of the DAO variant as described herein, specifically the heparin/heparan sulfate binding affinity is at least 10%, 25%, 50%, 60%, 70%, 80%, specifically 90% reduced compared to wild type DAO.

Herein provided is also a recombinant DAO variant or functional derivative or analogue thereof comprising the amino acid sequences of SEQ ID NOs:9, 10, 11, 12, 13, 14, 15 or 16 or having at least 90% sequence identity with any one of SEQ ID NOs: 9, 10, 11, 12, 13, 14, 15 or 16.

Human DAO also has multiple N-glycosylation sites playing a role in secretion and retention in the endoplasmatic reticulum. Specifically, glycans at Asn-168 are predominantly sialylated with bi- to tetra-antennary branches.

According to an embodiment, the recombinant DAO variant described herein further comprises an amino acid substitution at position 168 with reference to SEQ ID NO:1. Specifically, the modification is a single modification at position 168. More specifically, Asn is replaced by Gln. Specifically, said modification increases the PK of the DAO variant described herein. More specifically, the DAO variant comprises the amino acid sequence of SEQ ID NO:106.

Herein provided is also a recombinant DAO variant or a functional derivative or analogue thereof encoded by any one of SEQ ID NOs:18, 19, 20, 21, 22, 23, 24, 25, 26, 27, 28, 29, 30, 31 or 32 or having at least 90% sequence identity with any one of SEQ ID NOs: 18, 19, 20, 21, 22, 23, 24, 25, 26, 27, 28, 29, 30, 31 or 32.

Herein provided is also an isolated nucleotide sequence encoding the DAO variant or a functional analogue or derivative thereof as described herein, specifically comprising sequences SEQ ID NOs:18, 19, 20, 21, 22, 23, 24, 25, 26, 27, 28, 29, 30, 31 or 32 or fragments thereof.

According to a specific embodiment of the invention, herein provided is a fusion polypeptide comprising the recombinant DAO variant described herein and an Fc domain of human IgG or human serum albumin (HSA) or a fragment thereof, wherein the fusion polypeptide retains the functional activity of the recombinant DAO variant.

Herein described is also a fusion polypeptide comprising the recombinant DAO variant described herein and an Fc domain of human IgG comprising the sequences of any one of SEQ ID Nos:56 to 70 or having at least 90% sequence identity with any one of SEQ ID NOs:56 to 70.

Herein described is also a fusion polypeptide comprising the recombinant DAO variant described herein and an Fc domain of human IgG or a functional derivative or analogue thereof encoded by any one of SEQ ID NOs:72 to 102 or having at least 90% sequence identity with any one of SEQ ID NOs:72 to 102.

Further provided herein is a recombinant vector comprising the nucleotide sequence described herein, specifically the vector is a bacterial, yeast, baculoviral, plant or mammalian expression vector.

According to an embodiment, herein provided is an expression cassette comprising the nucleotide sequence, operably linked to regulatory elements.

According to an embodiment, herein provided is a recombinant host cell or a host cell line of bacterial, yeast, baculoviral, plant or mammalian origin, comprising the recombinant DAO variant described herein, wherein the host cells are specifically selected from the group consisting of CHO cells, Vero cells, MDCK cells, Pichia pastoris cells, and SF9 cells.

Further provided herein is an expression system comprising the vector, or the expression cassette and a host cell, or host cell line described herein.

According to an embodiment, herein provided is also a method for producing the recombinant DAO variant described herein, said method comprising the steps of
i. cloning a nucleotide sequence encoding the DAO variant into an expression vector,
ii. transforming a host cell with said vector,
iii. cultivating the transformed host cell under conditions wherein the DAO variant is expressed,
iv. isolating the DAO variant from the host cell culture, optionally by disintegrating the host cells, and optionally
v. purifying the DAO variant.

According to an embodiment, a pharmaceutical composition is provided comprising the recombinant DAO variant and optionally one or more excipients.

Specifically, the pharmaceutical composition can be applied intravenously, intramuscularly and subcutaneously or via other parenteral routes of administration like intraperitoneally or intrathecally.

Further described is the use of the recombinant DAO variant for preparing a pharmaceutical composition.

Specifically, the recombinant DAO variant is provided for use in the treatment of a condition associated with excess histamine, specifically for the treatment of chronic allergic diseases or diseases associated with increase of histamine or decreased DAO activity, more specifically for the treatment of anaphylaxis, anaphylactic shock, chronic urticaria, acute urticaria, asthma, hay fever, allergic rhinitis, allergic conjunctivitis, histamine intoxication, headache, atopic dermatitis inflammatory diseases, mastocytosis, mast cell activation syndrome (MCAS), pre-eclampsia, hyperemesis gravidarum, pre-term labor, peptic ulcers, acid reflux, pruritus, and sepsis.

In an embodiment of the invention, the use of the recombinant DAO variant is encompassed for the manufacture of a medicament for the treatment of a condition associated with excess histamine, specifically for the treatment of chronic allergic diseases, or diseases associated with increase of histamine or decreased DAO activity, more specifically for the treatment of anaphylaxis, anaphylactic shock, chronic urticaria, acute urticaria, asthma, hay fever, allergic rhinitis, allergic conjunctivitis, histamine intoxication, headache, atopic dermatitis inflammatory diseases, mastocytosis, mast cell activation syndrome (MCAS), pre-eclampsia, hyperemesis gravidarum, pre-term labor, peptic ulcers, acid reflux, pruritus, and sepsis.

Also described is a target-specific ligand specifically binding to the GAG binding domain of DAO, specifically binding to one or more of amino acids at position 568-575 with reference to the numbering of SEQ ID NO:1.

Alternatively, herein described is a target-specific ligand specifically inhibiting heparin/heparan sulfate binding to the GAG binding domain of DAO, specifically to any one or more of amino acids at position 568-575 with reference to the numbering of SEQ ID NO:1.

Specifically, the ligand is selected from the group consisting of nucleic acid, small molecule inhibitor or antigen binding protein.

The ligand is an antigen binding protein, specifically selected from the group consisting of
- antibodies or antibody fragments, such as any of Fab, Fd, scFv, diabodies, triabodies, Fv tetramers, minibodies, nanobodies, single-domain antibodies like VH, VHH, IgNARs, or V-NAR;
- antibody mimetics, such as Adnectins^{™}, Affibodies^{®}, Affilins^{®}, Affimers^{®}, Affitins, Alphabodies, Aptamers, Anticalins, Avimers, DARPins^{®}, Fynomers^{®}, Kunitz domain peptides, Monobodies, or NanoCLAMPS; or
- fusion proteins comprising one or more immunoglobulin-fold domains, antibody domains or antibody mimetics.

Herein described is a method for identifying compounds which modulate the heparin binding of the DAO, comprising the steps of
(a) constructing a computer model of the GAG binding domain defined by the structure coordinates of the amino acids of the DAO sequence of SEQ ID NO:1,
(b) selecting a potential modulating compound by a method selected from the group consisting of:
   (i) assembling molecular fragments into said compound,
   (ii) selecting a compound from a small molecule database, and
   (iii) *de novo* ligand design of said compound;
(c) employing computational means to perform a fitting program operation between computer models of the said compound and the GAG binding domain in order to provide an energy-minimized configuration of the said compound in the heparin binding domain; and
(d) evaluating the results of said fitting operation to quantify the association between the said compound and the heparin/heparan sulfate binding domain, thereby evaluating the ability of said compound to associate with the said heparin binding domain.

### FIGURES

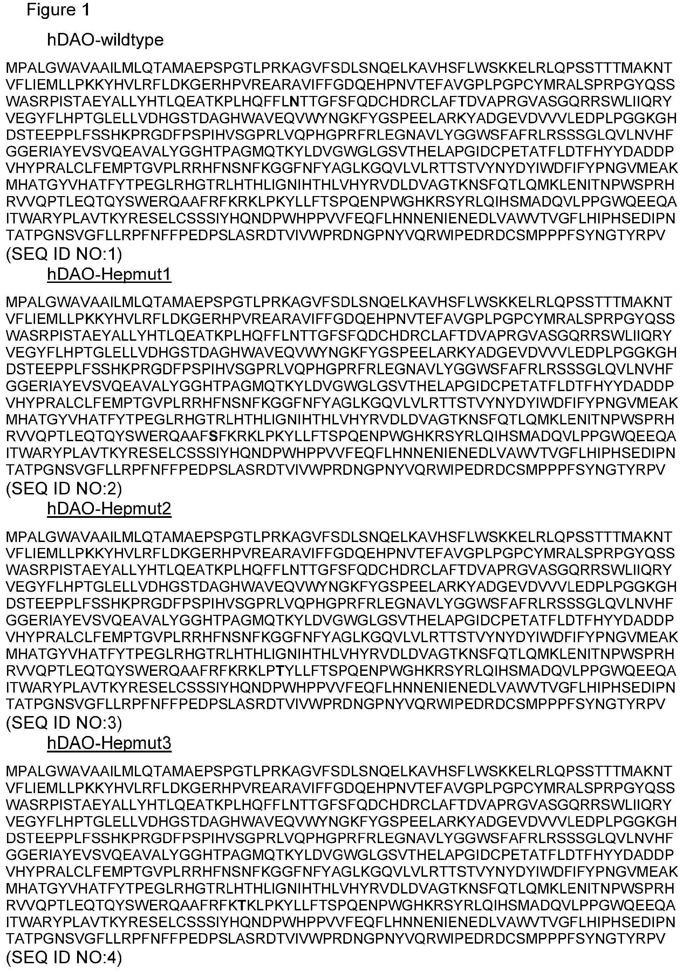

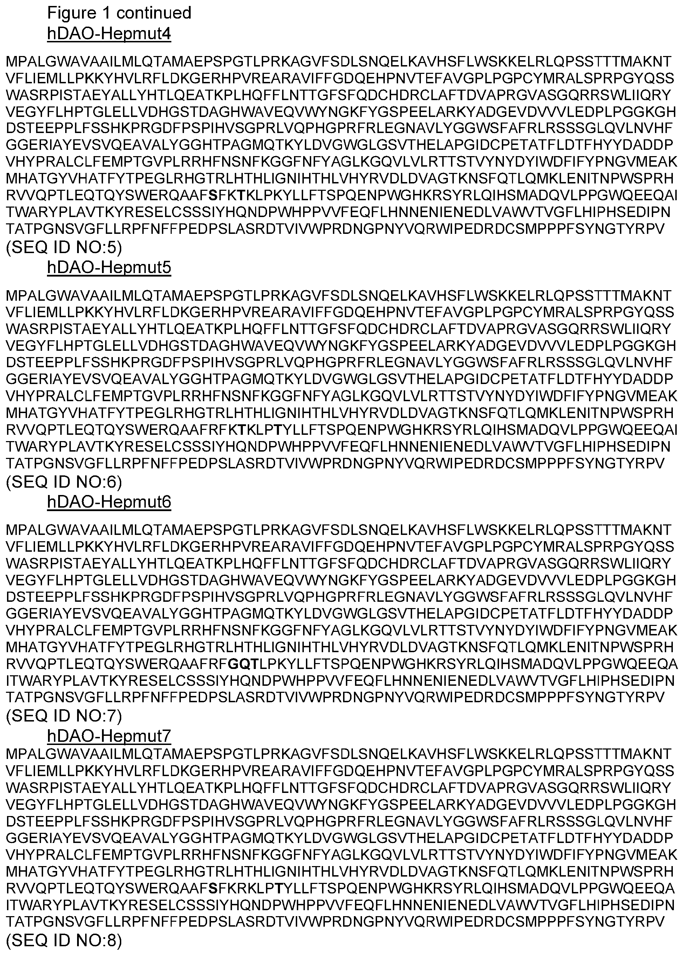

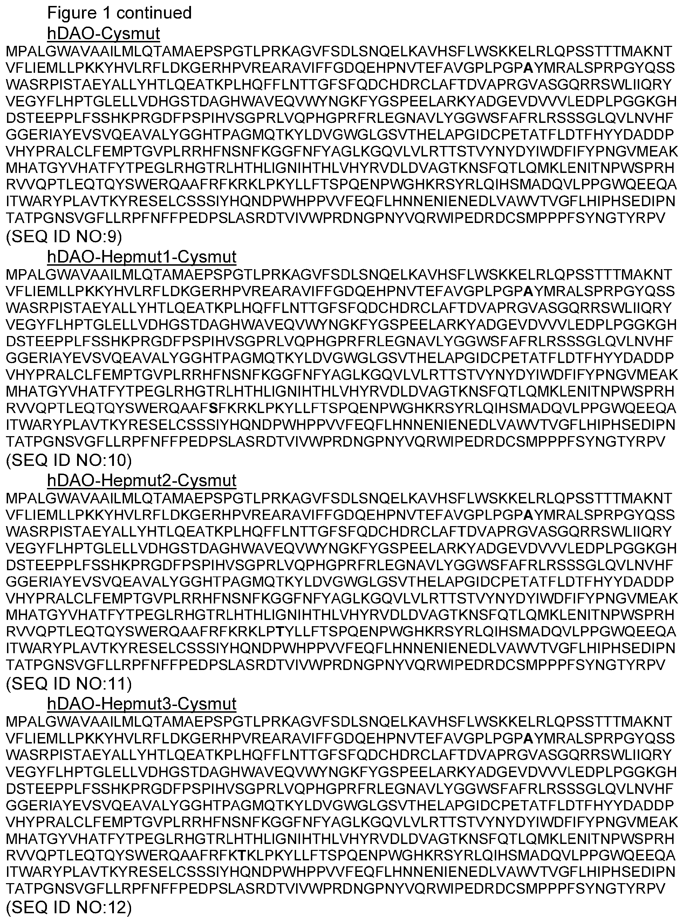

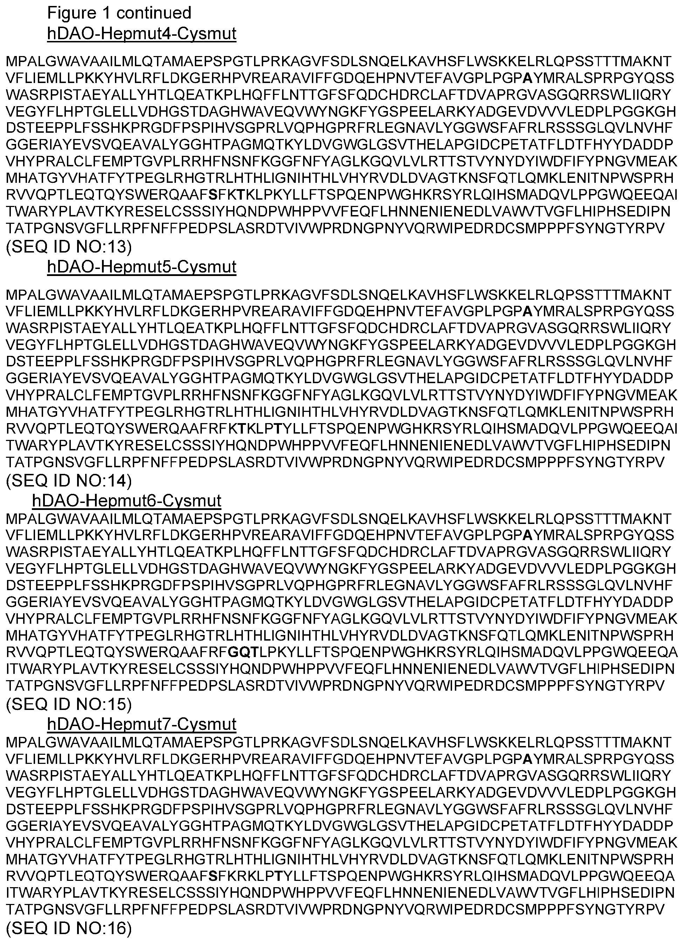

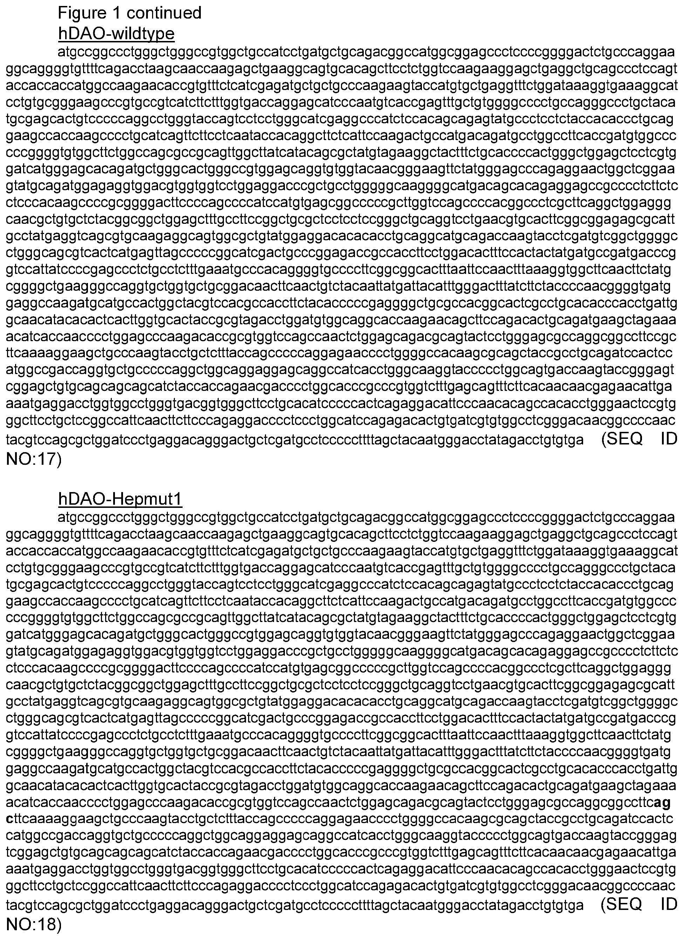

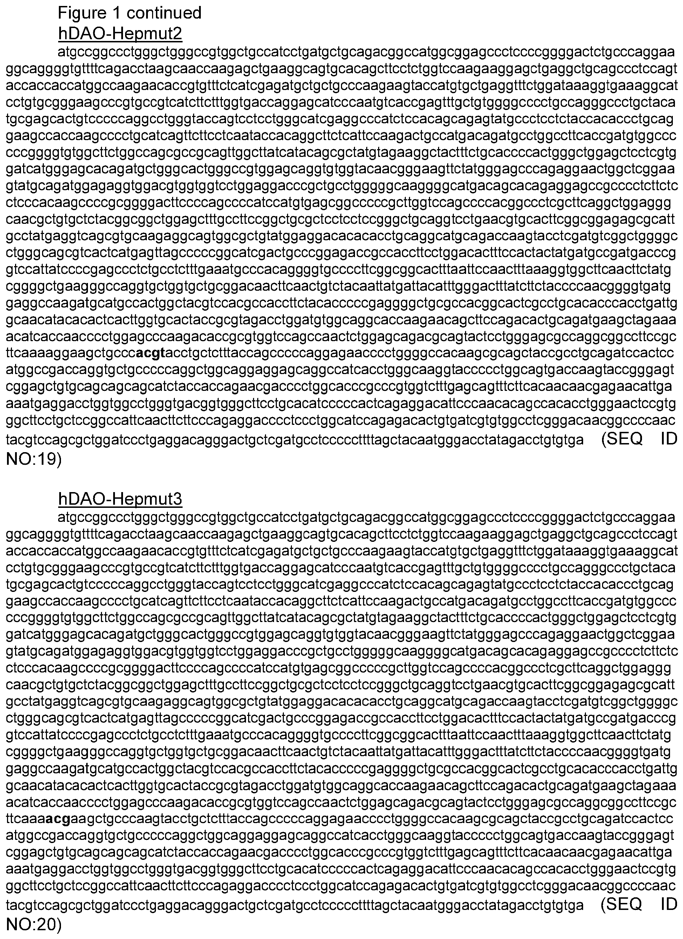

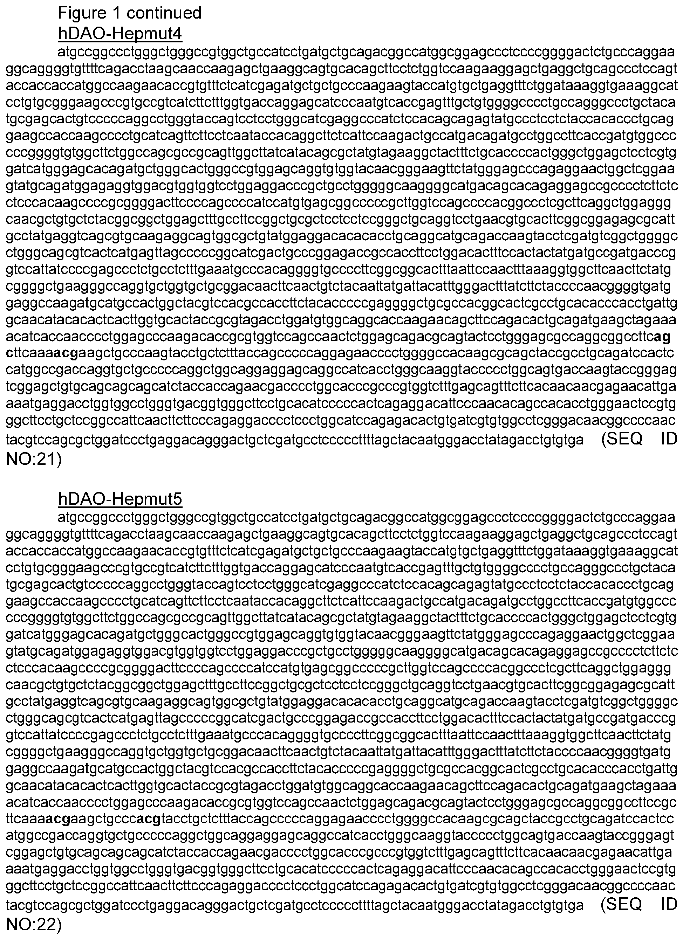

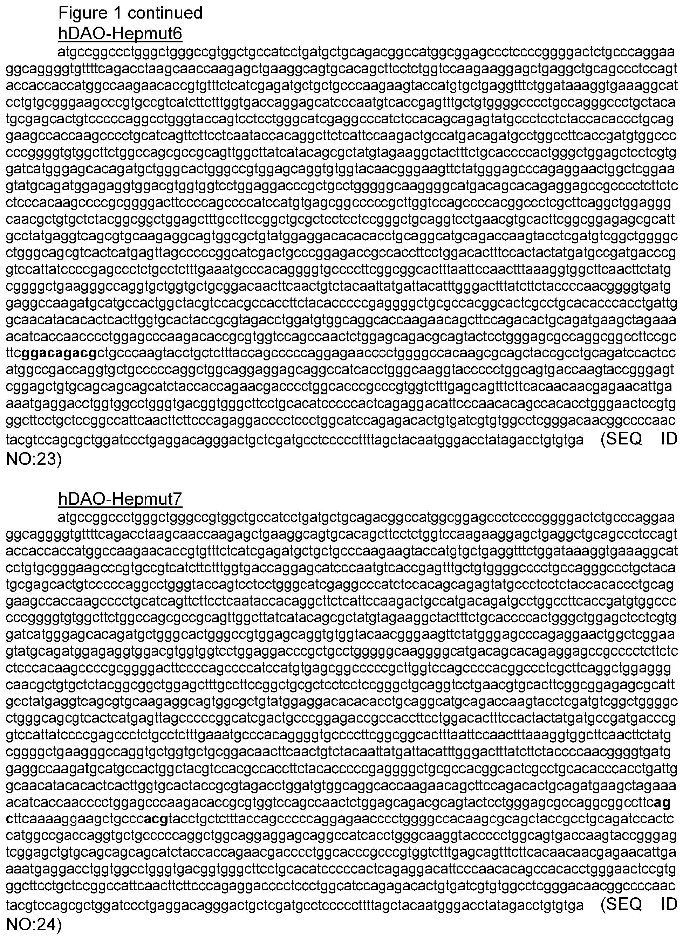

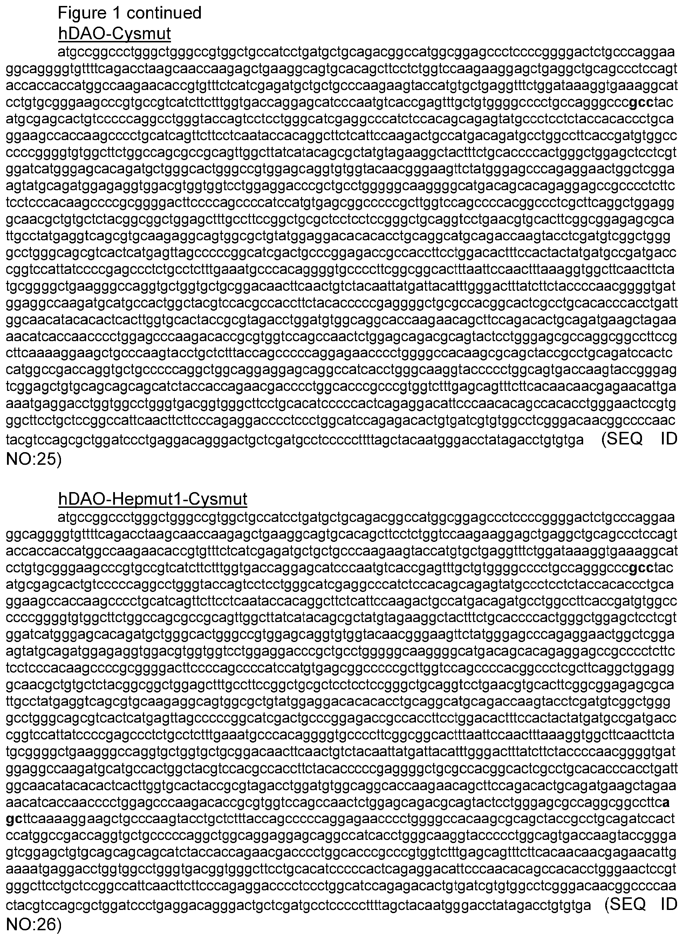

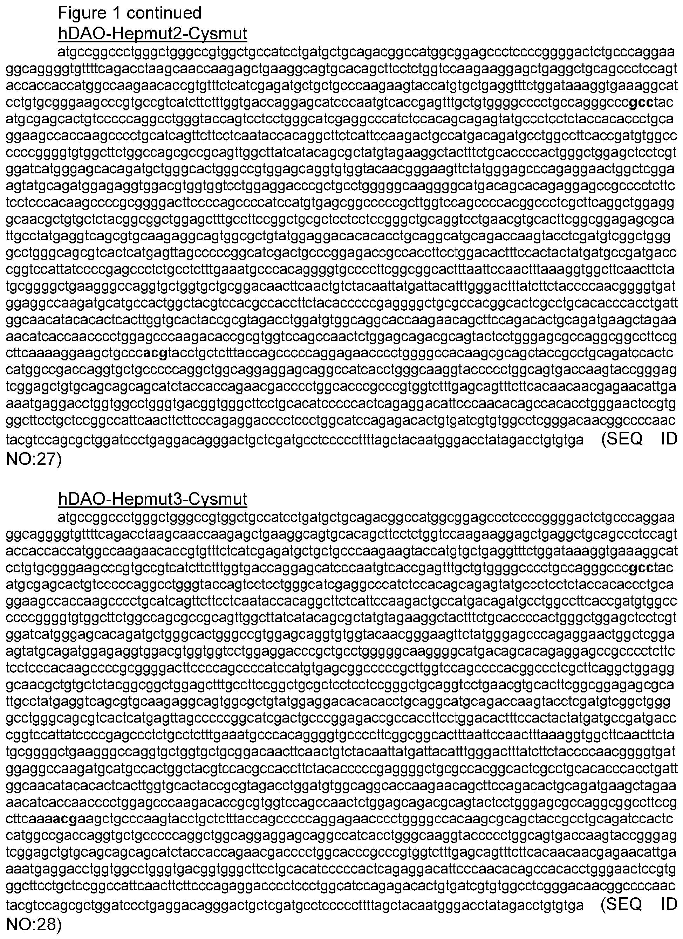

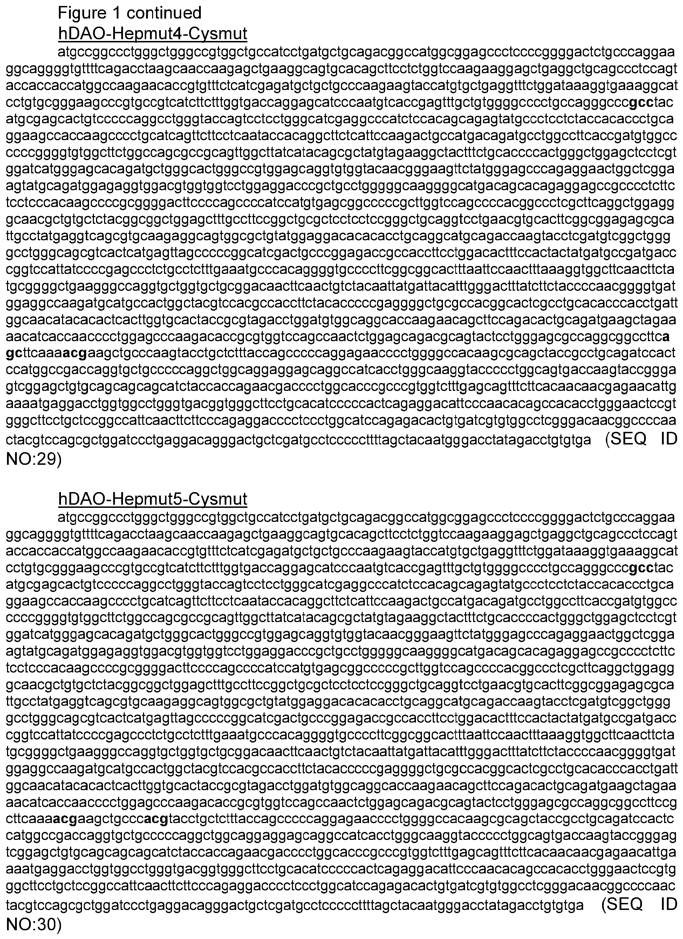

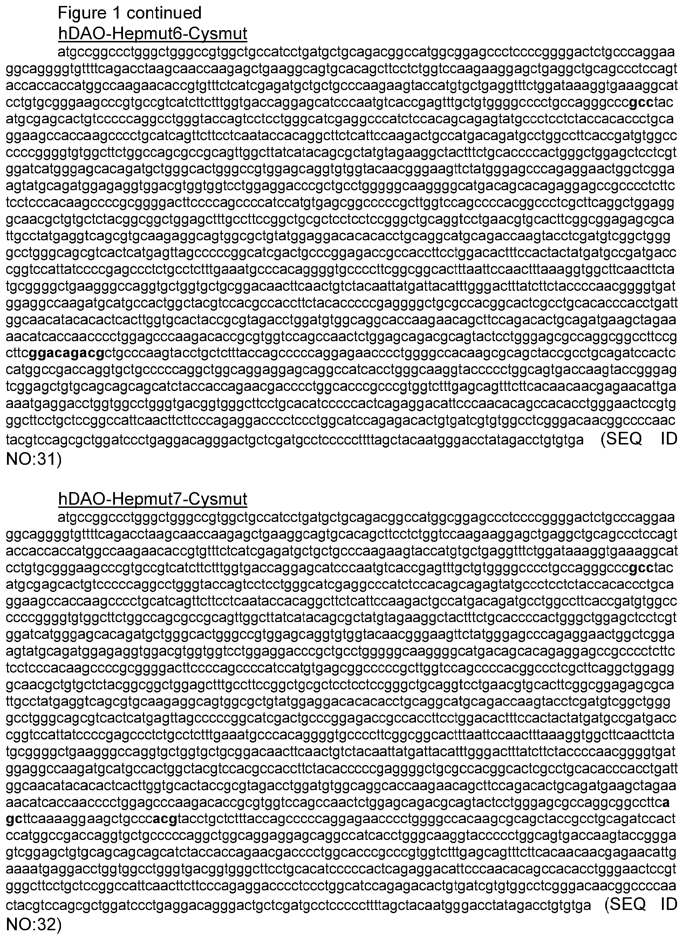

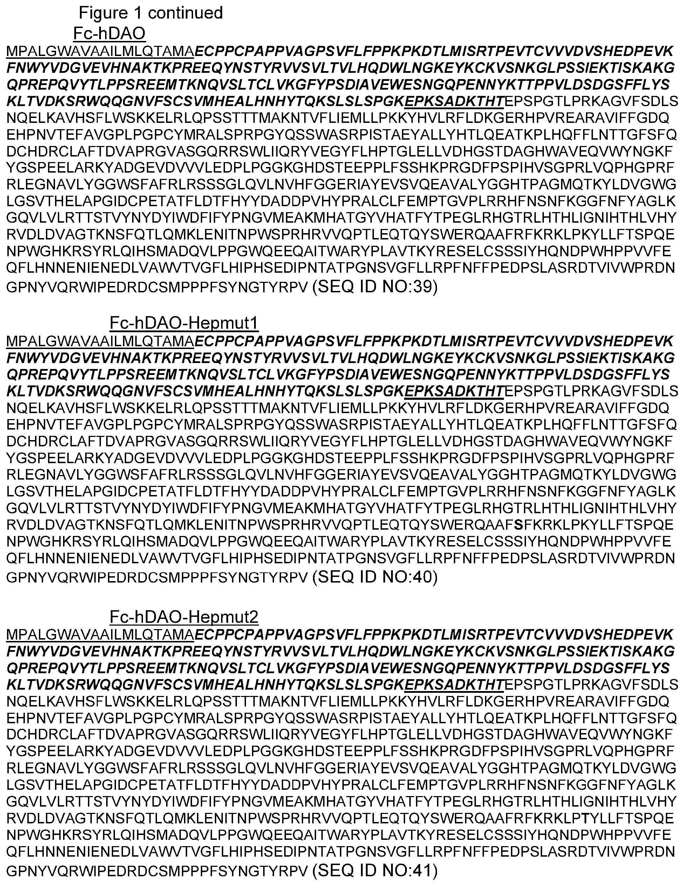

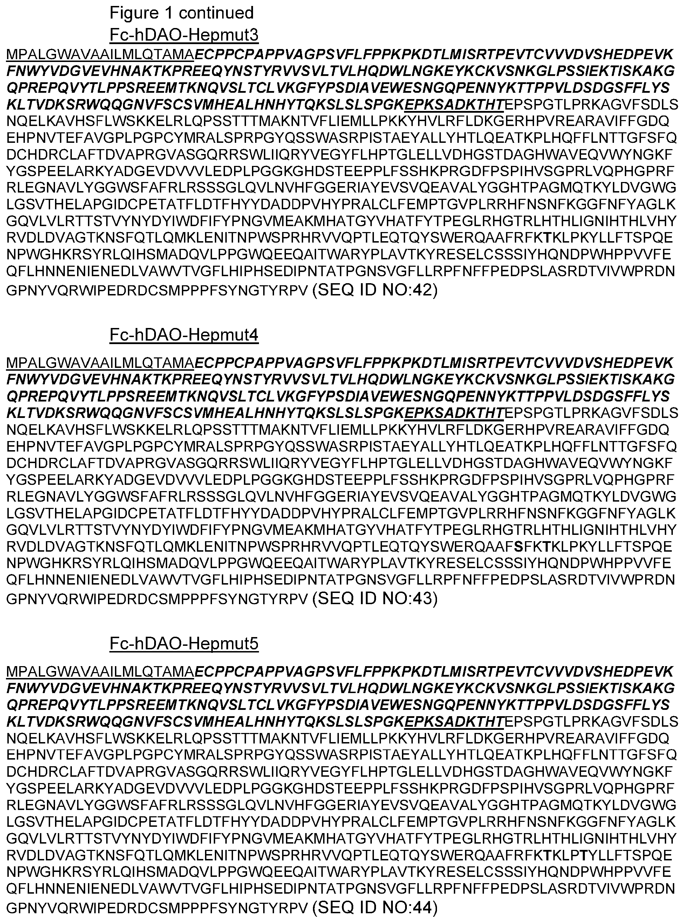

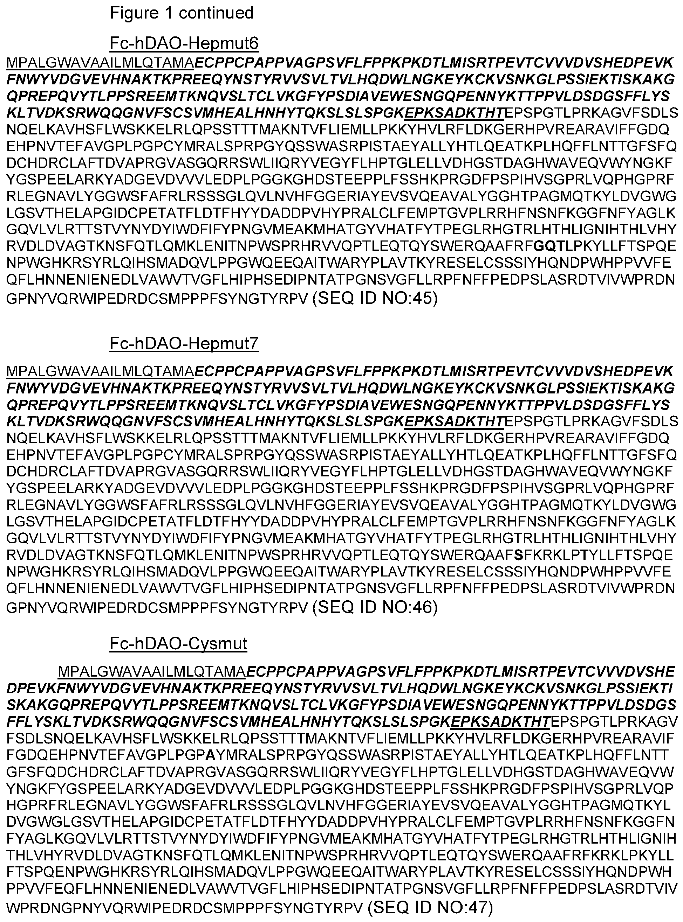

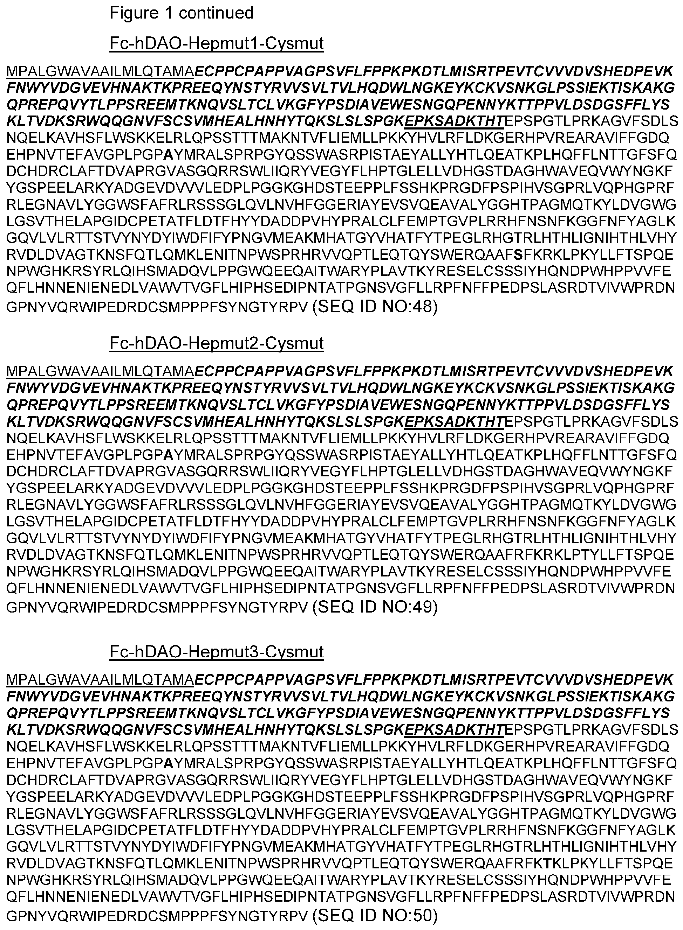

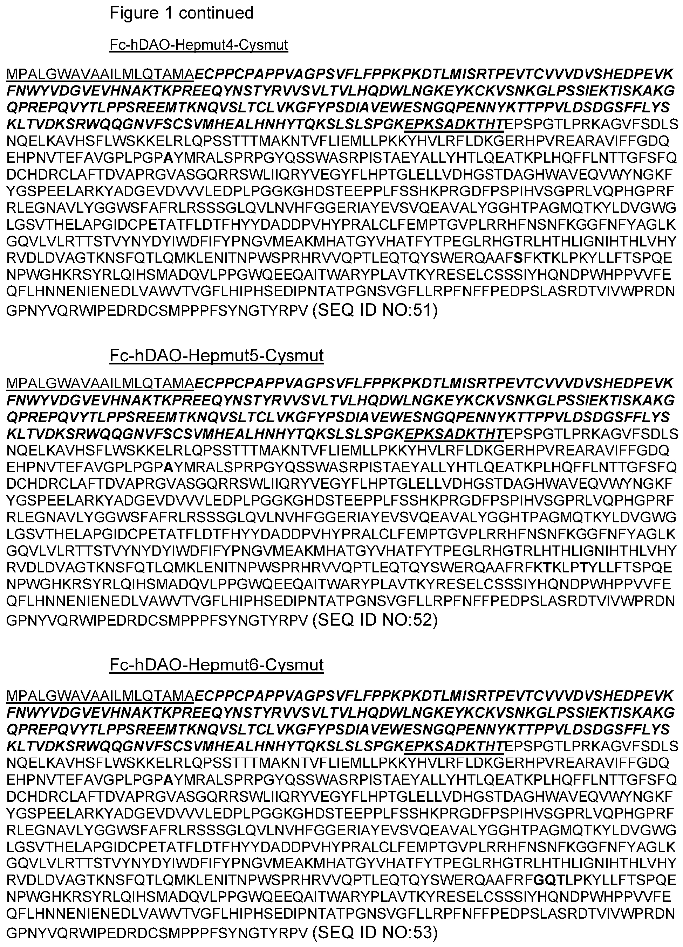

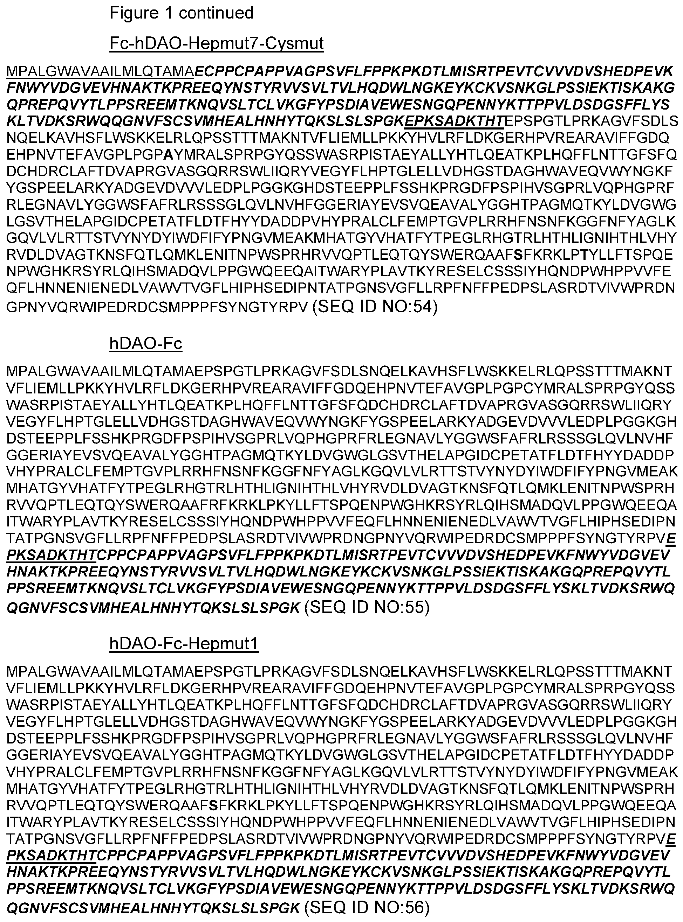

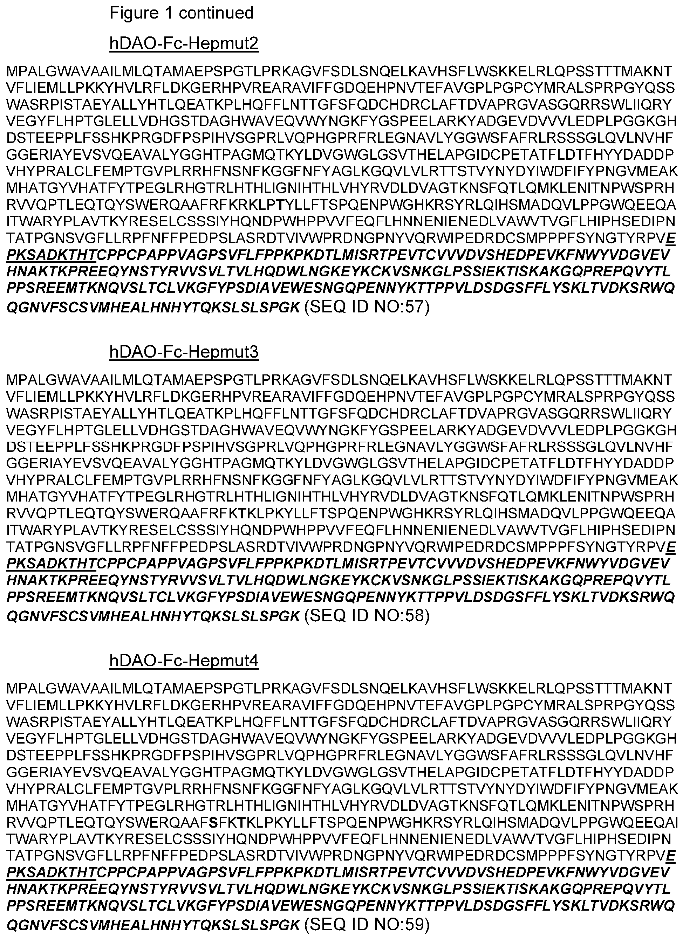

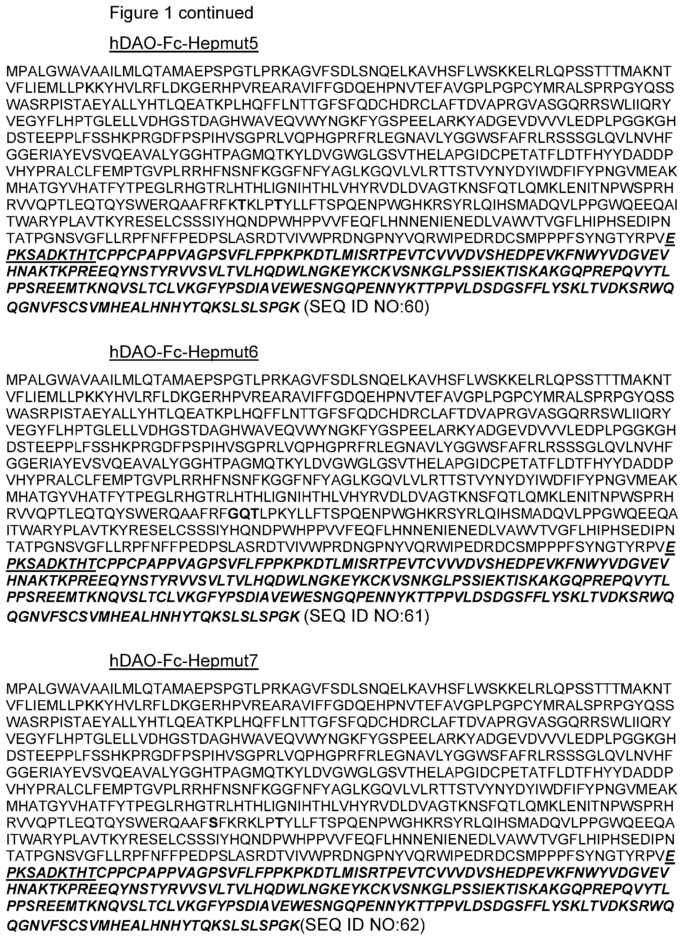

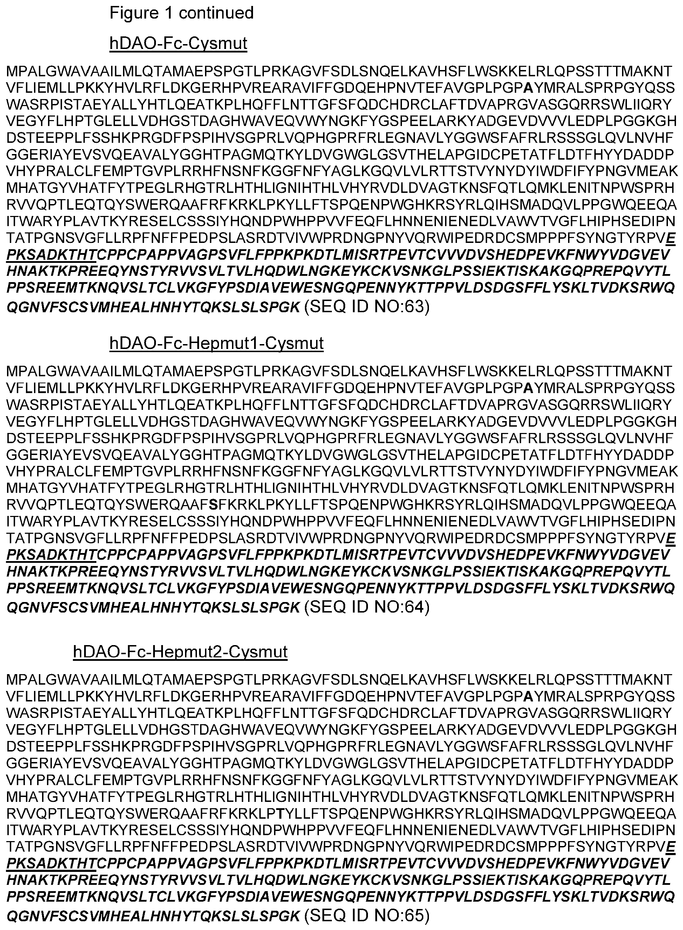

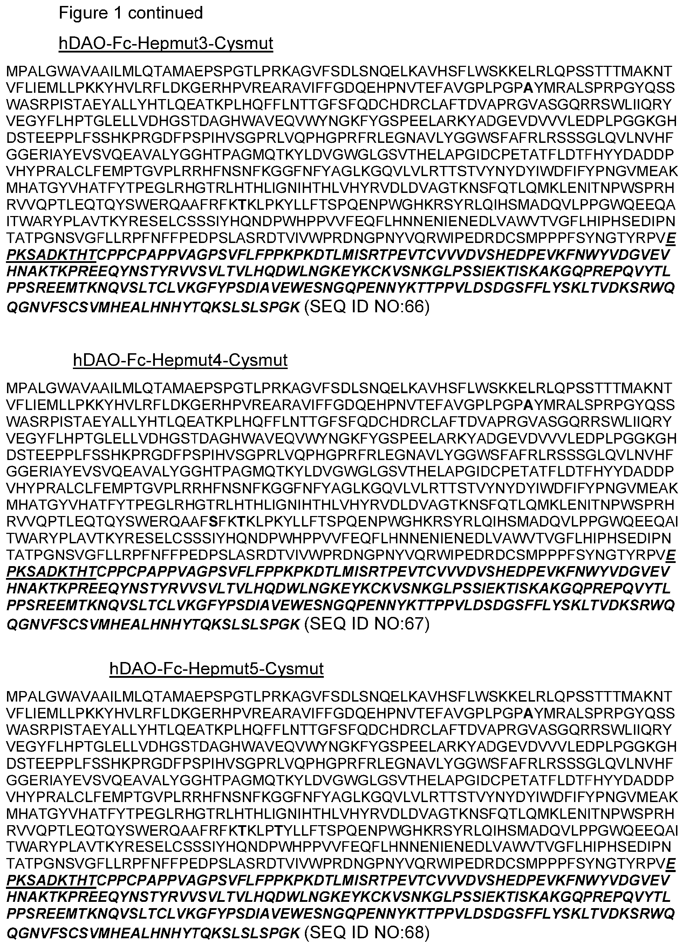

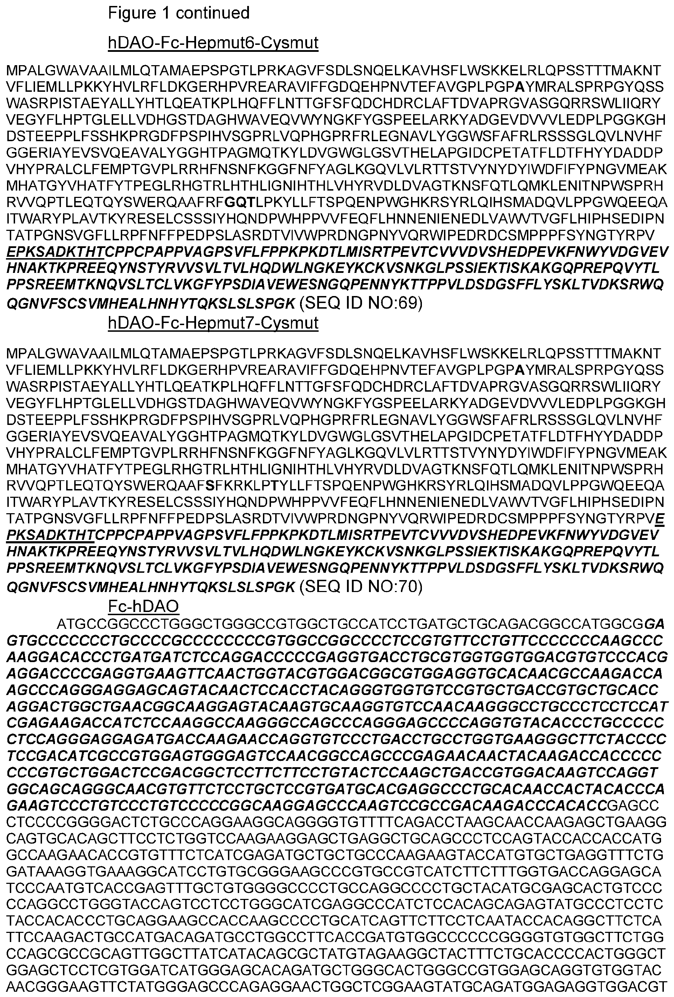

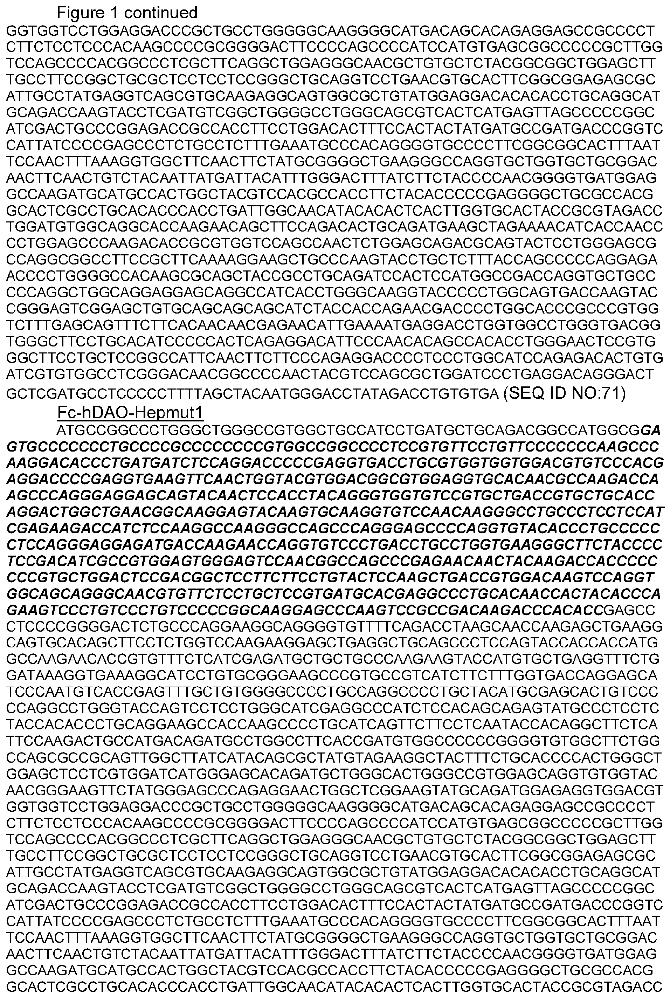

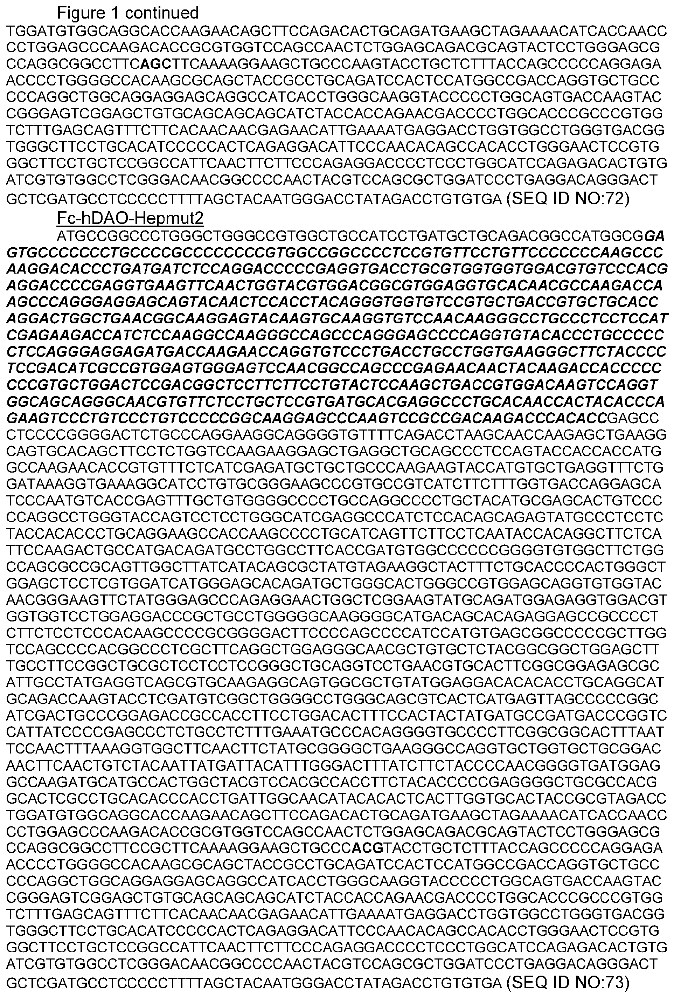

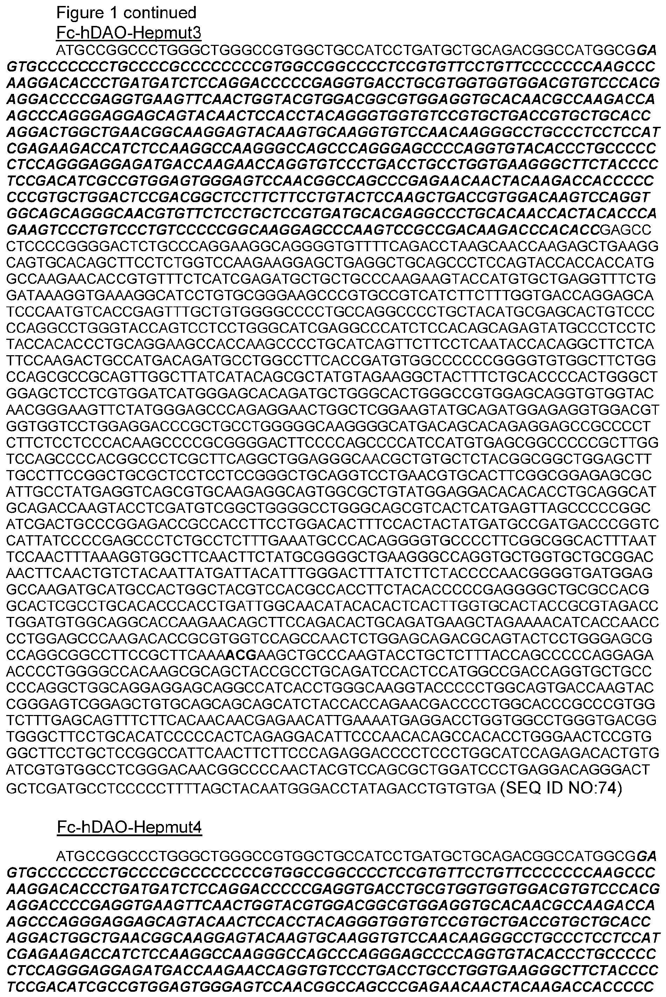

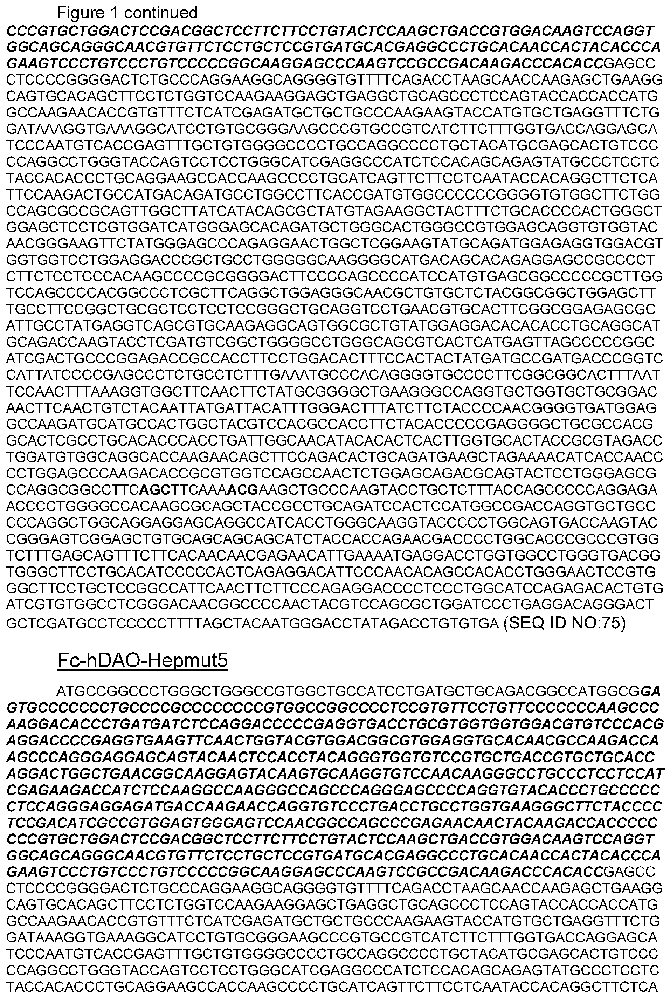

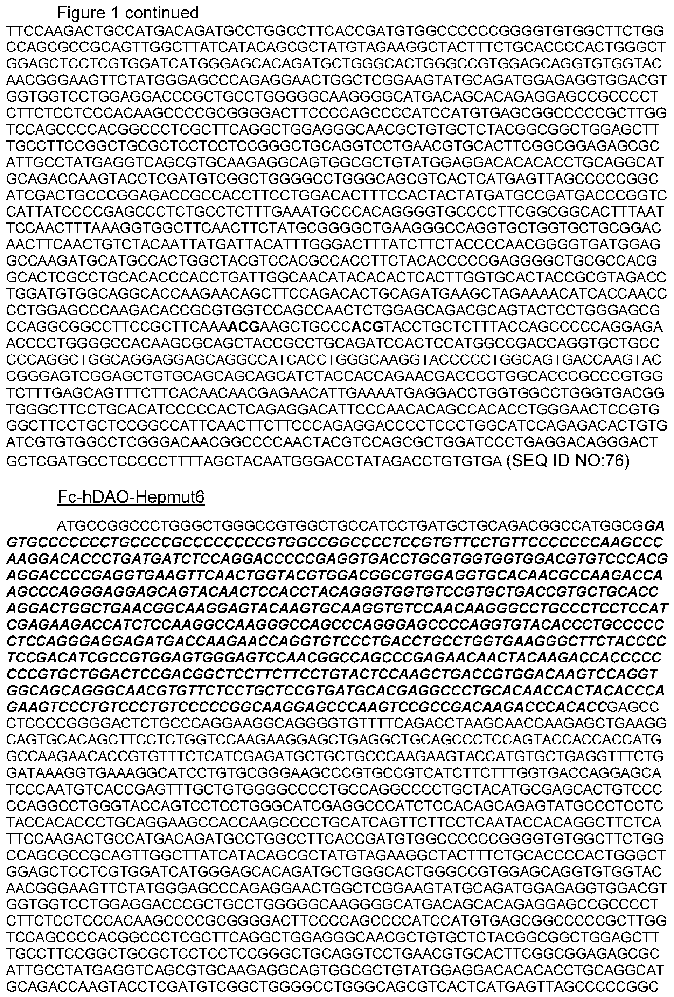

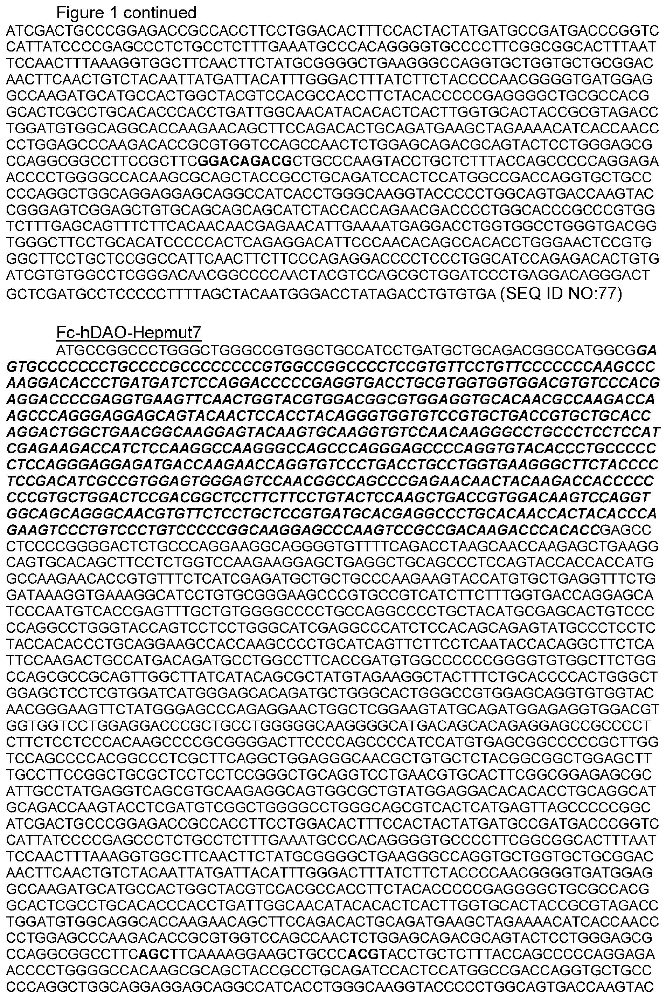

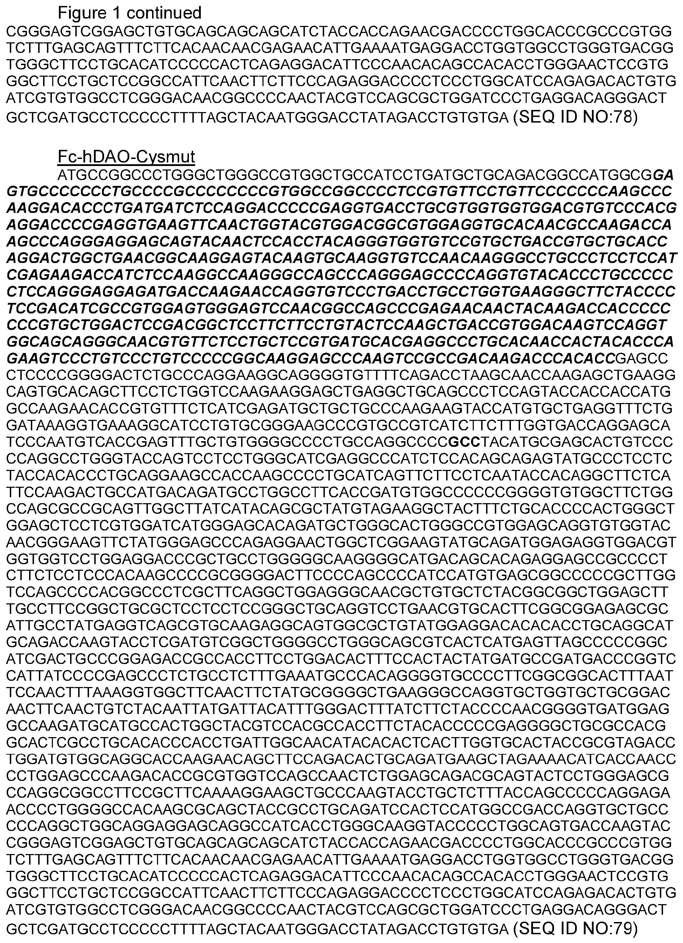

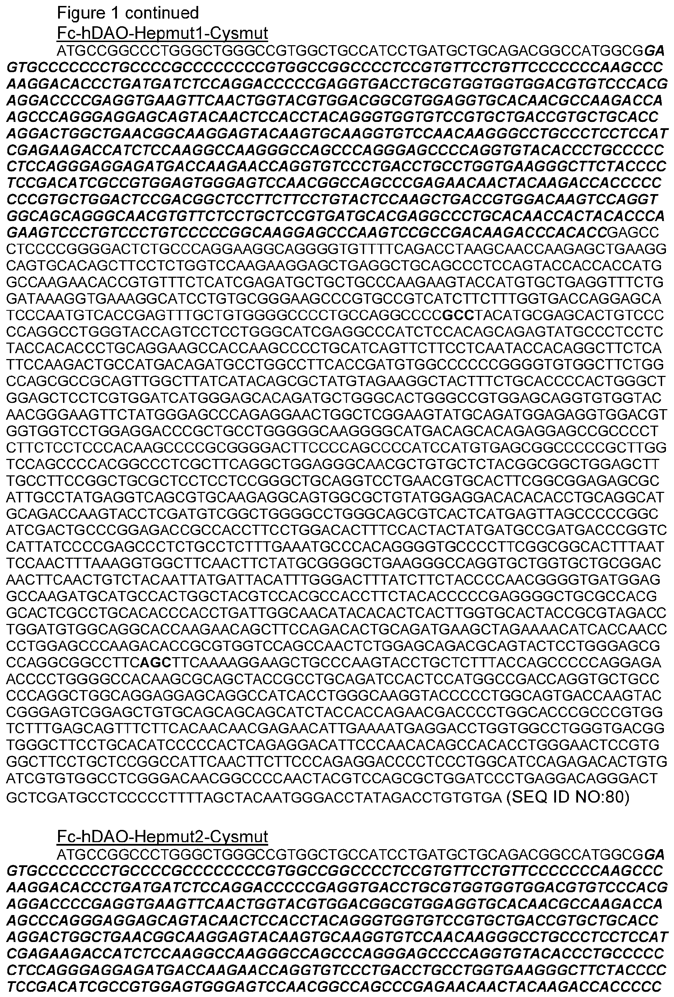

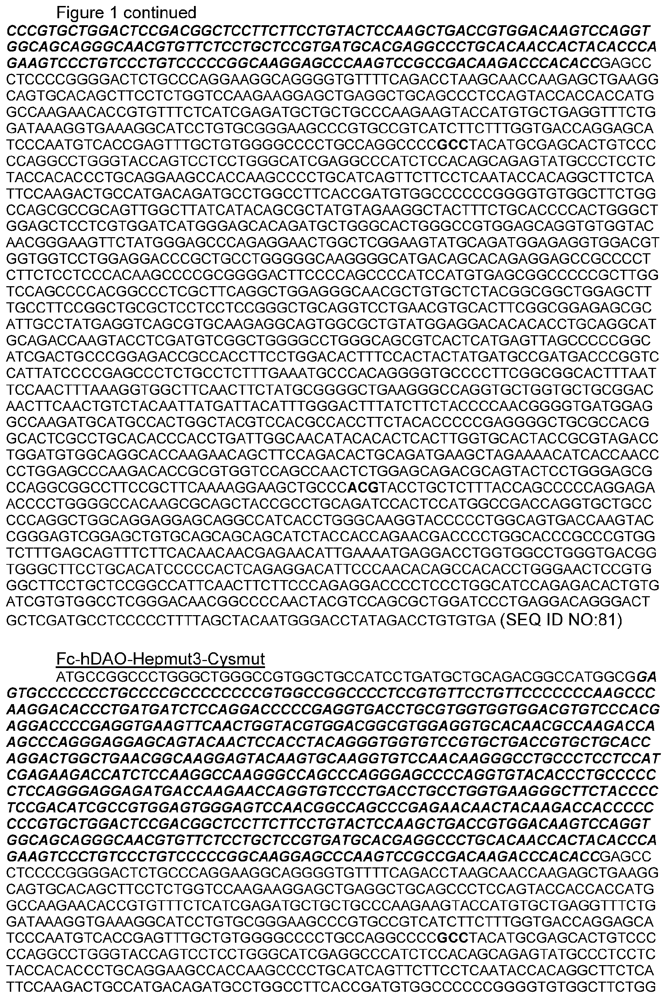

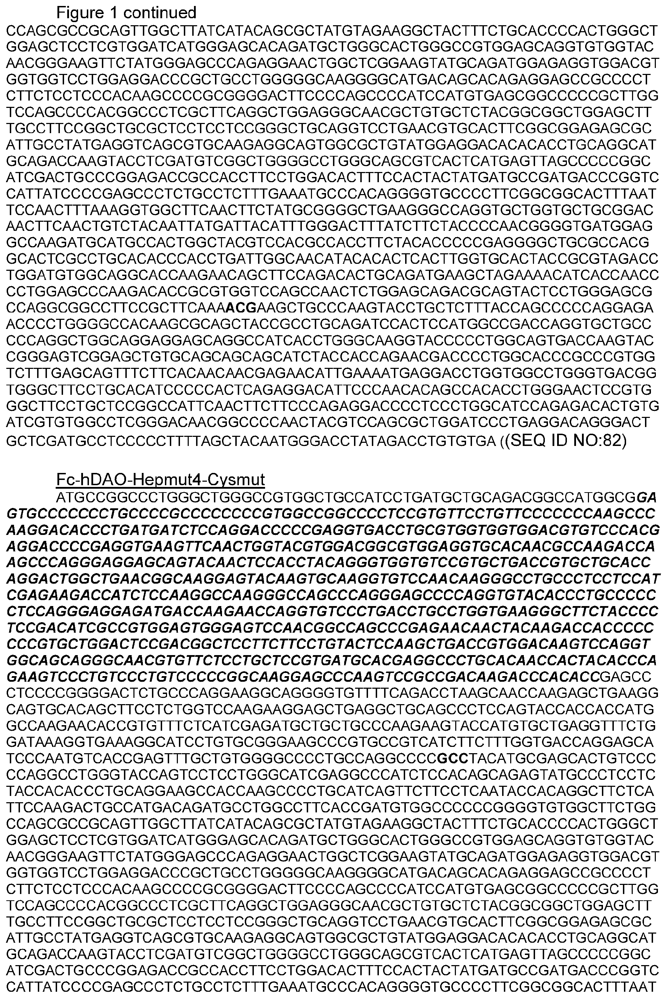

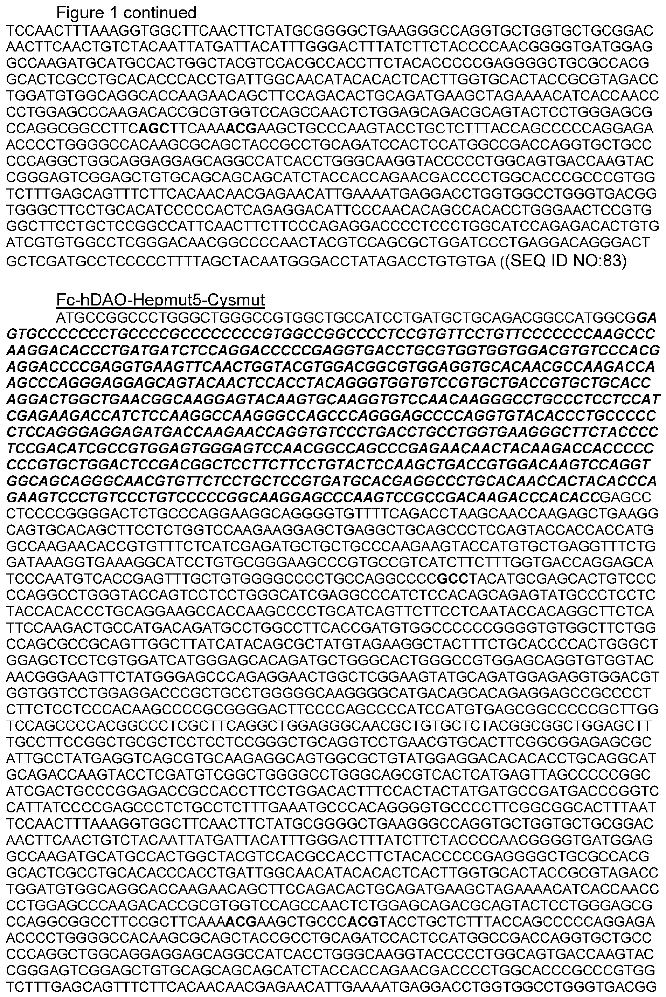

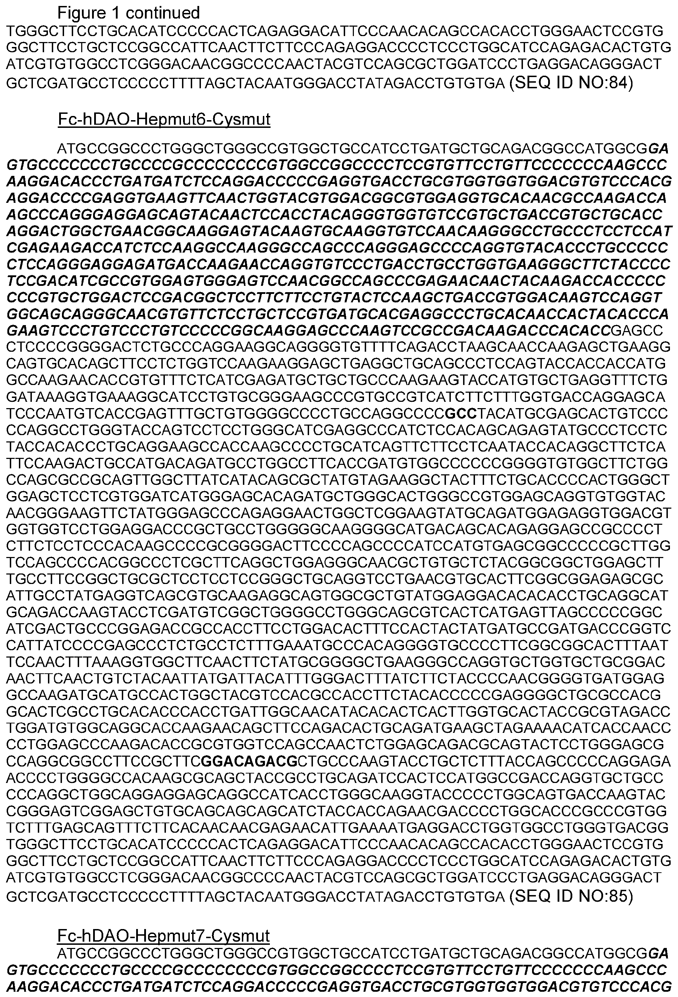

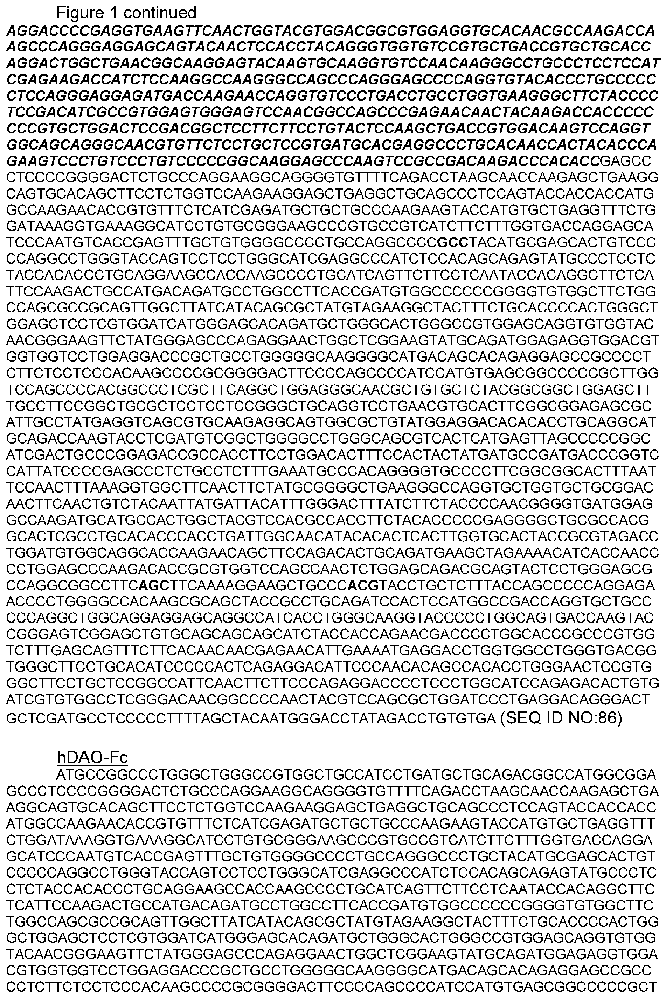

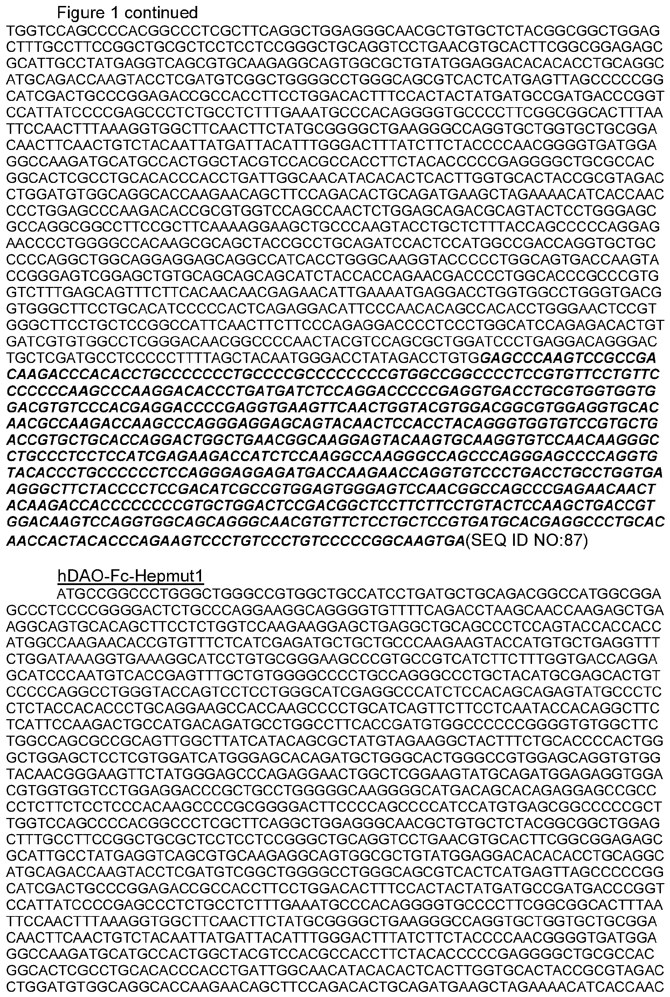

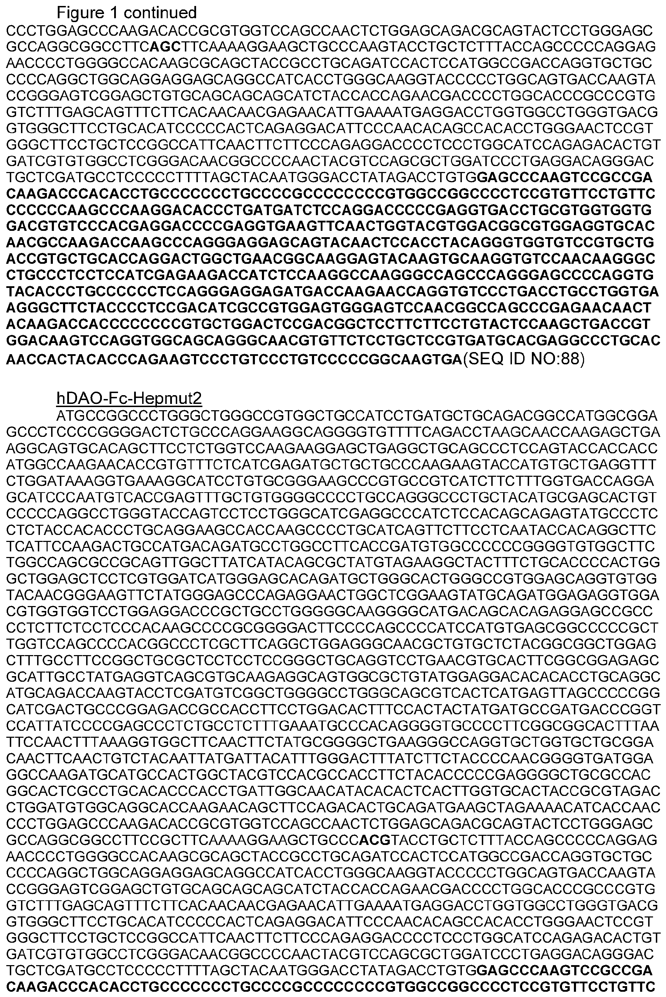

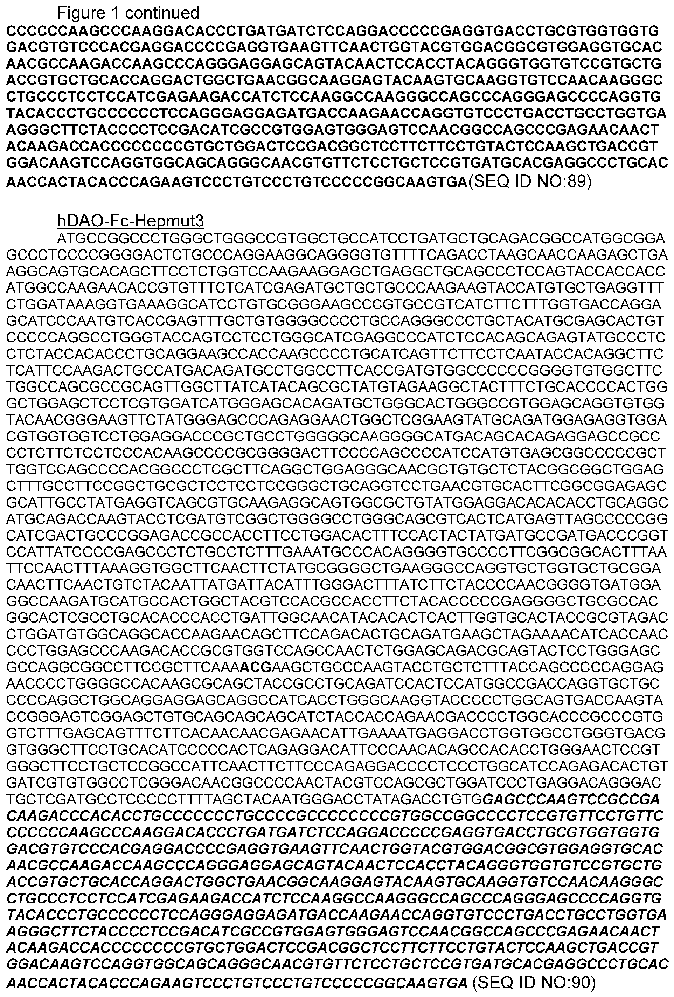

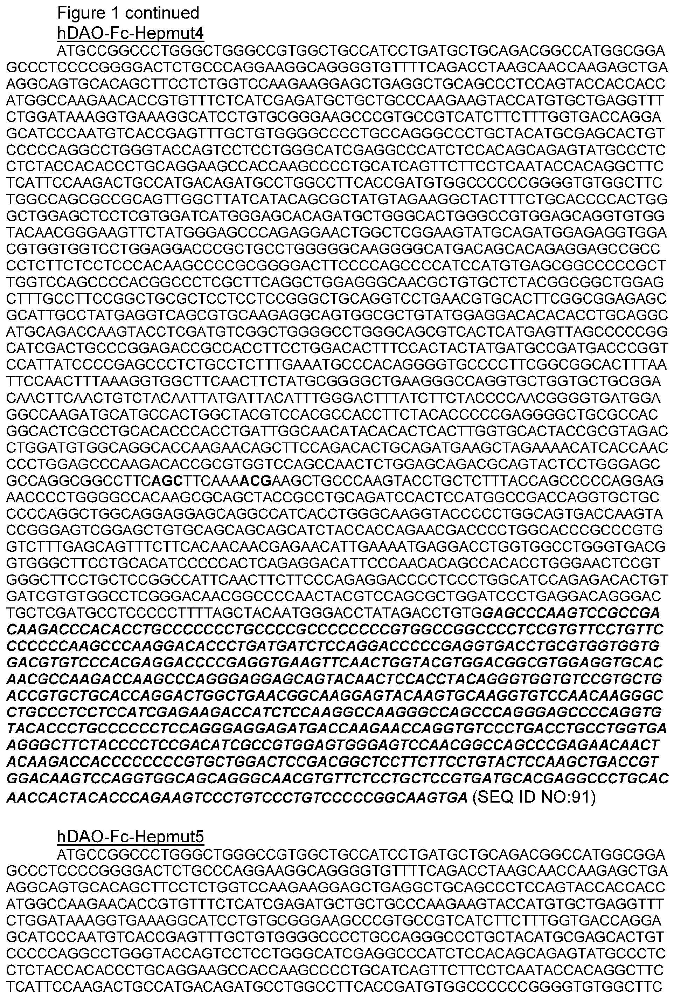

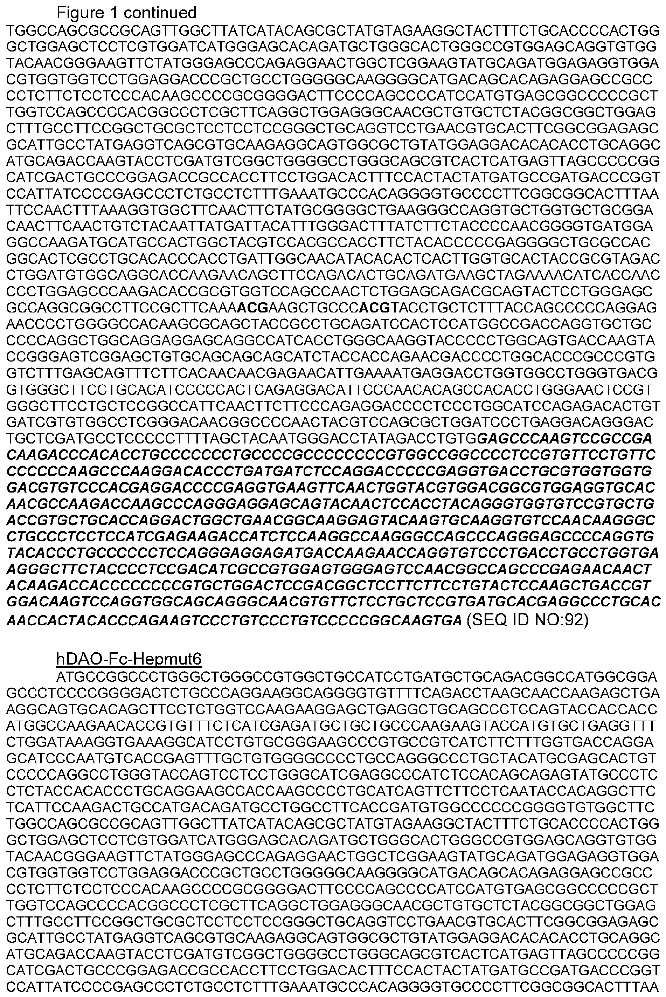

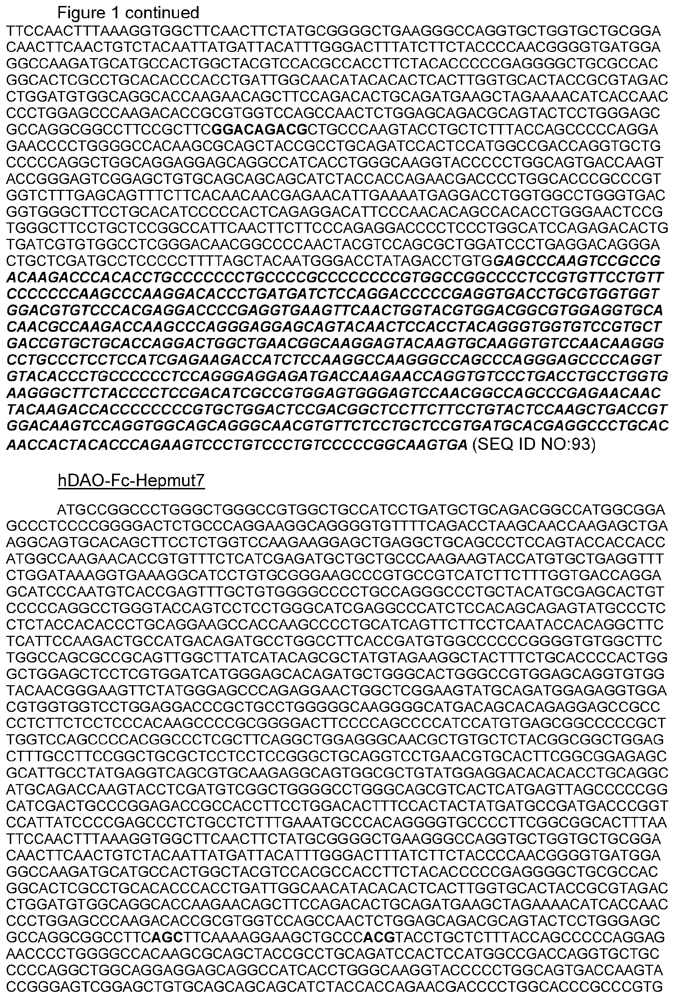

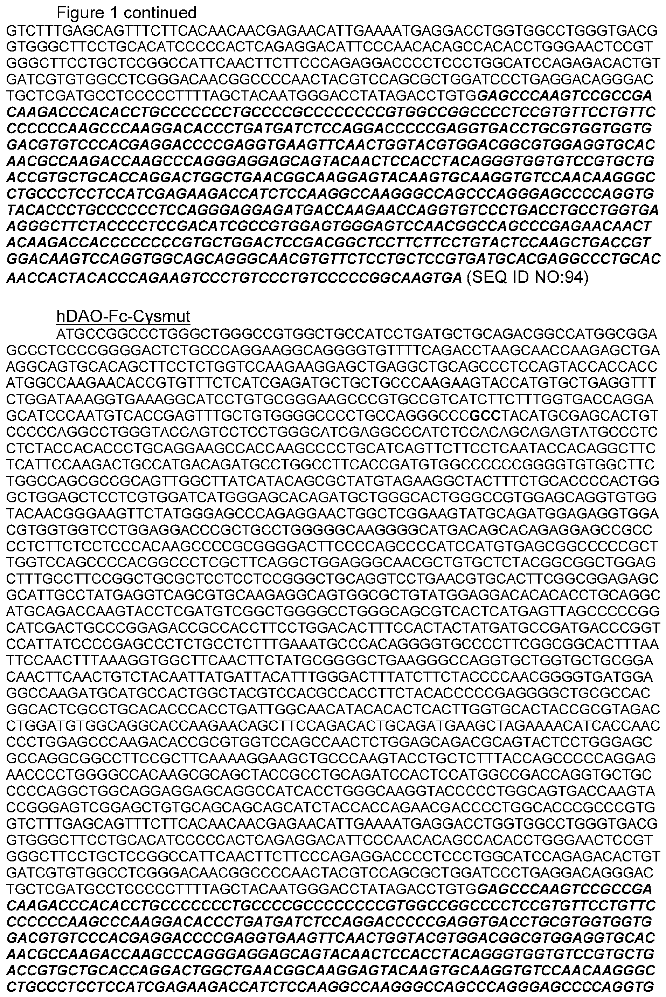

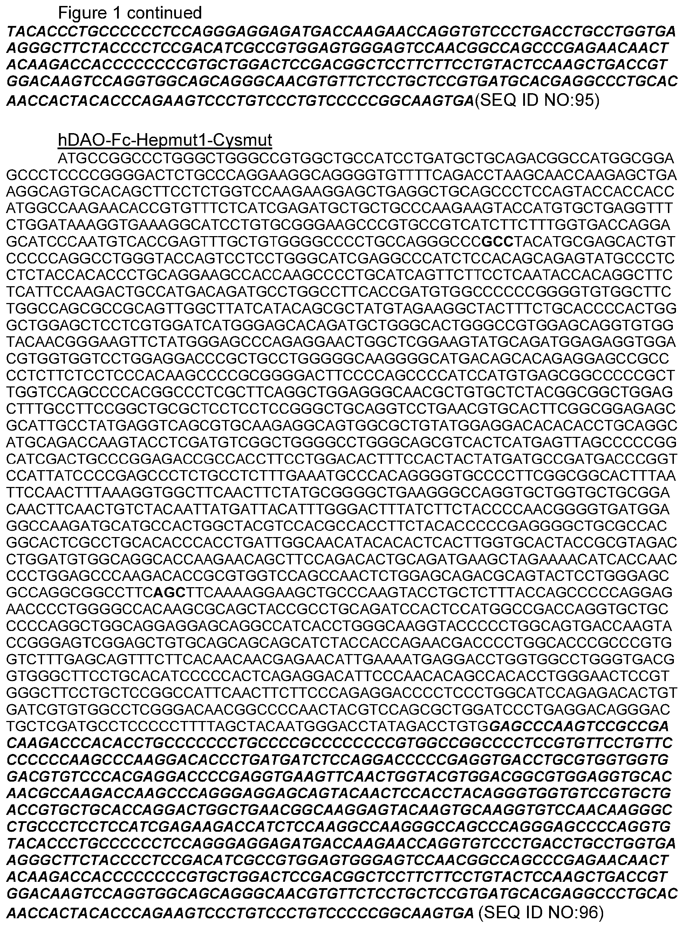

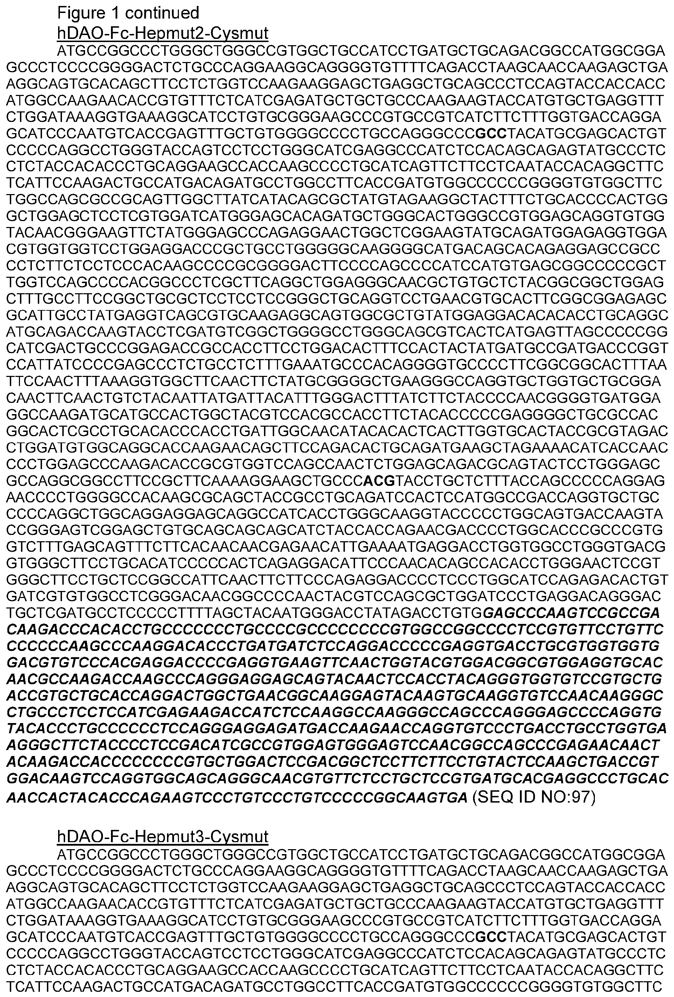

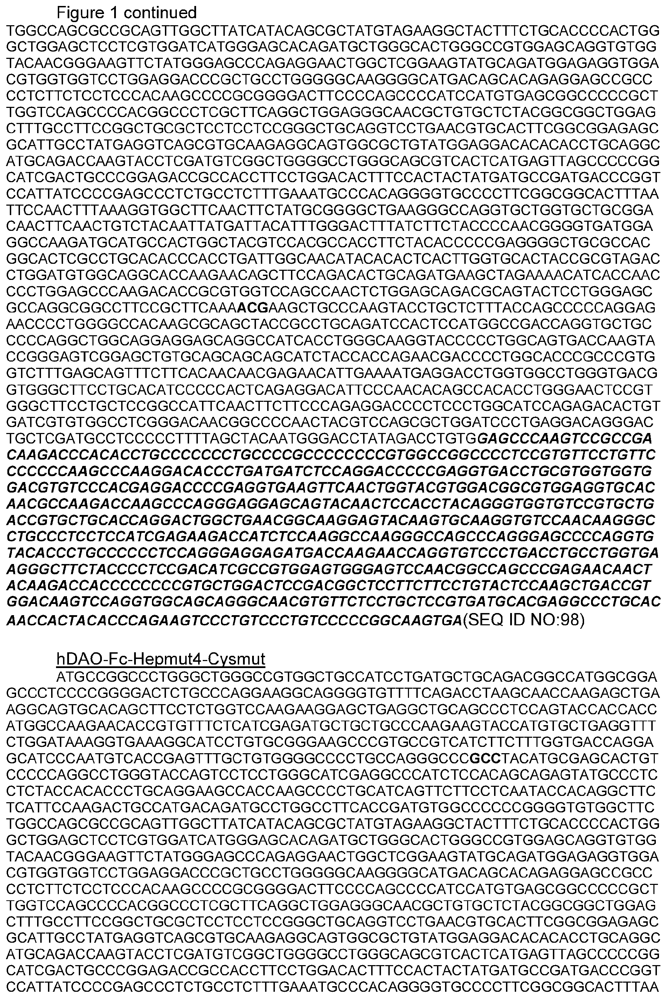

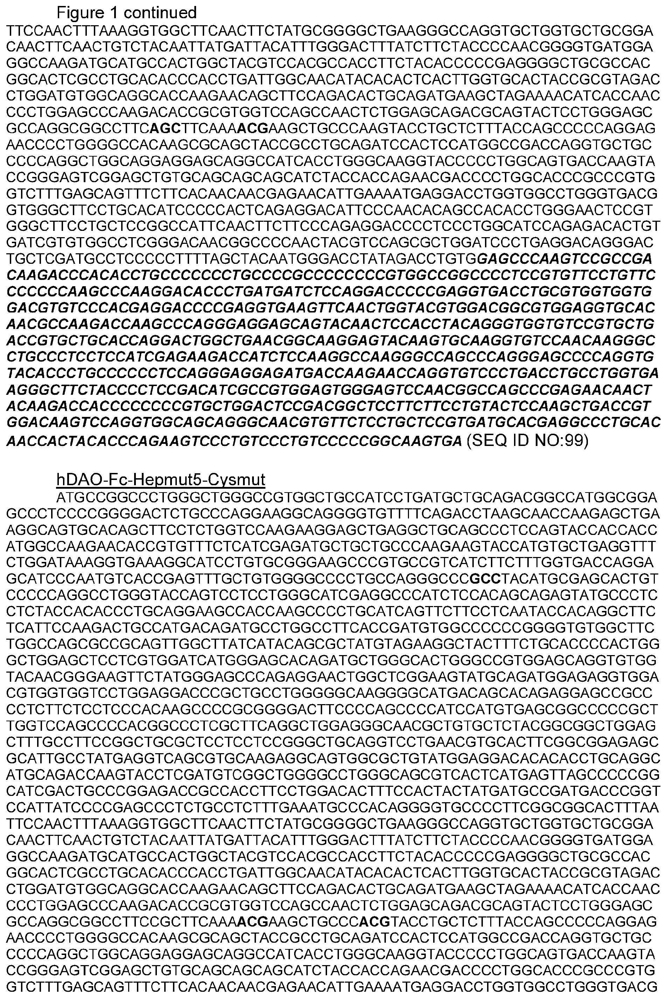

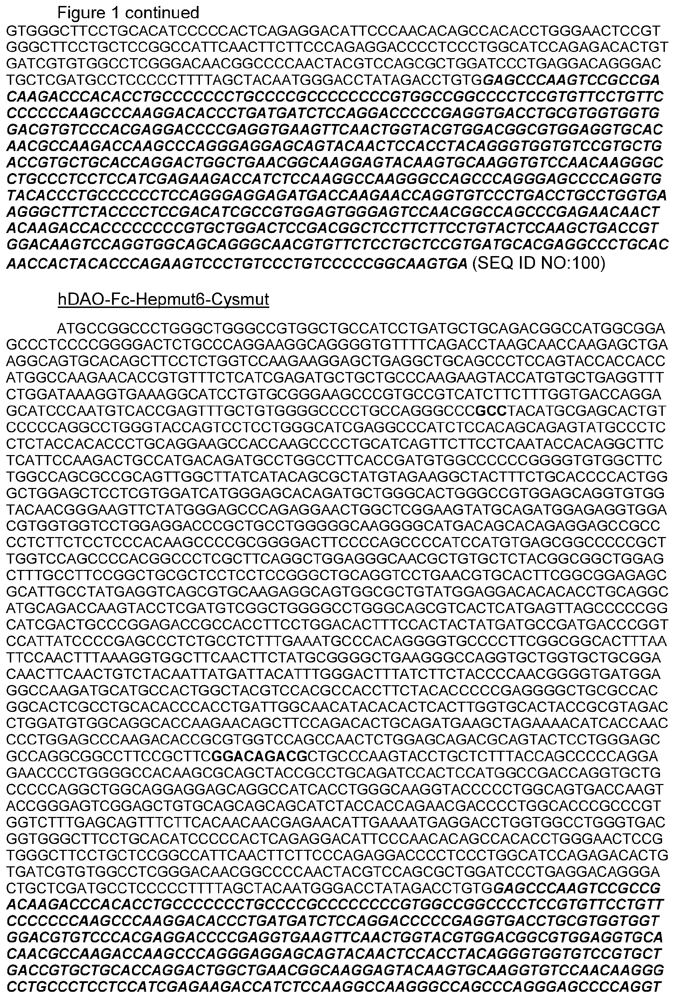

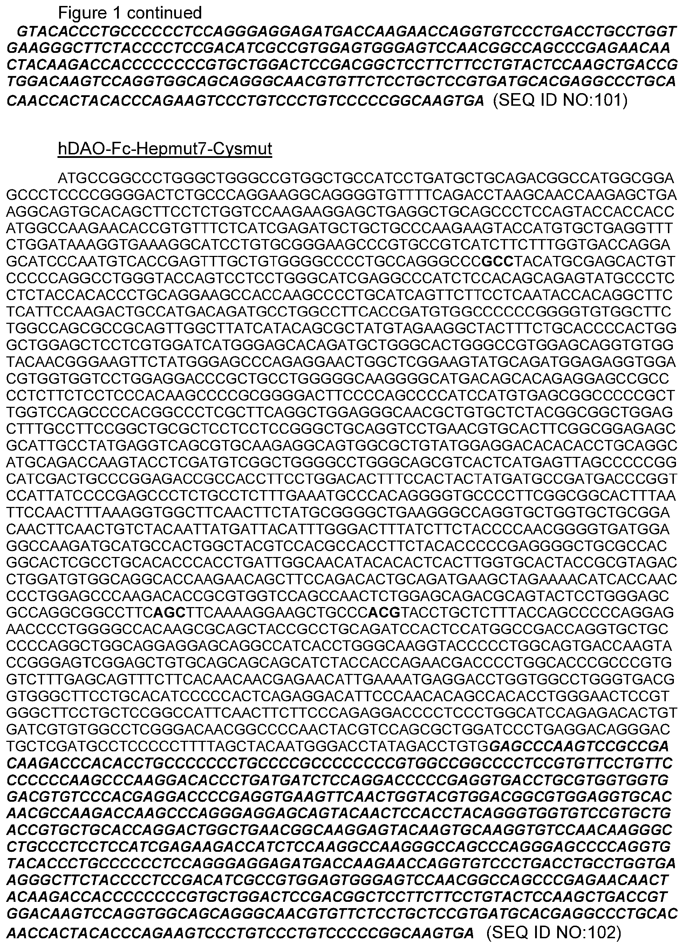

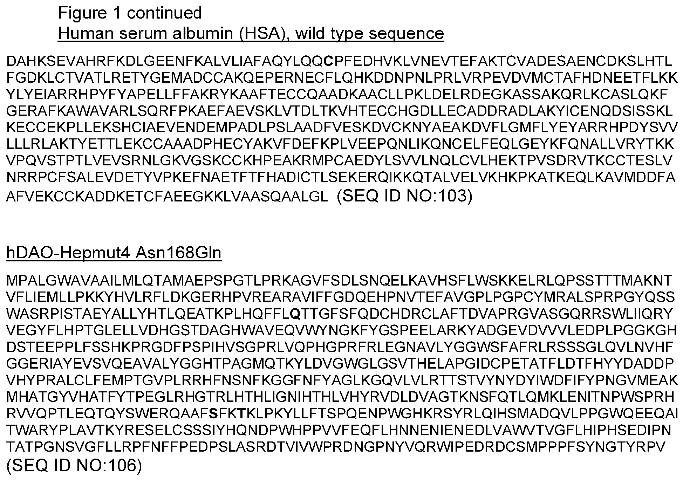

Figure 1: Amino acid and nucleotide sequences of wild type DAO and modified DAO. With reference to amino acid sequences: Bold letters refer to substitutions compared to wt sequences; underlined letters refer to secretion signal; bold, italic letters refer to Fc, bold, italic and underlined letters refer to linker sequences from IgG1 hinge region.
Figure 2: Heparin-sepharose elution profiles of recombinant human DAO wild type and heparin/heparan sulfate mutants.
Figure 3: Hepmut 1, 4 and 7 variants are eluted from heparin-sepharose at 50% lower salt concentrations compared to DAO_WT.
Figure 4: Western blot of SK-Hep1 cell lysates after incubation with DAO_WT and Hepmut variants
Figure 5: Hepmut4 variant shows reduced binding to SK-Hep1 cells compared to DAO_WT.
Figure 6: Isothermal titration calorimetry of DAO_WT and Hepmut4.
Figure 7: Linear (a) und log y-scales (b) are shown. Hepmut4 increases the AUC (Area Under the Curve) more than 19-fold compared to wild type DAO protein after intravenous injection of 1 mg/kg DAO variants.
Figure 8: Hepmut4 increases the AUC more than 16-fold compared to DAO_WT protein after intraperitoneal injection.
Figure 9: Means of the measured values using 1 mg/kg DAO wild-type and different Hepmut variants.
Figure 10: Slow clearance of heparin/heparan sulfate-binding domain mutants compared to DAO wild type protein administered at 1 mg/kg.
Figure 11: Slow clearance of heparin-binding domain mutants compared to DAO wild type protein.
Figure 12: Slow clearance of heparin-binding domain mutants compared to DAO wild type protein administered at 1 mg/kg.
Figure 13: Slow clearance of heparin-binding domain mutants compared to DAO wild type protein administered at 1 mg/kg. Linear y-axis scale.
Figure 14: Slow clearance of heparin-binding domain mutants compared to DAO wild type protein administered at 1 mg/kg. The first 90 minutes are shown; Linear y-axis scale.
Figure 15: Rapid clearance of Fc-DAO wild-type compared to Fc-Hepmut4 administered at 1 mg/kg in 6 or 4 rats respectively. The means with the standard deviations are shown; Linear y-axis scale.
Figure 16: Rapid clearance of Fc-DAO wild type compared to Fc-Hepmut4 administered at 1 mg/kg in 6 or 4 rats respectively. The means with the standard deviations are shown; Log y-axis scale.
Figure 17: Fc-DAO-Hepmut4 shows a strong increase in the AUC after intravenous administration of 1 mg/kg; Log y-axis scale.
Figure 18: Fc-DAO-Hepmut4 shows a strong increase in the AUC after intravenous administration of 1 mg/kg; Linear y-axis scale.
Figure 19: Western blot of DAO mutants.
Figure 20: No relevant difference in the DAO activity between DAO_WT and cys123 mutations.

### DETAILED DESCRIPTION

Unless indicated or defined otherwise, all terms used herein have their usual meaning in the art, which will be clear to the skilled person. Reference is for example made to standard handbooks, such as Sambrook et al, "Molecular Cloning: A Laboratory Manual" (2nd Ed.), Vols. 1 -3, Cold Spring Harbor Laboratory Press (1989); Lewin, "Genes IV", Oxford University Press, New York, (1990), and Janeway et al, "Immunobiology" (5th Ed., or more recent editions, Garland Science, New York, 2001).

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, fusion constructs, expression constructs, transformed host cells and modified proteins, are "man-made" or synthetic, and are therefore not considered as a result of "laws of nature".

The terms "**comprise**", "contain", "have" and "include" as used herein can be used synonymously and shall be understood as an open definition, allowing further members or parts or elements. "**Consisting**" is considered as a closest definition without further elements of the consisting definition feature. Thus "comprising" is broader and contains the "consisting" definition.

The term "**about**" as used herein refers to the same value or a value differing by +/-5 % of the given value.

Human DAO monomer comprises about 751 amino acids and forms dimers which are enzymatically active. Of 14 cysteines in the DAO dimer, 10 of them are involved in S-S formation and 4 are not. Specifically, cysteine 123 and 633 are not involved in disulfide bond formation.

As used herein, amino acids refer to twenty naturally occurring amino acids encoded by sixty-four triplet codons. These 20 amino acids can be split into those that have neutral charges, positive charges, and negative charges:
The "neutral" amino acids are shown below along with their respective three-letter and single-letter code and polarity:
Alanine: (Ala, A) nonpolar, neutral;
Asparagine: (Asn, N) polar, neutral;
Cysteine: (Cys, C) nonpolar, neutral;
Glutamine: (Gln, Q) polar, neutral;
Glycine: (Gly, G) nonpolar, neutral;
Isoleucine: (Ile, I) nonpolar, neutral;
Leucine: (Leu, L) nonpolar, neutral;
Methionine: (Met, M) nonpolar, neutral;
Phenylalanine: (Phe, F) nonpolar, neutral;
Proline: (Pro, P) nonpolar, neutral;
Serine: (Ser, S) polar, neutral;
Threonine: (Thr, T) polar, neutral;
Tryptophan: (Trp, W) nonpolar, neutral;
Tyrosine: (Tyr, Y) polar, neutral;
Valine: (Val, V) nonpolar, neutral; and
Histidine: (His, H) polar, positive (10%) neutral (90%).

The "positively" charged amino acids are:
Arginine: (Arg, R) polar, positive; and
Lysine: (Lys, K) polar, positive.

The "negatively" charged amino acids are:
Aspartic acid: (Asp, D) polar, negative; and
Glutamic acid: (Glu, E) polar, negative.

The term "**modification**" of the inventive DAO refers to any amino acid sequence alteration including, but not limited to, amino acid substitutions, additions, deletions, mutations, and insertions. Modifications can also be chemoselective modifications. Such modification can be a conjugation or coupling with a chemical moiety wherein a stable covalent link is formed between two molecules, at least one of which is a biomolecule. Such conjugation can be formed with one or more amino acid residues, such as, but not limited to, conjugation with maleimides, iodoacetamides, isoacetoamides, 2-thiopyridine, 3-arylpropiolonitrile, benzoyl fluorides, isothyocyanates, isocyanates, diazonium salts, PTAD, NaIO4, or PLP.

Free cysteine rarely occurs on protein surfaces and is an excellent choice for chemoselective modification. Specifically, Cys at amino acid positions 123 and 633 of the modified DAO as described herein are solvent accessible. By specifically substituting the solvent accessible cys123, specifically by exchanging cys123 to ala123, the so produced DAO becomes unable to form disulfide bonds or other molecular interactions leading to higher order aggregates. Upon expression of recombinant DAO in CHO cells certain percentages of DAO molecules (~20% to 30%) form not only dimers but also tetramers, hexamers and even octamers. These higher order oligomers have been described and can be considered to be natural variants with unknown function. It is not clear, whether they form during folding and transport from ER to Golgi and secretion into the extracellular environment or in general only in the extracellular environment. Nevertheless, these tetramers and larger oligomers complicate expression, purification, characterization, standardization and the selection of the optimal formulation of the recombinant DAO described herein. By mutating a relatively solvent accessible cysteine on the surface of DAO, specifically by mutating Cys123, only DAO dimers are found in the supernatant of the host cells, specifically of CHO cells.

In addition, according to a further specific embodiment, cysteine at amino acid position 633 may also be modified as described herein for cys123. Cys633 is also not involved in disulfide bond formation.

Under basic condition, cysteine residues can be deprotonated to generate a thiolate nucleophile, which will react with soft electrophiles, such as maleimides and iodoacetamides. As a result, a carbon-sulfur bond is formed. Another modification of cysteine residues involves the formation of disulfide bond. The reduced cysteine residues react with exogenous disulfides, generating new disulfides bond on protein. An excess of disulfides is often used to drive the reaction, such as 2-thiopyridone and 3-carboxy-4-nitrothiophenol. Electron-deficient alkynes were demonstrated to selectively react with cysteine residues of proteins in the presence of other nucleophilic amino acid residues. Depending on the alkyne substitution, these reactions can produce either cleavable (when alkynone derivatives are used), or hydrolytically stable bioconjugates (when 3-arylpropiolonitriles are used).

Within the term modification also the substitution of natural amino acids with **unnatural amino acids** is encompassed. Unnatural amino acids are not encoded by the Universal Genetic Code. Usually they can be found in nature as metabolic products, especially in plants and bacteria. Such unnatural amino acids can be selected from, but are not limited to D-amino acids, homo amino acids, N-methyl amino acids, alpha methyl amino acids, beta² amino acids, beta³ amino acids, beta³ homo amino acids, ACHC, peptoids or heavy amino acids, specifically substituted with 13C and/or 15N atoms, specifically E-acetyl-lysine, alanine(3-amino-proprionic acid), 6-aminocaproic acid, ?-aminobutyric acid, citrulline, cysteine acetamidomethyl protected, dimethyl-lysine, hydroxyl-proline, mercaptoproprionic acid, methyl-lysine, 3-notro-tyrosine, norleucines, pyro glutamic acid, carbobenzoxyl.

The term "**GAG binding domain**" as used herein refers to the region with the DAO which is involved in binding to or interaction with all four classes of glycosaminoglycans, including heparin/heparan sulfate, chondroitin/dermatan sulfate, keratin sulfate, and hyaluronan. Binding to heparin/heparan sulfate is preferred. Heparin is present in mast cells. Heparan sulfate is present on almost all cells such as endothelial cells.

The term "**GAG binding**" refers to the interaction/binding of DAO with glycosaminoglycan, specifically with heparin or heparan sulfate. GAGs bind to many different classes of proteins mostly through electrostatic interactions between negatively charged sulfate groups and uronic acids and positively charged amino acids in the protein. Heparin binding affinity can be determined by any method known to the skilled person. Numerous methods are available for analyzing GAG-protein interactions, and some provide a direct measurement of K_{d} values. A common method involves affinity fractionation of proteins on sepharose columns containing covalently linked GAG chains, usually heparin. The bound proteins are eluted with different concentrations of sodium chloride, and the concentration required for elution is generally proportional to the K_{d}. High-affinity interactions require at least 1 M NaCl to displace bound ligand, which translates into K_{d} values of 10⁻⁷-10⁻⁹ M (determined under physiological salt concentrations by equilibrium binding). Proteins with low affinity (10⁻⁴-10⁻⁶ M) either do not bind under "normal" conditions (0.15 M NaCl) or require only 0.3-0.5 M NaCl to elute. This method is based on the assumption that GAG-protein interaction is entirely ionic and can provide an assessment of relative affinity, when comparing different GAG-binding proteins. Alternative methods can be, but are not limited to affinity co-electrophoresis, analytical ultracentrifugation, circular dichroism, competition ELISA, fluorescence microscopy, ion mobility mass spectrometry, isothermal titration calorimetry, laser light scattering, NMR, surface plasmon resonance, and X-ray and thereby provide detailed thermodynamic data (Δ*H* [change in enthalpy], Δ*S* [change in entropy], Δ*Cp* [change in molar heat capacity], etc.), kinetic data (association and dissociation rates), and high-resolution data on atomic contacts in GAG-protein interactions (Esko JD. et al., Essentials of Glycobiology, 3rd edition, Chapter 38, 2017).

Specifically, the DAO mutants as described herein have diminished or show reduced binding to heparin and/or heparan sulfate. Specifically, heparin/heparan sulfate binding affinity of the recombinant DAO is at least 10%, 25%, 50%, 60%, 70%, 80%, specifically 90% reduced compared to wild type DAO. According to a specific method, binding affinity is determined using heparin-sepharose chromatography, wherein the DAO variants are incubated at low salt concentrations and eluted with increasing salt concentrations. The salt concentrations with the peak in DAO protein (measured using absorbance at 280 nm) is used as the mM salt concentration at which DAO is eluted.

The DAO of the invention further show decreased internalization into endothelial cells compared to wild type DAO which is internalized. Specifically, internalization by endothelial cells is at least 10%, 25%, 50%, 60%, 70%, 80%, specifically 90% reduced compared to wild type DAO.

Specifically, the GAG binding domain comprises amino acids at positions 568-575 with reference to the numbering of SEQ ID NO:1. Within said domain, 1, 2, 3, 4, 5, 6, 7, or all amino acids can be modified.

A DAO monomer contains 24 lysines and 44 arginines in the primary amino acid sequence, most of them being on the surface of the molecule. It is likely that other lysines and arginines are involved in heparin binding. Besides the GAG binding domain encompassing amino acids at positions 568-575 further lysines or arginines on the surface of DAO may be involved in heparin/heparan sulfate binding.

Modification of one or more of these lysines and/or arginines may further decrease heparin/heparan sulfate binding of the recombinant DAO.

The term "**enzymatic activity**" of DAO refers to the polypeptide's ability to catalyze the oxidative deamination of an appropriate substrate like putrescine or histamine to aminobutyraldehyde or imidazole acetaldehyde. Preservation of enzymatic activity means that the DAO of the invention has the same or similar enzymatic activity as the respective wild type DAO. Enzymatic activity that is at least 80%, specifically at least 90% of the wild type activity is interpreted to be the similar enzymatic activity as a wild type DAO.

For determining enzymatic activity of DAO, any methods can be used known in the art. These methods can be, but are not limited to assays using horseradish peroxidase (HRP)-mediated luminol oxidation (Bartko J. et al., Alcohol. 2016 Aug;54:51-9), spectrophotometric methods as described by Holmstedt B.O. and Tham R. (Acta Physiol. Scand., 1959, 45, 152-163) and Bardsley W.G. et al., (Biochem.J. 1972, 127, 875-879), mass spectrometry (Gludovacz E. et al., 2016), liquid scintillation counting (Okuyama T. and Kobayashi Y., Archives Biochem. Biophys., 19661, 95, 242-250), titrimetric, manometric, fluorometric, biological or radioactive assay methods (Zeller EA., The enzymes, (J.B. Sumner and K Maybäck, eds.) Vol II, Part A, p. 536, Academic Press, New York 1951; Shore P.A. et al., J.Pharmacol.,Exptl.Therap. 127, 1959, 182; Ahlark A., Acta Physiol.Scand.Suppl., 1944, 28, 9)

The term "**functional variant**" or "**functionally active variant**" also includes naturally occurring allelic variants, as well as mutants or any other non-naturally occurring variants. As is known in the art, an allelic variant is an alternate form of a nucleic acid or peptide that is characterized as having a substitution, deletion, or addition of one or more nucleotides or one or more amino acids that does essentially not alter the biological function of the nucleic acid or polypeptide.

Functional variants may be obtained by sequence alterations in the polypeptide or the nucleotide sequence, e.g. by one or more point mutations, wherein the sequence alterations retain or improve a function of the unaltered polypeptide or the nucleotide sequence, when used in combination of the invention. Such sequence alterations can include, but are not limited to, (conservative) substitutions, additions, deletions, mutations and insertions. Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

A **point mutation** is particularly understood as the engineering of a polynucleotide that results in the expression of an amino acid sequence that differs from the non-engineered amino acid sequence in the substitution or exchange, deletion or insertion of one or more single (non-consecutive) or doublets of amino acids for different amino acids.

In an alternative embodiment, a GAG binding domain can be introduced into DAO at any position within the polypeptide by recombinant means. The respective domain can be comprised of amino acid sequence X1FX2X3X4LPX5, X1 being any amino acid, specifically being A or S, more specifically being S; X2 being any amino acid, specifically being K; X3 being any amino acid, specifically A or T, more specifically T, X4 being any amino acid, specifically K, and X5 being any amino acid, specifically K or T, more specifically T. In a specific embodiment one or more of the amino acid sequences SFKAKLPK (SEQ ID NO:33), AFKAKLPT (SEQ ID NO:34), AFKTKLPK (SEQ ID NO:35), SFKTKLPK (SEQ ID NO:36), AFKTKLPT (SEQ ID NO:37), SFKAKLPK (SEQ ID NO:38) are introduced into the DAO polypeptide described herein.

The term "**sequence identity**" as used herein is understood as the relatedness between two amino acid sequences or between two nucleotide sequences and described by the degree of sequence identity or sequence complementarity. The sequence identity of a variant, homologue or orthologue as compared to a parent nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%.

Sequence similarity searching is an effective and reliable strategy for identifying homologs with excess (*e.g.,* at least 50%) sequence identity. Sequence similarity search tools frequently used are *e.g.,* BLAST, FASTA, and HMMER.

Sequence similarity searches can identify such homologous proteins or polynucleotides by detecting excess similarity, and statistically significant similarity that reflects common ancestry. Homologues may encompass orthologues, which are herein understood as the same protein in different organisms, e.g., variants of such protein in different different organisms or species.

To determine the % complementarity of two complementary sequences, one of the two sequences needs to be converted to its complementary sequence before the % complementarity can then be calculated as the % identity between the first sequence and the second converted sequences using the above-mentioned algorithm.

"**Percent (%) identity**" with respect to an amino acid sequence, homologs and orthologues described herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

For purposes described herein, the sequence identity between two amino acid sequences is determined using the NCBI BLAST program version 2.2.29 (Jan-06-2014) with blastp set at the following exemplary parameters: Program: blastp, Word size: 6, Expect value: 10, Hitlist size: 100, Gapcosts: 11.1, Matrix: BLOSUM62, Filter string: F, Genetic Code: 1, Window Size: 40, Threshold: 21, Composition-based stats: 2.

"**Percent (%) identity**" with respect to a nucleotide sequence e.g., of a nucleic acid molecule or a part thereof, in particular a coding DNA sequence, is defined as the percentage of nucleotides in a candidate DNA sequence that is identical with the nucleotides in the DNA sequence, after aligning the sequence and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for measuring alignment, including any algorithms needed to achieve maximal alignment over the full length of the sequences being compared.

Optimal alignment may be determined with the use of any suitable algorithm tor aligning sequences, non-limiting examples of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at novocraft.com), ELAND (Illumina, San Diego, CA), SOAP (available at soap.genomies.org.cn), and Maq (available at maq.sourceforge.net).

The DAO as described herein can comprise the amino acid sequences of SEQ ID NOs:2 to 16 and any functional variants thereof having 90%, 95%, 99% sequence identity with any of SEQ ID NOs:2 to 16.

The recombinant DAO has increased plasma half-life compared to wild type DAO, specifically said half-life is increased at least 1.5 fold, specifically at least 2 fold compared to wild type DAO.

The duration of action or physical presence of a drug is known as its half-life. This is the period of time required for the concentration or amount of drug in the body or whole blood or plasma to be reduced by one-half. Half-life of a drug is usually considered in relation to the amount of the drug in plasma or serum. A drug's plasma or serum half-life depends on how quickly the drug is eliminated from the plasma or serum. A drug molecule may be eliminated from the body, or it can be translocated to another body fluid compartment such as the intracellular fluid or it can be destroyed in the blood. The removal of a drug from the plasma is known as clearance and the distribution of the drug in the various body tissues is known as the volume of distribution.

The **area under the plasma drug concentration-time** curve (**AUC**) reflects the actual body exposure to drug, i.e. the recombinant DAO described herein, after administration of a dose of the drug and is expressed in µg/min/ml. This area under the curve is dependent on the rate of elimination of the drug from the body and the dose administered. The total amount of drug eliminated by the body may be assessed by adding up or integrating the amounts eliminated in each time interval, from time zero (time of the administration of the drug) to infinite time. This total amount corresponds to the fraction of the dose administered that reaches the systemic circulation. The AUC is directly proportional to the dose when the drug follows linear kinetics. The AUC is inversely proportional to the clearance of the drug. That is, the higher the clearance, the less time the drug spends in the systemic circulation and the faster the decline in the plasma drug concentration. Therefore, in such situations, the body exposure to the drug and the area under the concentration-time curve are smaller.

The recombinant DAO as described herein has an at least 2-fold, at least 5-fold, 10-fold, at least 15-fold, at least 20-fold, at least 25-fold, at least 30-fold increased AUC compared to wild type DAO.

The term "**expression**" is understood in the following way. Nucleic acid molecules containing a desired coding sequence of an expression product such as e.g., a fusion protein as described herein may be used for expression purposes. Hosts transformed or transfected with these sequences are capable of producing the encoded proteins. In order to effect transformation, the expression system may be included in a vector; however, the relevant DNA may also be integrated into the host chromosome. Specifically, the term refers to a host cell and compatible vector under suitable conditions, *e.g.,* for the expression of a protein coded for by foreign DNA carried by the vector and introduced to the host cell.

Coding DNA is a DNA sequence that encodes a particular amino acid sequence for a particular polypeptide or protein. Promoter DNA is a DNA sequence which initiates, regulates, or otherwise mediates or controls the expression of the coding DNA. Promoter DNA and coding DNA may be from the same gene or from different genes, and may be from the same or different organisms. Recombinant cloning vectors often include one or more replication systems for cloning or expression, one or more markers for selection in the host, *e.g.,* antibiotic resistance, one or more nuclear localization signals (NLS) and one or more expression cassettes.

"**Expression vector**" as used herein is defined as DNA sequences that are required for the transcription of cloned recombinant nucleotide sequences, *i.e.* of recombinant genes and the translation of their mRNA in a suitable host organism. To obtain expression, a sequence encoding a desired expression product, such as the DAO described herein, is typically cloned into an expression vector that contains a promoter to direct transcription. Suitable bacterial and eukaryotic promoters are well known in the art. The promoter used to direct expression of a nucleic acid depends on the particular application. For example, a strong constitutive promoter is typically used for expression and purification of fusion proteins. In contrast, when the expression product is to be administered in vivo for gene regulation, either a constitutive or an inducible promoter can be used, depending on the particular use of the expression product. In addition, a preferred promoter for administration can be a weak promoter. The promoter can also include elements that are responsive to transactivation, e.g., hypoxia response elements, Gal4 response elements and lac repressor response elements. Expression vectors comprise the expression cassette and additionally usually comprise an origin for autonomous replication in the host cells or a genome integration site, one or more selectable markers (*e.g.,* an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together.

An "**expression cassette**" refers to a DNA coding sequence or segment of DNA coding for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct".

Any suitable host cell or cell line can be used for expressing the recombinant DAO which allows proper folding, post-translational modifications, and enzymatic activity. Specifically, recombinant host cells may be selected from CHO cells, COS cells, Vero cells, MDCK cells, Pichia pastoris cells, SF9 cells, human cell lines such as HEK and HeLa.

The term "**vector**" as used herein includes autonomously replicating nucleotide sequences as well as genome integrating nucleotide sequences. A common type of vector is a "plasmid", which generally is a self-contained molecule of double-stranded DNA that can readily accept additional (foreign) DNA and which can readily be introduced into a suitable host cell. A plasmid vector often contains coding DNA and promoter DNA and has one or more restriction sites suitable for inserting foreign DNA. Specifically, the term "vector" or "plasmid" refers to a vehicle by which a DNA or RNA sequence (*e.g*., a foreign gene) can be introduced into a host cell, so as to transform the host and promote expression (*e.g.,* transcription and translation) of the introduced sequence. Vectors are transfected into the cells and the DNA may be integrated into the genome by homologous recombination in the case of stable transfection, or the cells may be transiently transfected. Specifically the vector is a bacterial, yeast, baculoviral, plant or mammalian expression vector.

Any of the known procedures for introducing foreign nucleotide sequences into host cells may be used. These include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell (see, e.g., Sambrook et al.).

Examples of mammalian expression vectors include the adenoviral vectors, the pSV and the pCMV series of plasmid vectors, vaccinia and retroviral vectors, as well as baculovirus. The promoters for cytomegalovirus (CMV) and SV40 are commonly used in mammalian expression vectors to drive gene expression. Non-viral promoter, such as the elongation factor (EF)-1 promoter, is also known.

Using above described vectors and host cells, the present invention provides a method for producing the recombinant DAO comprising the sequential steps of cloning a nucleotide sequence encoding the DAO into an expression vector, transforming a host cell, specifically a mammalian cell with said vector, cultivating the transformed host cell under conditions wherein the DAO is expressed, isolating the DAO from the host cell culture, optionally by disintegrating the host cells or isolating the DAO from cell culture supernatant, and optionally purifying the DAO.

Further described is a pharmaceutical composition, comprising the recombinant DAO provided herein. According to a specific embodiment, such pharmaceutical composition comprising the DAO or its functional variants as described herein is used for the treatment of any condition associated with excess histamine, specifically of excess histamine of >1 ng/ml plasma concentration, specifically for the treatment of chronic allergic diseases, more specifically for the treatment of anaphylaxis, anaphylactic shock, chronic urticaria, acute urticaria, asthma, hay fever, allergic rhinitis, allergic conjunctivitis, histamine intoxication, headache, itching, vomiting, tachycardia, hypotension, cardiac arrest, atopic dermatitis inflammatory diseases, mastocytosis, mast cell activation syndrome (MCAS), pre-eclampsia, hyperemesis gravidarum, pre-term labor, peptic ulcers, acid reflux, pruritus, and sepsis.

Specifically, the pharmaceutical composition described herein further comprises pharmaceutically acceptable carriers or excipients, such as for example bulking agents, when used for diagnosis or therapy. These pharmaceutical compositions can be administered in accordance with the present invention as a bolus injection or infusion or by continuous infusion. Pharmaceutical carriers suitable for facilitating such means of administration are well-known in the art.

Pharmaceutically acceptable carriers generally include any and all suitable solvents, dispersion media, coatings, isotonic and absorption delaying agents, and the like that are physiologically compatible with the DAO provided by the invention. Further examples of pharmaceutically acceptable carriers include sterile water, saline, phosphate buffered saline, dextrose, glycerol, ethanol, and the like, as well as combinations of any thereof.

Additional pharmaceutically acceptable carriers are known in the art and described in, *e.g.,* Remington's Pharmaceutical Sciences (Gennaro, AR, ed., Mack Publishing Co, 1985). Liquid formulations can be solutions, emulsions or suspensions and can include excipients such as suspending agents, solubilizers, surfactants, preservatives, and chelating agents.

Exemplary formulations as used for parenteral administration include those suitable for subcutaneous, intramuscular or intravenous injection as, for example, a solution, emulsion or suspension.

The DAO described herein is specifically administered at a therapeutically effective amount, meaning a quantity or activity sufficient to effect beneficial or desired results, including clinical results, when administered to a subject, e.g. a patient suffering from cancer. As such, an effective amount or synonymous quantity thereof depends upon the context in which it is being applied. An effective amount is intended to mean that amount of a compound that is sufficient to treat, prevent or inhibit such diseases or disorders.

The amount of the compound, i.e. the recombinant DAO described herein, that will correspond to such an effective amount will vary depending on various factors, such as the given drug or compound, the pharmaceutical formulation, the route of administration, the type of disease or disorder, the identity of the subject or host being treated, and the like, but can nevertheless be routinely determined by one skilled in the art.

According to a specific embodiment of the invention, a fusion polypeptide is provided wherein the recombinant DAO is conjugated to a second moiety, which can be, but is not limited to Fc or human serum albumin (HSA).

According to a specific embodiment, the DAO conjugated to an Fc comprises any one of SEQ ID NOs: 40 to 70 or a functional fragment thereof having at least 80%, specifically at least 85%, 90%, 95%, 99% sequence identity with any one of SEQ ID NOs:40 to 70.

According to a specific embodiment, the DAO conjugated to an Fc is encoded by any one of SEQ ID NOs: 72 to 102 or by a fragment thereof having at least 80%, specifically at least 85%, 90%, 95%, 99% sequence identity with any one of SEQ ID NOs:72 to 102.

The term "**fusion polypeptide**" in the context of the present invention concerns a sequence of amino acids, predominantly (but not necessarily) connected to each other by peptide bonds. The term "fused" in accordance with the fusion polypeptide of the present invention refers to the fact that the amino acid sequences of at least two different origins, namely, the modified DAO as herein defined and the second moiety, specifically the Fc domain of human IgG or albumin, are linked to each other by covalent bonds either directly or via an amino acid linker or spacer, joining (bridging, conjugating, covalently binding) the amino acid sequences. The fusion may be performed by chemical conjugation or by genetic engineering methods that are well known in the art.

In some embodiments the DAO polypeptide as herein defined is covalently linked through its C-terminus to either the Fc domain of human IgG or to HSA. Namely, in some embodiments, in the N- to C-terminal direction, the fusion polypeptide according to the invention comprises the DAO polypeptide and either the Fc domain component or HSA.

In other embodiments the DAO polypeptide as herein defined is covalently linked through its N-terminus to either the Fc domain of human IgG or to HSA. Namely, in some embodiments, in the N- to C-terminal direction, the fusion polypeptide of the invention comprises the Fc domain component or HSA and the DAO polypeptide.

The term "**polypeptide**" as used herein refers to amino acid residues, connected by peptide bonds. A polypeptide sequence is generally reported from the N-terminal end containing free amino group to the C-terminal end containing free carboxyl group. A polypeptide may also be termed amino acid sequence, peptide, or protein and can be modified, for example, by manosylation, glycosylation, amidation, carboxylation or phosphorylation.

By the term "**covalently linked**" or "covalently linking" it is meant that the indicated domains are connected or linked by covalent bonds.

Specifically, as used herein, the term "**Fc fusion polypeptide**" encompasses the DAO of the present disclosure comprising a full length Fc domain as well as proteins comprising Fc domain fragments (e.g., a full CH2 domain, a full CH3 domain, a CH2 fragment, a CH3 fragment, or combinations thereof). An Fc fusion protein may also comprise all or a portion of the hinge region.

As used herein the Fc region includes the polypeptides comprising the constant region of an antibody excluding the first constant region immunoglobulin domain, and fragments thereof. Thus Fc refers to the last two constant region immunoglobulin domains of IgA, IgD, and IgG, and the last three constant region immunoglobulin domains of IgE and IgM, and optionally the flexible hinge region N-terminal to these domains. For IgA and IgM the Fc region can include the J chain. For IgG, Fc comprises immunoglobulin domains Cgamma2 and Cgamma3 (Cy2 and Cy3) and optionally the hinge region between Cgammal (Cy1) and Cgamma2 (Cy2). Although the boundaries of the Fc region can vary, the human IgG heavy chain Fc region is usually defined to comprise residues C226 or P230 to its carboxyl-terminus, wherein the numbering is according to the EU index as set forth in Kabat (Kabat et al., Sequences of Proteins of Immunological Interest, 5th Ed. Public Health Service, National Institutes of Health, Bethesda, Md. (1991)). Fc can refer to this region in isolation, or this region in the context of an antibody, antibody fragment, or Fc fusion protein.

The HSA sequence fused to the DAO polypeptide described herein can comprise the wild type sequence or 90%, specifically at least 95%, more specifically at least 99%, more specifically at least 99.9% sequence identity with the wild type sequence (SEQ ID No. 103). Optionally a linker sequence is contained between DAO and HSA, specifically comprising 2 to about 10 amino acid residues.

According to a specific embodiment, GAG binding of DAO, specifically heparin/heparan sulfate binding is inhibited thus leading to decreased internalization of DAO and increased amount of DAO in the body's circularization which can be determined by AUC. By modifying the DAO as described herein, the DAO indeed shows significantly decreased heparin/heparan sulfate binding.

A further option to increase DAO in the body's circularization is to provide a target-specific ligand specifically binding to the heparin/heparan sulfate binding domain. Specifically, an antibody or antibody fragment or any ligand may bind to DAO close to the heparin binding domain and thereby blocks access to the heparin binding domain. Alternatively it is a fusion protein where parts of the fusion protein cover and block the function of the heparin binding domain and therefore have the same effect as mutations or antibody binding directly to the heparin binding domain.

As an alternative, heparin/heparan sulfate binding to the GAG binding domain of DAO is inhibited. The ligand can be an antigen binding protein, specifically selected from the group consisting of antibodies or antibody fragments, such as any of Fab, Fd, scFv, diabodies, triabodies, Fv tetramers, minibodies, nanobodies, single-domain antibodies like VH, VHH, IgNARs, or V-NAR; antibody mimetics, such as Adnectins^{™} (sharing with antibody variable domains a beta-sheet sandwich fold with diversified loops, but differing from antibodies in primary sequence and having a single-domain structure without disulfide bonds), Affibodies^{®} (small, simple proteins composed of a three-helix bundle based on the scaffold of one of the IgG-binding domains of Protein A), Affilins^{®} (structurally derived from human ubiquitin, constructed by modification of surface-exposed amino acids of these proteins and isolated by display techniques such as phage display and screening. Affilins resemble antibodies in their affinity and specificity to antigens but not in structure, which makes them a type of antibody mimetic), Affimers^{®} (small proteins that bind to target molecules with similar specificity and affinity to that of antibodies), Affitins (artificial proteins with the ability to selectively bind antigens), Alphabodies, Aptamers, Anticalins, Avimers, DARPins^{®} (genetically engineered antibody mimetic proteins typically exhibiting highly specific and high-affinity target protein binding), Fynomers^{®} (small binding proteins (7 kDa) derived from the human SH3 domain of Fyn kinase which can be engineered to yield specific and high-affinity binding domains to target the specific proteins), Kunitz domain peptides, monobodies, or NanoCLAMPS (CLostridal Antibody Mimetic Proteins); or fusion proteins comprising one or more immunoglobulin-fold domains, antibody domains or antibody mimetics.

Further described is a method for identifying compounds which modulate the heparin binding of the DAO, comprising the steps of
(a) constructing a computer model of the GAG binding domain defined by the structure coordinates of the amino acids of the DAO sequence of SEQ ID No. 1,
(b) selecting a potential modulating compound by a method selected from the group consisting of:
   (i) assembling molecular fragments into said compound,
   (ii) selecting a compound from a small molecule database, and
   (iii) *de novo* ligand design of said compound;
(c) employing computational means to perform a fitting program operation between computer models of the said compound and the GAG binding domain in order to provide an energy-minimized configuration of the said compound in the heparin binding domain; and
(d) evaluating the results of said fitting operation to quantify the association between the said compound and the heparin/heparan sulfate binding domain, thereby evaluating the ability of said compound to associate with the said heparin/heparin sulfate binding domain.

The term "**structure coordinates**" refers to a set of values that define the position of one or more amino acid residues with reference to a system of axes. The term refers to a data set that defines the three-dimensional structure of a molecule or molecules (e.g., Cartesian coordinates, temperature factors, and occupancies). Structural coordinates can be slightly modified and still render nearly identical three-dimensional structures. A measure of a unique set of structural coordinates is the root mean square deviation of the resulting structure. Structural coordinates that render three-dimensional structures (in particular, a three-dimensional structure of a heparin/heparin sulfate binding domain) that deviate from one another by a root mean square deviation of less than 3 Å, 2 Å, 1.5 Å, 1.0 Å, or 0.5 Å may be viewed by a person of ordinary skill in the art as very similar.

As used herein, the term "**constructing a computer model**" includes the quantitative and qualitative analysis of molecular structure and/or function based on atomic structural information and interaction models. The term "modeling" includes conventional numeric-based molecular dynamic and energy minimization models, interactive computer graphic models, modified molecular mechanics models, distance geometry, and other structure-based constraint models.

The term "**fitting program operation**" refers to an operation that utilizes the structure coordinates of a chemical entity, an enzymatically active center, a binding pocket, molecule or molecular complex, or portion thereof, to associate the chemical entity with the enzymatically active center, the binding pocket, molecule or molecular complex, or portion thereof. This may be achieved by positioning, rotating or translating the chemical entity in the enzymatically active center to match the shape and electrostatic complementarity of the enzymatically active center. Covalent interactions, non-covalent interactions such as hydrogen bond, electrostatic, hydrophobic, van der Waals interactions, and non-complementary electrostatic interactions such as repulsive charge-charge, dipole-dipole and charge-dipole interactions may be optimized. Alternatively, one may minimize the deformation energy of binding of the chemical entity to the enzymatically active center.

### EXAMPLES

### Example 1

### DAO with modified GAG binding domain:

Summary: Amino acids in the GAG binding (heparin/heparan sulfate-binding domain) of DAO were mutated. After mutations in the heparin-binding domain of DAO, animal studies were performed with these mutants. The short alpha half-life could be almost eliminated and the beta half-life increased to 6 hours in rats. The alpha half-life refers to the rate of decline in plasma concentrations due to the process of drug redistribution from the central to the peripheral compartment, the beta half-life refers to the rate of decline due to the process of drug elimination due to metabolism or excretion. The area under curve (AUC) increased more than 20-fold. A half-life of 6 hours in rats extrapolates to 24 to 48 hours in humans and this is certainly sufficient for the treatment of acute and subacute conditions with excess histamine. For example, anaphylaxis or MCAS events last a few hours to 1 to 2 days, and anaphylaxis may be biphasic in 10-20% of patients meaning that a second episode may occur within 24 hours. Several mutations have been tested, and a distinct double mutant in the heparin-binding domain showed the most pronounced improvements in the pharmacokinetic (PK) parameters. Because DAO is a dimer and the heparin-binding domain forms a ring structure composed of both monomers, four mutations are located closely to each other. Nevertheless, expression, stability and activity of DAO wild type and DAO mutants are identical.

### DAO with modified cysteine

When DAO were expressed in CHO cells, certain percentages of DAO molecules (~20% to 30%) formed not only dimers but also tetramers, hexamers and even octamers. These higher order oligomers can be considered to be natural variants with unknown function. It is not clear, whether they form during folding and transport from ER to Golgi and secretion into the extracellular environment or in general only in the extracellular environment. Nevertheless, these tetramers and larger oligomers complicate expression, purification, characterization, standardization and the selection of the optimal formulation of rhDAO. Therefore a relatively solvent accessible cysteine on the surface of DAO was mutated and in this mutant only DAO dimers are found in the supernatant of CHO cells.

DAO constructs, having 2 point mutations in the heparin/heparan sulfate-binding domain and a single mutation in a distinct cysteine are specific embodiments.

The following table shows recombinant DAO. Threonine and serine mutations were preferred over alanine and glycine to exchange lysine and arginine with polar amino acids. This helped to keep the confirmation unaltered.

**Table 1: DAO Mutations in the GAG binding domain with exchange of arginine and lysine to serine and threonine**

| | | Amino Acid | | | | |
|---|---|---|---|---|---|---|
| DAO Variant | | 568 | 570 | 571 | 572 | 575 |
| WT | | Arginine | Lysine | Arginine | Lysine | Lysine |
| Hepmut1 | Arg_568_Ser | Serine | Lysine | Arginine | Lysine | Lysine |
| Hepmut2 | Lys_575_Thr | Arginine | Lysine | Arginine | Lysine | Threonine |
| Hepmut3 | | Arginine | Lysine | Threonine | Lysine | Lysine |
| Hepmut4 | Arg_568_Ser; Arg_571_Thr | Serine | Lysine | Threonine | Lysine | Lysine |
| Hepmut5 | | Arginine | Lysine | Threonine | Lysine | Threonine |
| Hepmut7 | Arg_568_Ser; Lys_575_Thr | Serine | Lysine | Arginine | Lysine | Threonine |

The Hepmut4 mutation showed the strongest loss of binding to heparin. Hepmut6 is a triple mutation replacing 570/571/572 with Gly/Gln/Thr, which is present in rodents. The glutamine in the rodent sequence can also bind negatively charged sulfate and can sometimes replace arginine or lysine. All animal experiments were done in rodents so far (mice and rats).

Below are data from heparin-sepharose experiments using KCI and NaCl to elute purified DAO wild type and Hepmut variants from heparin-sepharose. Heparin is irreversibly coupled to sepharose and purified DAO is incubated at low salt concentrations followed by a linear gradient of increasing concentrations of KCI or NaCl. The salt concentrations with the peak in DAO protein (measured using absorbance at 280 nm) is used as the mM salt concentration at which DAO is eluted.

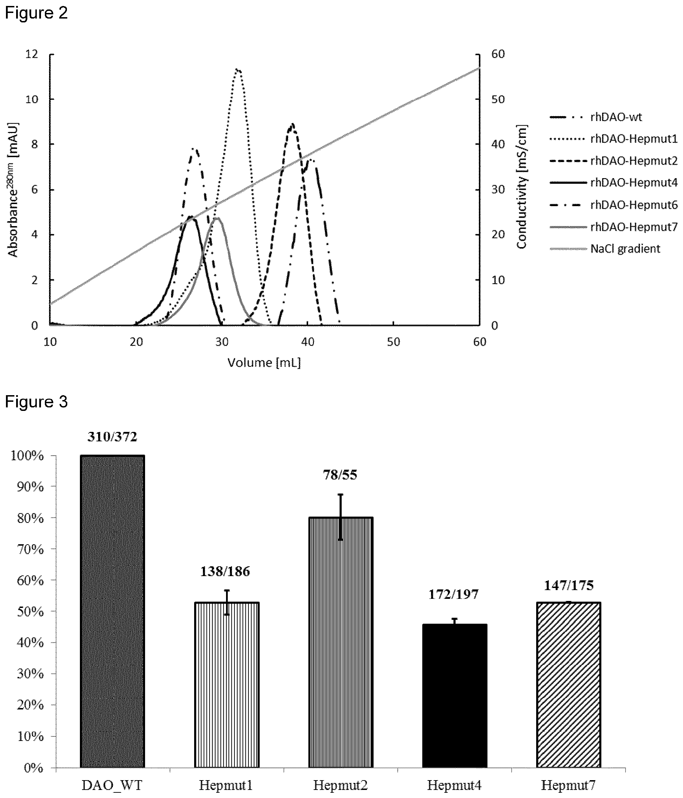

Figure 2 shows the Heparin-sepharose elution profiles of recombinant human DAO wild type (WT) and heparin mutants. Hepmut variants are eluted earlier compared to DAO_WT, Purified DAO_WT and hepmut variants were loaded onto a HiTrap Heparin HP (High Performance) 1 ml column using a 50 mM HEPES buffer, pH 7.4 and a flow rate of 0.2 ml/min. Elution was performed using a linear gradient of 0% to 70% 50 mM HEPES with 1 M sodium chloride, pH 7.4 with a gradient time of 60 minutes; rh=recombinant human. For KCI elution purified rhDAO variants were loaded onto a HiTrap Heparin HP 1 ml column using a 10 mM potassium phosphate buffer, pH 7.2 and a flow rate of 0.2 ml/min. Elution was performed using a linear gradient of 0% to 100% 10 mM potassium phosphate buffer with 1M potassium chloride, pH 7.2 with a gradient time of 60 minutes. The elution profiles are not shown but the salt concentrations at peak elution are shown below.

Table 2 and figure 3 show the raw results but also normalized based on data using the DAO wild-type protein. The mean and the standard deviation (SD) from the two salt experiments were calculated and presented as bar graph. The numbers above the bars correspond to the numbers in the table. For DAO_WT they represent the salt concentration of elution. For the mutations the difference to the DAO_WT salt concentration is shown.

DAO_WT was eluted from the heparin sepharose at 372 mM NaCl or 310 mM KCI salt concentration. Hepmuts were eluted at significantly lower salt concentrations. The correlation coefficient R between the NaCl and KCI elution profiles is 97% with a p-value of 0.0056. Therefore, it seemed justified to combine both profiles and calculate a mean with SD. The mean plus/minus SD are represented by the bar height (mean) and by the error bars (plus/minus SD).

**Table 2: Elution of DAO_WT and hepmut variants from heparin-sepharose at different NaCl or KCI concentrations**

| | **KCL Conc. [mM]** | **NaCl Conc. [mM]** | **DAO_WT binding KCI %** | **DAO_WT binding NaCl %** | **Mean %** | **SD %** |
|---|---|---|---|---|---|---|
| **DAO_WT** | 310 | 372 | 100% | 100% | 100% | 0,0% |
| **Hepmut1** | 172 | 186 | 56% | 50% | 53% | 3,9% |
| **Hepmut2** | 233 | 317 | 75% | 85% | 80% | 7,3% |
| **Hepmut4** | 138 | 175 | 44% | 47% | 46% | 1,8% |
| **Hepmut7** | 164 | 197 | 53% | 53% | 53% | 0,1% |
| WT = Wild-Type; Conc. = Concentration; SD = Standard Deviation | | | | | | |

Figure 3 shows Hepmut 1, 4 and 7 variants that are eluted from heparin-sepharose at 50% lower salt concentrations compared to DAO_WT. The data are from Table 2.

Table 3 shows the delta salt concentration from DAO_WT versus mutants for NaCl and KCI. For example 200 mM means that 200 mM less NaCl were necessary to elute the mutant from the heparin-sepharose compared to DAO_WT.

**Table 3: Hepmut variants are eluted at significantly lower NaCl or KCI concentrations compared to DAO wild-type protein from heparin-sepharose**

| | **Delta mM KCI DAO** | **Delta mM NaCl DAO** |
|---|---|---|
| **DAO_WT** | 0 | 0 |
| **Hepmut1** | 138 | 186 |
| **Hepmut2** | 78 | 55 |
| **Hepmut4** | 172 | 197 |
| **Hepmut7** | 147 | 175 |

Based on these data Hepmut4 might be the weakest heparin-binding mutant but the difference to Hepmut1 and Hepmut7 is not significant. Mutating a single lysine residue in Hepmut2 reduces the affinity to heparin-sepharose.

Serum and plasma contain about 145 to 150 mM Na+ and K+ combined and 100 mM Cl- ions. Elution of Hepmut1, 4 and 7 variants from the heparin-sepharose is close to the physiological concentration of ions in serum and therefore these mutants should be minimally active in vivo and this is the case as shown below in vitro using cells and in vivo in rats and in mice.

These data clearly show that DAO binds heparin via the heparin/heparan sulfate binding domain. To place these results in a bigger context, mutation of 1 to 2 arginine/lysine residues among 48 lysine and 88 arginine amino acids in the DAO sequence (and most of them are surface exposed) almost abolish heparin binding, although using Hepmut4 with two arginine mutations only 2/88 = 2.3% of arginine residues have been mutated.

The clearance of DAO_WT in mice and rats is significantly faster compared to Hepmut variants. It was assumed that the heparin-binding domain is involved in the reduced Area Under the Curve (AUC) values. Heparin-binding mutants show elution from the heparin-sepharose at lower salt concentrations implying the heparin or heparan sulfate binding is reduced.

In the next set of experiments it was discovered that DAO does not only bind to but is actually internalized into endothelial cells and shows vesicular staining inside cells. Hepmut variants are not internalized anymore by endothelial cells. Endothelial cells are the first cells (except blood cells) exposed to DAO after intravenous administration. They are in direct contact with blood. They also exposed to DAO after subcutaneous administration because DAO will be transported via the lymph system into the blood compartment.

Immunofluorescence microscopy of SK-Hep1 cells, an endothelial cell-like immortal cell line from a patient with liver cancer, after incubation with DAO_WT and Hepmut variants showed that internalization of Hepmut variants was inhibited. SK-Hep1 cells were incubated for 60 minutes with 20 µg/ml of the purified recombinant human DAO and Hepmut variants (negative control = no rhDAO added) were incubated at 37°C, washed twice with 150 mM glycine buffer with 150 mM sodium chloride, pH 3.0 and once with PBS to remove membrane-bound DAO. After fixation and permeabilization the cells were incubated with a 1:500 dilution of anti-ABP1 (ABP1 = amiloride binding protein 1, alternative name for DAO) antibody produced in rabbits (Sigma Aldrich). A 1:500 dilution of Alexa Fluor 488 donkey anti-rabbit (H+L) (Jackson Research, 711-545-152) was used as the secondary antibody. DAPI was used for counter-staining of the nuclei. The cells were analyzed by fluorescence microscopy on an inverted DMI-6000B microscope equipped with a HCX PL APO 63x/1.30 glycerol immersion objective and filter sets A4 (for DAPI) and L5 (for Alexa Fluor 488) (Leica Microsystems). Analogous results were obtained using HUVEC = human umbilical vein endothelial cells, the prototypical endothelial cells (EC) used for EC research.

Hepmut4 and Hepmut7 variants showed strongly reduced uptake into the SK-Hep1 cells, whereas Hepmut1 caused some staining similar to the vesicular-like staining of DAO_WT. These results not only demonstrated that Hepmut DAO variants block uptake into EC, but also for the first time that DAO is internalized by cells. It has been shown that DAO binds to the surface of EC, and DAO has been localized inside cells, but incubation of cells with DAO and proof that DAO_WT is internalized implying the existence of a DAO receptor. It was confirmed that DAO binds to heparan sulfate glycosaminoglycans present on ECs (heparin is only present in mast cells in the human body) and Hepmut variants are not able anymore to efficiently bind and are therefore not internalized. This is also reflected in the in vivo rat and mouse data described herein.

The immunofluorescence data discussed above were also reflected in Western blotting data. There is a more than a 6-fold difference in Western blotting signal of Hepmut4 and Hepmut7 versus DAO_WT and Hepmut1. These data are in agreement with the immunofluorescence data. Hepmut4 and Hepmut7 were not internalized by SK-Hep1 cells.

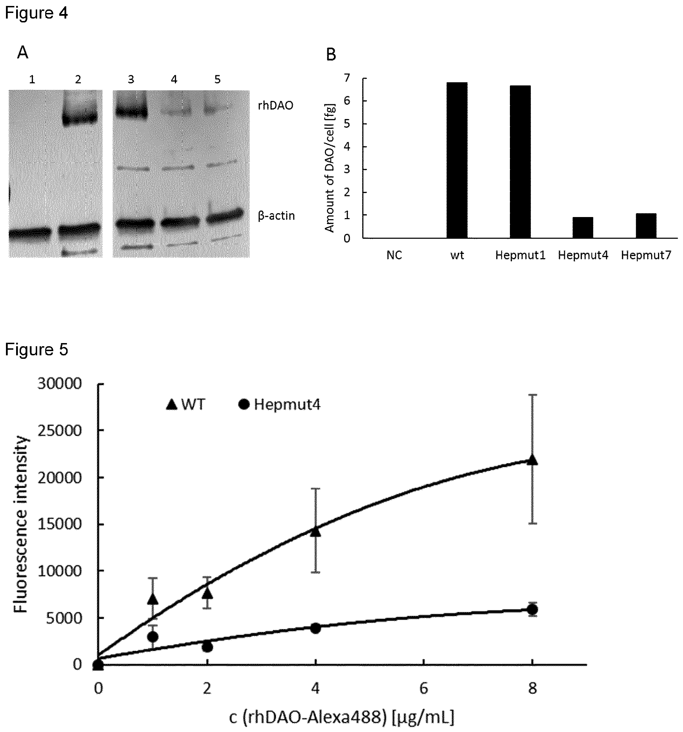

Figure 4 shows a Western blot of SK-Hep1 cell lysates after incubation with DAO_WT and Hepmut variants A. Lane 1: Negative control, 2: rhDAO_WT, 3: rhDAO-Hepmut1, 4: rhDAO-Hepmut4, 5: rhDAO-Hepmut7. SK-Hep1 cells were incubated for 60 minutes with 20 µg/ml of the respective purified DAO variants (negative control = no rhDAO added) at 37°C, washed twice with 150 mM glycine buffer with 150 mM sodium chloride, pH 3.0 and once with PBS to remove membrane-bound DAO. The cells were then lysed with RIPA buffer and sonication. The cell lysates were loaded onto a SDS-PAGE gel, followed by blotting onto a PVDF membrane. The membrane was incubated after blocking with a 1:1000 dilution of a serum IgG fraction from rabbits immunized with purified rhDAO. This step was followed by incubation with a 1:5000 dilution of β-actin mABAC-15 (Invitrogen). 1:5000 dilutions of IRDye^{®}800CW goat anti-rabbit IgG (H + L) secondary antibody and IRDye^{®}680RD goat anti-mouse IgG (H + L) (Li-Cor) were used as secondary antibodies. The membrane was scanned at 700 and 800 nm using the Odyssey Infrared Imaging System (Li-Cor). B. Band intensities were determined in reference to purified rhDAO_WT (not shown) and used to calculate the amount of internalized rhDAO.

Binding of DAO_WT and Hepmut4 variants to SK-Hep1 cells in an in vitro assay is about 5-times lower. DAO was labelled with Alexa488 fluorescent dye on the glycosaminoglycans and incubated with SK-HEP1 cells. This assay is performed in microtiter plates. The fluorescent signal is measured after washing and cell lysis.

In Figure 5, Hepmut4 variant shows reduced binding to SK-Hep1 cells compared to DAO_WT.

SK-Hep1 cells were grown in a 96-well plate and incubated with 0 to 8 µg/ml Alexa488-labelled rhDAO_WT and rhDAO-Hepmut4 for 60 minutes at 37°C. The cells were washed twice with 150 mM glycine buffer with 150 mM sodium chloride, pH 3.0 and once with PBS to remove membrane-bound DAO. After cell lysis using RIPA buffer the fluorescence intensities were determined using a Tecan plate reader. The means of triplicates with the standard errors of the mean (SEM) are shown.

The binding of DAO_WT and Hepmut4 to high and low molecular weight heparin (HMWH, LMWH) using Isothermal Titration Calorimetry (ITC), the state of the art instrument for the determination of label-free binding affinities, was also tested.

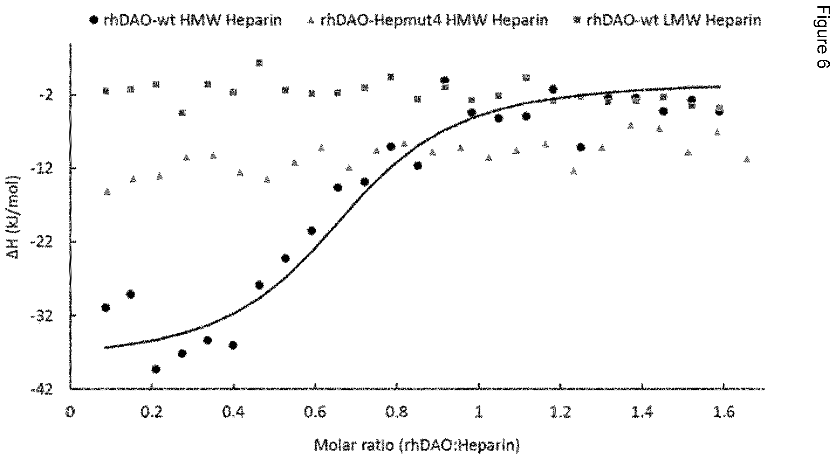

Figure 6 provides the ITC data of DAO_WT and Hepmut4.

12.5 µM purified rhDAO_WT and 12.0 µM purified rhDAO-Hepmut4 were subjected to isothermal titration calorimetry (MicroCal PEAQ-ITC, Malvern) using 25 x 1 µl injections of 160 µM high molecular weight (HMWH, Gilvasan, average molecular weight = 15 kDa) and low molecular weight heparin (LMWH, Lovenox, average molecular weight = 4.5 kDa). The buffer was 50 mM HEPES with 150 mM KCI pH 7.3. Binding was only observed for rhDAO_WT and HMWH with a K_{D} value of 423 nM.

Both components, DAO protein and the two heparin molecules, are unaltered and not immobilized. This allowed the determination of true binding affinities. Heparin-sepharose represents a multivalent matrix, where molecules are gliding from one heparin molecule to the next. DAO_WT was binding to HMWH with a K_{d} of 423 nM, whereas Hepmut4 did not bind to HMWH. DAO_WT was also not binding to LMWH.

### Intravenous Injection of DAO_WT and Hepmut4 into C57BL6 mice

DAO_WT and Hepmut4 protein were injected at 1 mg/kg into the tail vein of C57BL6 mice with a body weight of about 20 gram. Protein concentrations and enzymatic activity of the two purified DAO variantes were comparable. Protein purification was performed as published recently (Gludovacz 2016). Linear and log y-axis scales are shown.

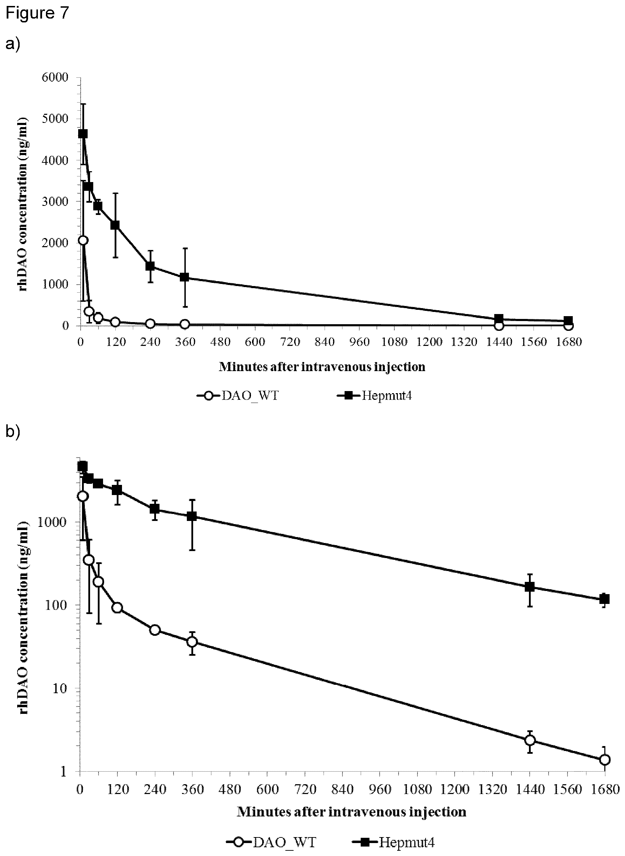

In Figure 7 linear (a) und log y-scales (b) are shown. Hepmut4 increases the AUC (Area Under the Curve) more than 19-fold compared to wild-type DAO protein after intravenous injection of 1 mg/kg DAO variants. The measured values (n = 3 to 4 mice per time point) plus/minus standard deviations are shown. DAO concentrations were measured using our in-house developed human DAO ELISA, which does not recognize mouse or rat DAO (Boehm 2017). Half-life and AUC data are summarized in the following table.

**Table 4: The AUC increases 19-fold in Hepmut4 versus DAO_WT treated mice after intravenous administration**

| | Half-life minutes* | AUC µg*min/ml§ |
|---|---|---|
| DAO_WT | 76,1 | 76 |
| Hepmut4 | 192,1 | 1468 |
| Ratio Hepmut4 to DAO_WT | 2,5 | 19,4 |

| | | |
|---|---|---|
| *Calculated from 60 to 1680 minutes §AUC = Area Under the Curve; Calculated from 10 to 1680 minutes | | |

The half-life was calculated from 60 to 1680 minutes to exclude the fast alpha distribution half-life using DAO_WT, which is about 10 minutes. Using the Hepmut4 variant this very rapid elimination of DAO from the circulation is more or less not present anymore. There is a high curve fit using a mono-exponential decay function from 60 to 1680.

### Intraperitoneal Injection of DAO_WT and Hepmut4 into C57BL6 mice

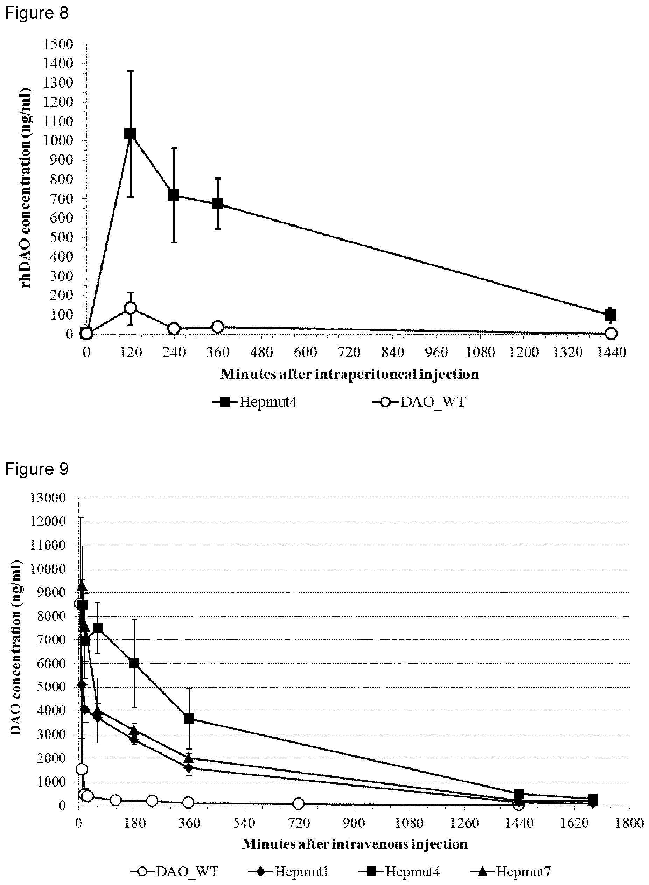

DAO_WT and Hepmut4 protein was injected intraperitoneally at 1 mg/kg in mice with a body weight of 21 gram. As shown in Figure 8, Hepmut4 increases the AUC (Area Under the Curve) more than 16-fold compared to DAO_WT protein after intraperitoneal injection. Each time point represents the mean of 3 mice and therefore in total 15 mice DAO_WT and 15 mice Hepmut4 were used. The means with the standard deviations are shown. DAO concentrations were measured using a recently published human DAO ELISA (Boehm 2017). AUC data are summarized in the following table. Half-life data are not included because we would need to assume that the absorption of DAO_WT and Hepmut4 from the intraperitoneal space is the same and this might not be the case.

**Table 5: The AUC is increased 16-fold in Hepmut4 versus DAO_WT treated mice.**

| | AUC µg*min/ml§ |
|---|---|
| DAO_WT | 41,2 |
| Hepmut4 | 666,3 |
| Ratio Hepmut4 to DAO_WT | 16,2 |

| | |
|---|---|
| §AUC = Area Under the Curve; Calculated from 0 to 1440 minutes | |

### Intravenous Injection of DAO_WT and Hepmut1, 4 and 7 into rats

Figure 9 shows means of the measured values plus/minus the standard deviation (SD) using 1 mg/kg DAO wild-type and different Hepmut variants. Slow clearance of Heparin-binding domain mutants compared to DAO wild-type protein administered at 1 mg/kg. DAO_WT n = 9; Hepmut1 n = 4; Hepmut4 n = 5; Hepmut7 n = 4; Linear y-axis scale. The means with the SD are shown.

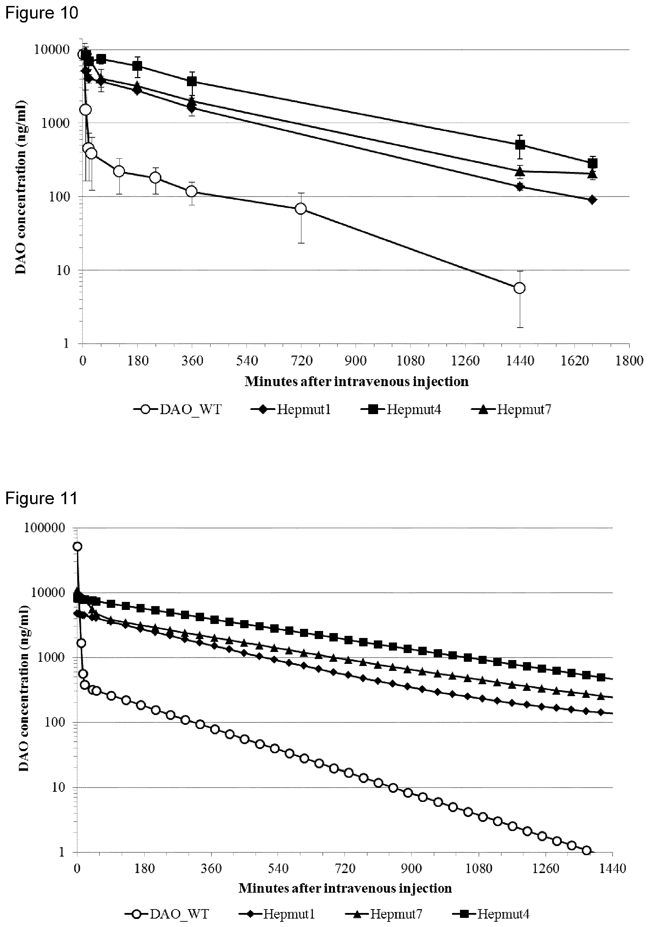

Figure 10 exhibits the slow clearance of Heparin-binding domain mutants compared to DAO wild-type protein administered at 1 mg/kg. DAO_WT n = 9; Hepmut1 n = 4; Hepmut4 n = 5; Hepmut7 n = 4; Log y-axis scale. The means with the SD are shown.

DAO_WT and Hepmut7 show a fast alpha half-life and two equations are used to derive the best fit curve. Nevertheless, the alpha half-life for Hepmut7 is significantly longer compared to DAO_WT. The half-lives are shown below. For Hepmut1 and Hepmut4 mono-exponential decay shows the best fit with the highest adjusted R square and lowest p-value. This can be also seen in the figure with the original data.

In Figure 11, the curves using the different derived exponential equations are shown. These curves have been used to calculate the AUCs and half-lives shown later.

Figure 11: Slow clearance of Heparin-binding domain mutants compared to DAO wild-type protein administered at 1 mg/kg. DAO_WT n = 9; Hepmut1 n = 4; Hepmut4 n = 5; Hepmut7 n = 4; Log y-axis scale; These curves have been generated using the best fit exponential equations.

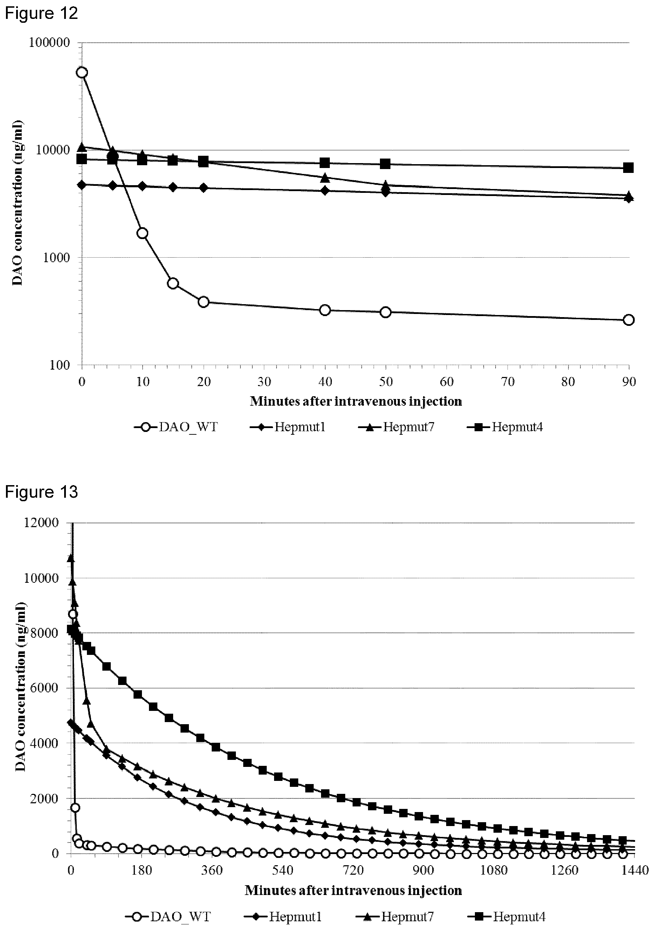

Figures 12, 13, and 14 show the first 90 minutes to see the fast clearance using DAO wild-type protein.

Figure 12 shows slow clearance of Heparin-binding domain mutants compared to DAO wild-type protein administered at 1 mg/kg. DAO_WT n = 9; Hepmut1 n = 4; Hepmut4 n = 5; Hepmut7 n = 4; Log y-axis scale; Only the first 90 minutes are shown.

Below are the linear y-axis versions of these figures.

Figure 13 shows slow clearance of Heparin-binding domain mutants compared to DAO wild-type protein administered at 1 mg/kg. DAO_WT n = 9; Hepmut1 n = 4; Hepmut4 n = 5; Hepmut7 n = 4. The curves have been generated using the best fit exponential equations; Linear y-axis scale.

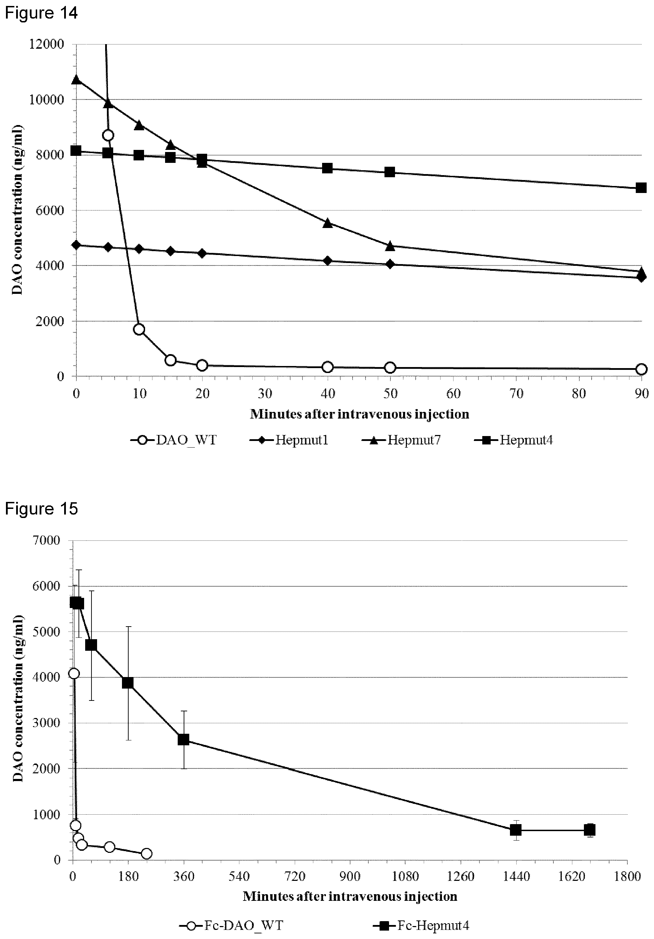

Figure 14 shows slow clearance of Heparin-binding domain mutants compared to DAO wild-type protein administered at 1 mg/kg. DAO_WT n = 9; Hepmut1 n = 4; Hepmut4 n = 5; Hepmut7 n = 4; only the first 90 minutes are shown; Linear y-axis scale.

Because of the fast alpha half-life in DAO_WT treated rats we calculated the AUCs from 0 but also 5 minutes to 240 and 1440 minutes. The data points within 10 minutes are likely quite variable. The data are summarized in the following tables.

**Table 6: The AUC is increased in Heparin-binding mutants versus DAO_WT treated rats**

| | **DAO Wild-type (n=9)** | **Hepmut1 (n=4)** | **Hepmut4 (n=5)** | **Hepmut7 (n=4)** | |
|---|---|---|---|---|---|
| **AUC_0-1440** | 270 | 1554 | 3829 | 2165 | µg/ml/min |
| **AUC_0-240** | 238 | 797 | 1550 | 1010 | µg/ml/min |
| **AUC_5-1440** | 116 | 1531 | 3788 | 2113 | µg/ml/min |
| **AUC_5-240** | 84 | 774 | 1509 | 958 | µg/ml/min |

For Hepmut variants 0 to 1440 or 5 to 1440 minutes do not make any relevant difference but for DAO wild-type protein starting at 5 minutes significantly reduced the AUC. The calculated DAO concentrations using the best fit exponential equations are above the theoretical DAO concentrations and therefore not possible. The strong increase in the AUCs is also clearly seen using the time window from 0 to 1440 minutes.

Also in this set of experiment the Hepmut4 variant shows the strongest effect. This is also reflected in the half-life data.

**Table 7: The AUC is increased between 3 and 33-fold in Heparin-binding mutants versus DAO_WT treated rats with Hepmut4 showing the largest increase.**

| | **DAO Wild-type (n=9)** | **Hepmut1 (n=4)** | **Hepmut4 (n=5)** | **Hepmut7 (n=4)** |
|---|---|---|---|---|
| **AUC_0-1440** | 1,0 | 5,8 | 14,2 | 8,0 |
| **AUC_0-240** | 1,0 | 3,4 | 6,5 | 4,2 |
| **AUC_5-1440** | 1,0 | 13,2 | 32,6 | 18,2 |
| **AUC_5-240** | 1,0 | 9,2 | 17,9 | 11,4 |

**Table 8: Elimination of the very fast alpha half-life in DAO wild-type and increase in the beta half-life in heparin-binding mutants.**

| | **t_{1/2} alpha minutes** | **t_{1/2} beta minutes** |
|---|---|---|
| **DAO_WT** | 1,9 | 162 |
| **Hepmut1** | na | 214 |
| **Hepmut4** | na | 343 |
| **Hepmut7** | 42 | 297 |

| | | |
|---|---|---|
| na = not applicable | | |

In conclusion mutations in the putative Heparin-binding domain of DAO strongly increase the AUC in rats after intravenous injection of 1 mg/kg body weight. The best performing mutant is the double mutant Hepmut4 with 2 arginines removed. This fits well to prediction and heparin-sepharose elution data.

### Example 2

### Fc-DAO with modified GAG binding domain:

Also Fc-DAO fusion variants were tested with the heparin-binding mutations and the PK parameters further improved with a beta half live of 9 hours and increased AUC. Amino acids involved in the high affinity interaction with Fc_gamma receptor were removed and amino acids involved in the interaction with FcRN have not been altered.

### Intravenous Injection of Fc-DAO_WT and Fc-DAO-Hepmut4 into rats

Below are the results from 6 and 4 rats after intravenous injection of 1 mg/kg Fc-DAO wild-type and Fc-Hepmut4 protein using linear and log scales. We only measured for 4 hours using Fc-DAO wild-type. Nevertheless, the derived exponential function can be used to extrapolate to 1680 minutes like it was measured using the Fc-Hepmut4 variant (see below). The Fc-DAO fusion protein shows similarly to the DAO wild-type protein a very fast alpha distribution half-life. Most of the fusion variant is removed from plasma within 20 minutes. Afterwards the half-life is about 120 minutes calculated using the 30, 120 and 240 time point values.

Fc-Hepmut4 is much more stable in plasma. The DAO clearance mechanism is clearly dominant over the Fc part. The half-life of human IgGs in rats is several days and this half-life is mainly determined by binding of the Fc part to the FcRN receptor.

Figure 15 shows the rapid clearance of Fc-DAO wild-type compared to Fc-Hepmut4 administered at 1 mg/kg in 6 or 4 rats respectively. The means with the standard deviations are shown; Linear y-axis scale.

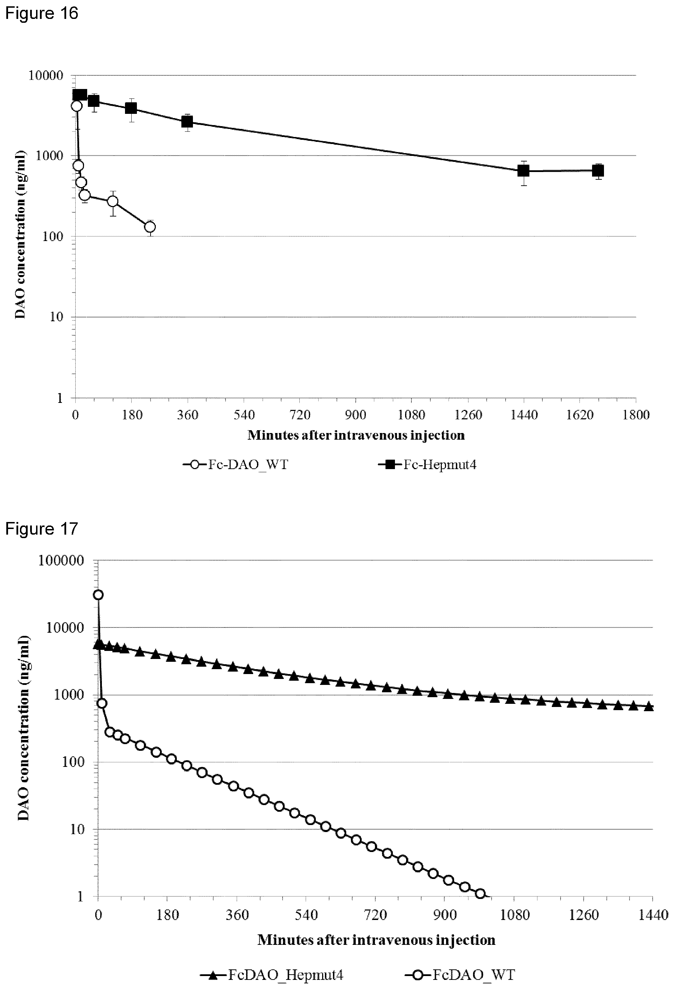

Figure 16 shows the rapid clearance of Fc-DAO wild-type compared to Fc-Hepmut4 administered at 1 mg/kg in 6 or 4 rats respectively. The means with the standard deviations are shown; Log y-axis scale.

Both data sets can be best fit to a mono-exponential decay function with p-values of less than 0.001 and adjusted R square values of > 97%. For Fc-DAO-wild-type protein the half-life after 30 minutes was calculated to be 120 minutes based on the data from time points 30, 120 and 240 minutes. A two factor decay exponential curve fitting did not converge. The best fit equations are used to extrapolate the Fc-DAO data to 24 hours. The curves are shown below.

In Figure 17, Fc-DAO-Hepmut4 shows a strong increase in the AUC after intravenous administration of 1 mg/kg; Log y-axis scale.

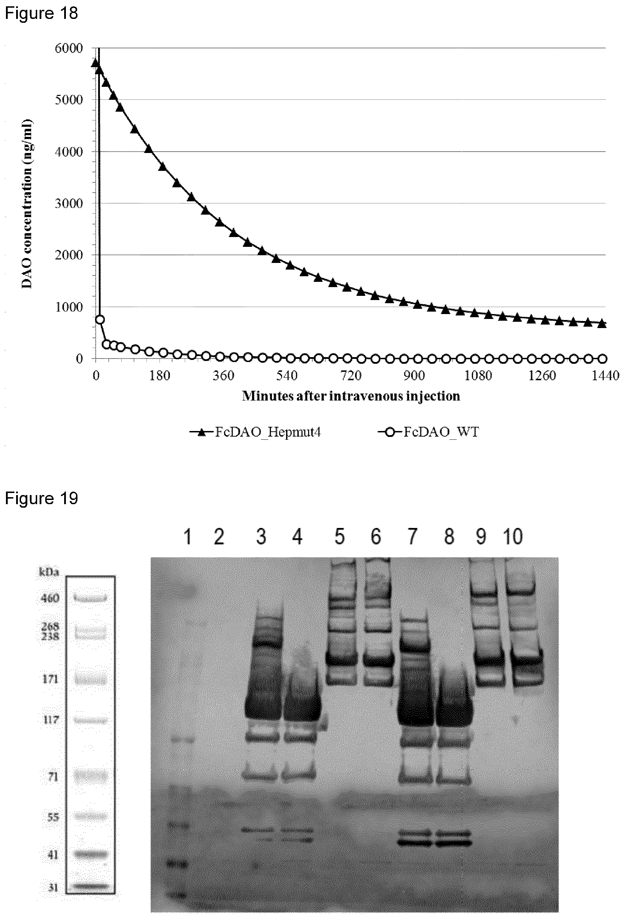

In Figure 18, Fc-DAO-Hepmut4 shows a strong increase in the AUC after intravenous administration of 1 mg/kg; Linear y-axis scale.

Areas under the curves were calculated from 0 and 5 minutes to 240 or 1440 minutes after intravenous injection. Curve fitting in the first minutes resulted in very high values at 0 minutes in the Fc-DAO data and therefore we also calculated the AUC starting at 5 minutes.

**Table 9: The AUC is strongly increased in Fc-Hepmut4 versus Fc-DAO_WT treated rats**

| | **Fc-DAO Wild-type (n=6)** | **Fc-Hepmut4 (n=4)** | |
|---|---|---|---|
| **AUC_0-1440** | 157 | 2751 | µg/ml/min |
| **AUC_0-240** | 142 | 1057 | µg/ml/min |
| **AUC_5-1440** | 69 | 2723 | µg/ml/min |
| **AUC_5-240** | 54 | 1028 | µg/ml/min |

The ratios of the AUCs are shown in the next tables followed by a table with the calculated half-lives.

**Table 10: The AUCs of Fc-Hepmut4 versus Fc-DAO_WT are 7 to 40-fold larger**

| | **Fc_DAO Wild-type (n=6)** | **Fc_Hepmut4 (n=4)** |
|---|---|---|
| **AUC_0-1440** | 1,0 | 17,6 |
| **AUC_0-240** | 1,0 | 7,4 |
| **AUC_5-1440** | 1,0 | 39,5 |
| **AUC_5-240** | 1,0 | 19,0 |

**Table 11: The fast alpha half-live of Fc-DAO_WT is eliminated in Fc-Hepmut rats**

| | **t_{1/2} alpha minutes** | **t_{1/2} beta minutes*** |
|---|---|---|
| **Fc_DAO_WT** | 1,7 | 120 |
| **Fc_Hepmut4** | na | 268 |

| | | |
|---|---|---|
| *Starting at 30 minutes after intravenous injection | | |

Fc-Hepmut4 variants are more stable in plasma compared to Fc-DAO. This is in agreement with mice and rat data using non-fusion DAO variants. Nevertheless, the half-life of Fc-Hepmut4 is still rather short compared to IgG antibodies. DAO clearance seems still dominant over slower Fc clearance mechanisms.

### Example 3

### DAO with modified GAG binding domain and modified cys123:

Cysteine 123 and 633 are not involved in disulfide bond formation of the DAO dimer.

Relative accessible surface area or relative solvent accessibility (RSA) of a protein residue is a measure of residue solvent exposure. It can be calculated by the formula:RSA=ASA/MaxASA, wherein ASA is the solvent accessible surface area and MaxASA is the maximum possible solvent accessible surface area or the residue. Both ASA and MaxASA are commonly measured in Å².

### RSA Cys123

Cys123 = 93.84 Å² accessible of 148 Å² = 63.4% (monomer B)
Cys123 = 90.72 Å² accessible of 148 Å² = 61.3% (monomer A) **Mean** = **62.4%**

### RSA Cys633

Cys633 = 34.62 accessible of 148 = 23.4% (monomer B)
Cys633 = 33.49 accessible of 148 = 22.6% (monomer A) **Mean** = **23%**

Cys123 is on the surface and this is unusual. The amino acid cysteine is the rarest and the "least and highest" conserved amino acid in proteins (Marino SM and Gladyshev VN, J Mol Biol. 2010 Dec 17;404(5):902-16), likely because it is available for oxidation. DAO produces hydrogen peroxide, which might cause exactly this oxidation of cys123 and consequently it can form a disulfide bond which might severely disturb function. It is highly conserved in enzymes as catalytic amino acid and if involved in disulfide bond formation, as it is also the case in DAO. Cys633 is deeper inside the structure and less accessible and may not play a role in aggregate formation. It might play a role at high DAO concentrations.

Because a DAO dimer possesses two accessible Cys123 amino acids, one dimer can form a disulfide bridge with another dimer resulting in a tetramer, but it can also interact with two dimers forming a hexamers, etc. The molecular weight of DAO lacking the secretion signal purely based on amino acids (732) would be 166872 Da for the dimer. The tetramer would be 333744 Da, the hexamer 500617 Da and the octamer 667489 Da but the glycans will increase this by probably about 25% (Elmore, 2002). The height of a DAO monomer or dimer is about 65 Å but increases to 130 Å in the tetramer, 195 Å and 260 Å in the hexamer and octamer respectively. We have no data about the structure of multimeric stack of DAO. The aggregate might be rigid or flexible. These aggregates are certainly more immunogenic because neo-epitopes might be created between two dimers and repeated 2 or 3 times with potential and likely negative consequences for efficacy and safety (see below).

**Table 12: Molecular weight of DAO monomer to octamer using 25% glycan weight**

| | **732 amino acids (aa)** | **732 aa plus Glycans 25%** | **DAO height nm** | **DAO length nm** |
|---|---|---|---|---|
| **Monomer** | 83436 | 104295 | 6.5 | 10.0 |
| **Dimer** | 166872 | 208590 | 6.5 | 10.0 |
| **Tetramer** | 333744 | 417180 | 13.0 | 10.0 |
| **Hexamer** | 500616 | 625770 | 19.5 | 10.0 |
| **Octamer** | 667488 | 834360 | 26.0 | 10.0 |

Higher order aggregates of DAO have been described. Paolucci et al (Biochimie. 1971;53(6):735-49) published that the molecular weight of human placenta DAO is comprised of 1-4 multiples of 125 kDa +/- 5 or in other words 125, 250, 375 and 500 kDa. Tufvesson (Scand J Clin Lab Invest. 1978 Sep; 38(5):463-72) published a DAO molecular weight of 245 and 485 kDa again after purification from human amniotic fluid corresponding to dimer and tetramer. Wilfingseder et al. (Inflamm Res. 2002 Apr;51 Suppl 1:S89-90) showed "complex formation" of human placental DAO using Western blotting.

Different DAO-expressing plasmids were transfected into ExpiCHO cells for transient expression. Western blotting was performed directly on the supernatants after 7 days of culture. The results are shown in Figure 19.

Figure 19: lane 1: HiMark Standard; lane 2: Negative Control (empty plasmid); lane 3: rhDAO_WT; lane 4: rhDAOΔ123; lane 5: rhFc-DAO; lane 6: rhFc-DAOΔ123; lane 7: rhDAO-Hepmut4; lane 8: rhDAO-Hepmut4Δ123; lane 9: rhFc-DAO-Hepmut4; lane 10: rhFc-DAO-Hepmut4Δ123. rhFc = recombinant human Fc fusion protein with DAO; 15 µl of each culture supernatant were loaded. SDS-PAGE was performed under nonreducing conditions. Antibody: MUV rabbit serum #408, 1:5000.

Cys123 to Ala123 mutation completely prevented tetramerization and higher order aggregate formation of recombinant human wild-type DAO and Hepmut4 variants. There is no effect in the rhFc-DAO fusion constructs. This implies that aggregate formation in these Fc fusion variants is not caused by Cys123 but more likely by the Fc part, which contains also cysteines.

Under reducing conditions using mercaptoethanol, the disulfide bonds are opened and no tetramer or higher order aggregates are seen neither in wild-type DAO and Hepmuts or fusion variants. The molecular weight is between 71 and 117 and would be predicted based just on amino acids to be 83.436 Da. These data clearly proof that the cys123 to ala123 mutation completely prevents tetramerization and higher order n-meric variants of DAO. Cys633 is not involved.

The signal intensity of each lane was determined using ImageJ software to roughly quantify the percent of higher n-meric variants. To compare the WT and the corresponding cys123 mutant lanes, equally sized areas were selected (as indicated) and a signal profile was generated. The area under the curve (AUC) for each lane was calculated.

**Table 13: Quantification of the effect of the ala123 mutation on aggregate formation in two replicates**

| | **Replicate 1** | | **Replicate 2** | |
|---|---|---|---|---|
| | **Signal** | **Percent** | **Signal** | **Percent** |
| **rhDAO_WT** | 77693 | 100% | 68048 | 100% |
| **rhDAO_Ala123** | 66523 | 86% | 57007 | 84% |
| **rhDAO_Hepmut4** | 76456 | 100% | 67066 | 100% |
| **rhDAO_Hepmut4_Ala123** | 56045 | 73% | 55377 | 83% |
| **rhDAO WT minus Ala123** | 11170 | 14% | 11041 | 16% |
| **Hepmut4 WT minus Ala123** | 20411 | 27% | 11689 | 17% |
| **Mean** | **19%** | | | |
| **SD** | **5,5%** | | | |
| **rhFc-DAO** | 70709 | 100% | 64270 | 100% |
| **rhFc-DAO_Ala123** | 61878 | 88% | 69892 | 109% |
| **rhFc-DAO-Hepmut4** | 56134 | 100% | 66383 | 100% |
| **rhFc-DAO-Hepmut4_Ala123** | 57673 | 103% | 82826 | 125% |
| **rhFc-DAO WT minus Ala123** | 8831 | 12% | -5622 | -9% |
| **rhFc-Hepmut4 WT minus Ala123** | -1538 | -3% | -16443 | -25% |
| **Mean** | **-6%** | | | |
| **SD** | **15,4%** | | | |

The mean (SD) difference between Cys123 and Ala123 mutation is on average combining data from replicate 1 and 2 about 19% (5.5%) for WT and Hepmut4 mutant. For the Fc-DAO variants the mean (SD) difference is -6% (15.4%) or in other words cys123 to ala123 mutation are not different.

Therefore, the ala123 mutation has a significant advantage for manufacturing and quality control in general but also for reduction of immunogenicity and therefore this mutation is also clinically highly relevant. It is well known that aggregates are more immunogenic and therefore using this mutation the immunogenicity of rhDAO will be reduced.

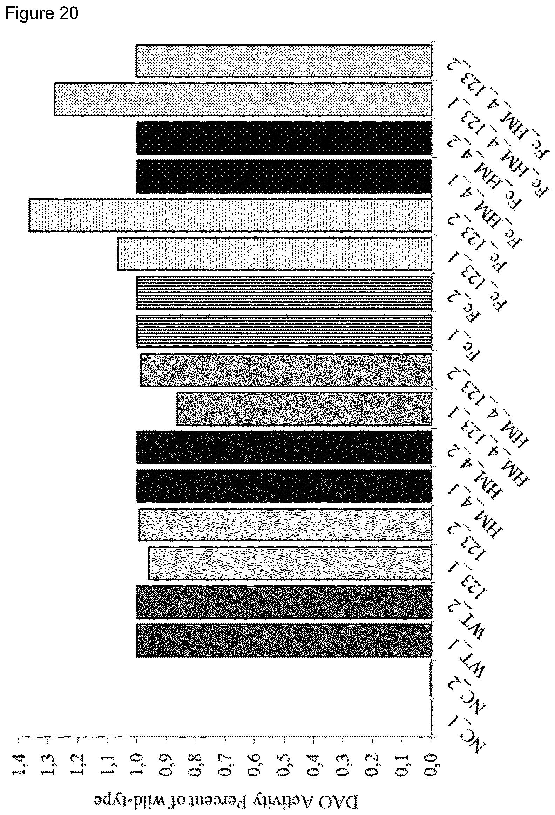

The supernatants were tested for DAO activity using a standard DAO activity assay as described in Bartko (Alcohol. 2016 Aug;54:51-9.) or Gludovacz 2016. It was confirmed that here is no effect of the cys123 to ala123 mutation on DAO activity. In Figure 20, DAO activity is presented. WT DAO, Hepmut4 or Fc variants are set to 100% and compared to cys123 to ala123 mutants. It is shown that there is no significant difference in the DAO activity between DAO_WT and cys123 mutations.

### Example 4

### Generation of Asn168 glycosylation mutants

### Site directed mutagenesis of Asn-168

To replace asparagine at N-glycosylation site Asn-168 (AAT) with glutamine (CAG), the respective codon in the DAO expression plasmid (Gludovacz E. et al., 2016, J. Biotechnol., 227, 120-130) was mutated using site-directed mutagenesis. Therefore, a PCR was conducted using the following 5-phosphorylated primers: Asn-168, CAGaccacaggcttctcattc (forward, SEQ ID NO:104) and gaggaagaactgatgcag (reverse, SEQ ID NO:105). Phusion polymerase (Thermo Fisher Scientific) was used: annealing temperature: 56.3 °C; elongation times, 4 min for 30 cycles. Ligation (T4 DNA ligase, New England Biolabs) and amplification of the final plasmids were performed. Correctness of sequence was verified by DNA sequencing (Eurofins MWG Operon). All cloning techniques were conducted according toGreen M.R. and Sambrook, J. (2012), Molecular Cloning: A Laboratory Manual 4th Ed. Cold Spring Harbor Laboratory Press, Cold Spring Harbor, NY; and according to the manufacturer's instructions.

### Intravenous administration of rhDAO, competing proteins and glycosylation mutants in rats and mice

The experimental protocols for the treatment of rats and mice were approved by the local Animal Welfare Committee and the Federal Ministry of Science, Research and Economy (GZ 66.009/0152-WF/V/3b/2014) and conducted in full accordance with the ARRIVE guidelines (Kilkenny, C et al., Br. J. Pharmacol. (2010) 160, 1577-1579). For implanting a vascular access port into the Vena jugularis (Rodent Vascular Access Port with Detachable Silastic Catheter, Hugo-Sachs Elektronic-Harvard Apparatus) rats are anesthetized via intraperitoneal injection of ketamine (100 mg/kg) and xylazine (5 mg/kg). After intubation anesthesia is maintained through continuous volume-controlled ventilation (O₂-air-mixture + 1.5% isoflurane). The fur is depilated at the area of surgery and subsequently disinfected with iodine solution (Betaisodona, Mundipharma). Metamizol (100 mg/kg) is administered subcutaneously for analgesia. A skin incision of approximately 1 cm is made at the dorsum of the animals to place the balloon of the vascular access port subcutaneously. The port is flushed with saline before the balloon is fixated with sutures between the scapulae. A second skin incision is made at the site of the V. jugularis sinistra to dissect the V. jugularis for catheterization. A small incision is placed at the exposed V. jugularis sinistra and the catheter is inserted and fixated with silk threats (7-0). Subcutaneous tissue and skin is closed with simple interrupted sutures (4-0). The surgery is performed aseptically. The average surgery time is approximately 2 hours. Post-operative analgesia is provided by ad libitum drinking water with piritramid and glucose (30 mg piritramid and 10 mL 10% glucose in 250 mL drinking water). During the experiments the vascular access port is filled with a solution containing 10 µg/mL argatroban (Argatra 100 mg/mL; Mitsubishi Pharma), 0.3 µg/mL tissue plasminogen activator (Alteplase, Actilyse, Boehringer Ingelheim) and 0.38% sodium citrate in 0.9% NaCl to prevent coagulation.

Venous blood withdrawals (0.5 mL) are conducted at pre-defined time points under short isoflurane anesthesia into tubes containing sodium citrate for anticoagulation. Plasma is prepared within 4 hours and stored at -32°C until analysis. Fluid substitution (0.5 mL) using physiological saline solution (0.9% NaCl) is provided to the animals. Animals are sacrificed after the last blood withdrawal time point under deep ketamine-xylazine (35 mg and 5 mg/kg) anesthesia by an overdose of pentobarbital (300 mg/kg). Male rats (Sprague-Dawley) with approximately 400 gram and female mice (C57BL/6N) with approximately 20 g body weight are included.

Male Sprague Dawley rats are purchased from commercial vendors (Janvier Labs, Le Genest-Saint-Isle, France and Division Laboratory Animal Science and Genetics, Medical University of Vienna, Himberg, Austria). The animals are housed under controlled and standardized conditions (artificial L/D cycle 12:12, room temperature 22 ±2°C, humidity 45 ±10%). The animals are kept in groups of two (Makrolon 3 cages) and are provided with environment enrichment. The animals have ad libitum access to water and to complete feed for rats (Alleinfutter für Ratten und Mäuse sniff R/M-H; sniff Spezialdiäten GmbH).

Female mice (C57BL/6N) are obtained from a commercial vendor (Charles River Laboratories, Sulzfeld, Germany and Division Laboratory Animal Science and Genetics, Medical University of Vienna, Himberg, Austria). The animals are housed under controlled and standardized conditions (artificial L/D cycle 12:12, room temperature 22 ±2°C, humidity 45 ±10%). The animals are kept in groups of five (Makrolon 2 long cages) and have ad libitum access to water and to complete feed for mice as described above for rats.

### Rapid plasma clearance of rhDAO in rats and mice after intravenous administration

Purified rhDAO, wild type rhDAO and rhDAO containing any one of mutations HepMut 1 to HepMut 7 or any of HepMut1 to HepMut4 further comprising Cys123 and/or Asn168GIn mutations, is intravenously administered at 1 mg/kg into rats and mice and blood samples are drawn at the indicated time points. DAO antigen concentrations are measured using a recently published human DAO ELISA (Boehm T. et al., 2017, Clin. Biochem., 50, 444-451). The distribution (alpha) and elimination (beta) half-lives are approximately 3 and 230 minutes respectively in rats and approximately 11 and 110 minutes respectively in mice. In rats more than 90% of the injected dose is removed from the plasma pool within 10 minutes. The rapid clearance in mice is somewhat slower.

## Claims

1. A recombinant human diamine oxidase (DAO) variant comprising any one of SEQ ID NOs:2 to 8 or a sequence having at least 99% sequence identity with any one of SEQ ID NOs:2 to 8, with decreased glycosaminoglycan binding affinity compared to the respective wild type human DAO, wherein said DAO comprises at least one amino acid modification of any one of amino acids at positions 568-575 of the glycosaminoglycan (GAG) binding domain with reference to the numbering of SEQ ID NO:1.

2. The recombinant DAO variant of claim 1, further comprising at least one modification of solvent accessible cysteine at amino acid position 123 (cys123) with reference to the numbering of SEQ ID No. 1, specifically the modification of the cysteine is an amino acid substitution, deletion or coupling with a chemical moiety, more specifically cys123 is substituted by alanine.

3. The recombinant DAO variant of claim 1 or 2, wherein the GAG binding domain is a heparin/heparan sulfate binding domain.

4. The recombinant DAO variant of any one of claims 1 to 3, wherein the at least one amino acid modification in the GAG binding domain is an amino acid substitution, deletion, insertion or coupling with a chemical moiety.

5. The recombinant DAO variant of any one of claims 1 to 4, comprising 2, 3, 4, 5, 6, 7, or 8 amino acid substitutions in the GAG binding domain.

6. The recombinant DAO variant of any one of claims 1 to 5, comprising a GAG binding domain of amino acid sequence X1FX2X3X4LPX5, wherein
X1 can be by any amino acid, specifically it is A or S, more specifically it is S;
X2 can be by any amino acid, specifically it is K;
X3 can be by any amino acid, specifically it is A or T, more specifically it is T,
X4 can be by any amino acid, specifically it is K, and
X5 can be by any amino acid, specifically it is K or T, more specifically it is T.

7. The recombinant DAO variant of claim 6, comprising the amino acid sequence selected from the group consisting of SFKAKLPK (SEQ ID NO:33), AFKAKLPT (SEQ ID NO:34), AFKTKLPK (SEQ ID NO:35), SFKTKLPK (SEQ ID NO:36), AFKTKLPT (SEQ ID NO:37), SFKAKLPK (SEQ ID NO:38).

8. The recombinant DAO variant of any one of claims 1 to 7 further comprising an amino acid substitution at position 168 with reference to SEQ ID NO 1, specifically Asn is replaced by Gln.

9. The recombinant DAO variant of any one of claims 1 to 8, wherein said DAO has increased plasma half-life compared to wild type DAO, specifically said half-life is increased at least 1.5 fold, specifically at least 2 fold compared to wild type DAO.

10. The recombinant DAO variant of any one of claims 1 to 9, wherein said DAO has an at least 10-fold increased area under the plasma drug concentration-time curve (AUC) compared to wild type DAO.

11. The recombinant DAO variant of any one of claims 1 to 10, wherein internalization by endothelial cells is at least 10%, 25%, 50%, 60%, 70%, 80%, specifically 90% reduced compared to wild type DAO.

12. The recombinant DAO variant of any one of claims 1 to 11, wherein GAG binding affinity, specifically heparin/heparan sulfate binding affinity is at least 10%, 25%, 50%, 60%, 70%, 80%, specifically 90% reduced compared to wild type DAO.

13. The recombinant DAO of any one of claims 2 to 12, comprising the amino acid sequences of SEQ ID NOs:9 to 16.

14. A fusion polypeptide comprising the recombinant DAO variant of any one of claims 1 to 12 and an Fc domain of human IgG or human serum albumin (HSA), wherein the fusion polypeptide retains the functional activity of the recombinant DAO.

15. An isolated nucleotide sequence encoding the DAO variant of any one of claims 1 to 13, specifically comprising sequence SEQ ID NOs:18 to 24 and 25 to 32.

16. A recombinant vector comprising the nucleotide sequence of claim 15, specifically the vector is a bacterial, yeast, baculoviral, plant or mammalian expression vector.

17. An expression cassette comprising the nucleotide sequence of claim 15, operably linked to regulatory elements.

18. A recombinant host cell or a host cell line comprising the recombinant DAO variant of any one of claim 1 to 13, wherein the host cells are selected from the group consisting of CHO cells, Vero cells, MDCK cells, Pichia pastoris cells, SF9 cells.

19. An expression system comprising the vector of claim 16 or the expression cassette of claim 17 and a host cell or host cell line of claim 18.

20. A method for producing the recombinant DAO variant according to claims 1 to 12, said method comprising the steps of
i. cloning a nucleotide sequence encoding the DAO variant of any one of claims 1 to 13 into an expression vector,
ii. transforming a host cell with said vector,
iii. cultivating the transformed host cell under conditions wherein the DAO variant is expressed,
iv. isolating the DAO variant from the host cell culture, optionally by disintegrating the host cells, and optionally
v. purifying the DAO variant.

21. Pharmaceutical composition comprising the recombinant DAO variant of any one of claims 1 to 13 and optionally one or more excipients.

22. The recombinant DAO variant of any one of claims 1 to 13 for use in the treatment of a condition associated with excess histamine, specifically for the treatment of chronic allergic diseases, more specifically for the treatment of anaphylaxis, anaphylactic shock, chronic urticaria, acute urticaria, asthma, hay fever, allergic rhinitis, allergic conjunctivitis, histamine intoxication, headache, atopic dermatitis inflammatory diseases, mastocytosis, mast cell activation syndrome (MCAS), pre-eclampsia, hyperemesis gravidarum, pre-term labor, peptic ulcers, acid reflux, pruritus, and sepsis.

23. The use of the recombinant DAO variant of any one of claims 1 to 13 for the manufacture of a medicament for the treatment of a condition associated with excess histamine, specifically for the treatment of chronic allergic diseases, more specifically for the treatment of anaphylaxis, anaphylactic shock, chronic urticaria, acute urticaria, asthma, hay fever, allergic rhinitis, allergic conjunctivitis, histamine intoxication, headache, atopic dermatitis inflammatory diseases, mastocytosis, peptic ulcers, acid reflux, pruritus, and sepsis.

## Patentansprüche

1. Rekombinante Variante der menschlichen Diaminoxidase (DAO), umfassend eine beliebige der SEQ ID NO:2 bis 8 oder eine Sequenz, die zumindest 99 % Sequenzidentität mit einer beliebigen der SEQ ID NO:2 bis 8 aufweist, mit verringerter Bindungsaffinität an Glykosaminoglykan verglichen mit der jeweiligen menschlichen DAO vom Wildtyp, wobei die DAO zumindest eine Aminosäure-Modifikation einer beliebigen der Aminosäuren an den Position 568-575 der Glykosaminoglykan- bzw. GAG-Bindungsdomäne unter Bezugnahme auf die Nummerierung von SEQ ID NO:1 umfasst.

2. Rekombinante DAO-Variante nach Anspruch 1, ferner umfassend zumindest eine Modifikation von lösungsmittelzugänglichem Cystein an der Aminosäure-Position 123 (cys123) in Bezug auf die Nummerierung von SEQ ID NO:1, wobei im Speziellen die Modifikation des Cysteins eine Substitution, Deletion oder Kopplung der Aminosäure mit einer chemischen Einheit ist, und noch spezieller cys123 durch Alananin substituiert ist.

3. Rekombinante DAO-Variante nach Anspruch 1 oder 2, wobei die GAG-Bindungsdomäne eine Heparin/Heparansulfat-Bindungsdomäne ist.

4. Rekombinante DAO-Variante nach einem der Ansprüche 1 bis 3, wobei die zumindest eine Aminosäure-Modifikation in der GAG-Bindungsdomäne eine Substitution, Deletion, Insertion oder Kopplung der Aminosäure mit einer chemischen Einheit ist.

5. Rekombinante DAO-Variante nach einem der Ansprüche 1 bis 4, umfassend 2, 3, 4, 5, 6, 7, oder 8 Aminosäure-Substitutionen in der GAG-Bindungsdomäne.

6. Rekombinante DAO-Variante nach einem der Ansprüche 1 bis 5, umfassend eine GAG-Bindungsdomäne mit der Aminosäuresequenz X1FX2X3X4LPX5, wobei
X1 eine beliebige Aminosäure sein kann, im Speziellen ein A oder S ist, noch spezieller S ist;
X2 eine beliebige Aminosäure sein kann, im Speziellen K ist;
X3 eine beliebige Aminosäure sein kann, im Speziellen ein A oder T ist, noch spezieller T ist,
X4 eine beliebige Aminosäure sein kann, im Speziellen K ist, und
X5 eine beliebige Aminosäure sein kann, im Speziellen ein K oder T ist, noch spezieller T ist.

7. Rekombinante DAO-Variante nach Anspruch 6, umfassend die Aminosäuresequenz ausgewählt aus der Gruppe bestehend aus SFKAKLPK (SEQ ID NO:33), AFKAKLPT (SEQ ID NOI34), AFKTKLPK (SEQ ID NO:35), SFKTKLPK (SEQ ID NOIS6), AFKTKLPT (SEQ ID NO:37), SFKAKLPK (SEQ ID NO:38).

8. Rekombinante DAO-Variante nach einem der Ansprüche 1 bis 7, ferner umfassend eine Aminosäure-Substitution an Position 168 in Bezug auf SEQ ID NO 1, wobei im Speziellen Asn durch Gln ersetzt ist.

9. Rekombinante DAO-Variante nach einem der Ansprüche 1 bis 8, wobei die DAO eine verlängerter Plasmahalbwertszeit im Vergleich zu DAO vom Wildtyp aufweist, im Speziellen die Halbwertszeit um das zumindest 1,5-Fache, im Speziellen das zumindest 2-Fache im Vergleich zu DAO vom Wildtyp verlängert ist.

10. Rekombinante DAO-Variante nach einem der Ansprüche 1 bis 9, wobei die DAO eine mindestens 10-fach vergrößerte Fläche unter der Kurve der Plasma-Arzneimittelkonzentration/Zeit (AUG) im Vergleich zu DAO vom Wildtyp aufweist.

11. Rekombinante DAO-Variante nach einem der Ansprüche 1 bis 10, wobei die Internalisierung durch Endothelzellen im Vergleich zu DAO vom Wildtyp um zumindest 10 %, 25 %, 50 %, 60 %, 70 %, 80 %, im Speziellen 90 % verringert ist.

12. Rekombinante DAO-Variante nach einem der Ansprüche 1 bis 11, wobei die GAG-Bindungsaffinität, insbesondere die Heparin/Heparansulfat-Bindungsaffinität, im Vergleich zu DAO vom Wildtyp um zumindest 10 %, 25 %, 50 %, 60 %, 70 %, 80 %, im Speziellen 90 % verringert ist.

13. Rekombinante DAO nach einem der Ansprüche 2 bis 12, umfassend die Aminosäuresequenzen von SEQ ID NO:9 bis 16.

14. Fusionspolypeptid, umfassend die rekombinante DAO-Variante nach einem der Ansprüche 1 bis 12 und eine Fc-Domäne von menschlichem IgG oder menschlichem Serumalbumin (HSA), wobei das Fusionspolypeptid die funktionelle Aktivität der rekombinanten DAO beibehält.

15. Isolierte Nukleotidsequenz, welche für die DAO-Variante nach einem der Ansprüche 1 bis 13 kodiert, im Speziellen umfassend die Sequenz SEQ ID NO:18 bis 24 und 25 bis 32.

16. Rekombinanter Vektor, umfassend die Nukleotidsequenz nach Anspruch 15, wobei im Speziellen der Vektor ein bakterieller, Hefe-, bakuloviraler, pflanzlicher oder Säugetier-Expressions-Vektor ist.

17. Expressionskassette, umfassend die Nukleotidsequenz nach Anspruch 15, die wirkmäßig mit regulatorischen Elementen verknüpft ist.

18. Rekombinante Wirtszelle oder Wirtszelllinie, umfassend die rekombinante DAO-Variante nach einem der Ansprüche 1 bis 13, wobei die Wirtszellen aus der Gruppe bestehend aus CHO-Zellen, Vero-Zellen, MDCK-Zellen, Zellen von Pichia pastoris oder SF9-Zellen ausgewählt sind.

19. Expressionssystem umfassend den Vektor nach Anspruch 16 oder die Expressionskassette nach Anspruch 17 und eine Wirtszelle oder Wirtszelllinie nach Anspruch 18.

20. Verfahren zur Herstellung einer rekombinanten DAO-Variante nach einem der Ansprüche 1 bis 12, wobei das Verfahren die Schritte umfasst:
i. Klonieren einer Nukleotidsequenz, welche für die DAO-Variante nach einem der Ansprüche 1 bis 13 kodiert, in einen Expressionsvektor,
ii. Transformieren einer Wirtszelle mit dem Vektor,
iii. Kultivieren der transformierten Wirtszelle unter Bedingungen, in welchen die DAO-Variante exprimiert wird,
iv. Isolieren der DAO-Variante aus der Wirtszellkultur, gegebenenfalls durch Aufbrechen der Wirtszellen, und gegebenenfalls
v. Reinigen der DAO-Variante.

21. Pharmazeutische Zusammensetzung, umfassend eine rekombinante DAO-Variante nach einem der Ansprüche 1 bis 13 und gegebenenfalls einen oder mehrere Exzipienten.

22. Rekombinante DAO-Variante nach einem der Ansprüche 1 bis 13 zur Verwendung bei der Behandlung eines Zustands, der mit überschüssigem Histamin assoziiert ist, im Speziellen zur Behandlung von chronischen allergischen Erkrankungen, noch spezieller zur Behandlung von Anaphylaxie, anaphylaktischem Schock, chronischer Urtikaria, akuter Urtikaria, Asthma, Heuschnupfen, allergischer Rhinitis, allergischer Konjunktivitis, Histaminvergiftung, Kopfschmerzen, atopischer Dermatitis, entzündlichen Erkrankungen, Mastozytose, Mastzellaktivierungssyndrom (MCAS), Präeklampsie, Hyperemesis gravidarum, vorzeitigen Wehen, Magengeschwüren, Säurereflux, Pruritus und Sepsis.

23. Verwendung der rekombinanten DAO-Variante nach einem der Ansprüche 1 bis 13 für die Herstellung eines Medikaments für die Behandlung eines Zustands, der mit überschüssigem Histamin assoziiert ist, im Speziellen zur Behandlung von chronischen allergischen Erkrankungen, noch spezieller zur Behandlung von Anaphylaxie, anaphylaktischem Schock, chronischer Urtikaria, akuter Urtikaria, Asthma, Heuschnupfen, allergischer Rhinitis, allergischer Konjunktivitis, Histaminvergiftung, Kopfschmerzen, atopischer Dermatitis, entzündlichen Erkrankungen, Mastozytose, Magengeschwüren, Säurereflux, Pruritus und Sepsis.

## Revendications

1. Variant de diamine oxydase (DAO) humaine recombinée comprenant l'une quelconque des SEQ ID N° : 2 à 8 ou une séquence présentant une identité de séquence d'au moins 99 % avec l'une quelconque des SEQ ID N° : 2 à 8, dotée d'une affinité de liaison de glycosaminoglycane diminuée par rapport à la DAO humaine de type sauvage respective, dans lequel ladite DAO comprend au moins une modification d'acide aminé de l'un quelconque des acides amminés au niveau des positions 568 à 575 du domaine de liaison de glycosaminoglycane (GAG) par référence à la numérotation de la SEQ ID N° : 1.

2. Variant de DAO recombinée selon la revendication 1, comprenant en outre au moins une modification de cystéine accessible aux solvants au niveau de la position d'acide aminé 123 (cys123) par référence à la numérotation de la SEQ ID N° 1, en particulier la modification de la cystéine est une substitution, une délétion ou un couplage d'acide aminé avec un groupement chimique, plus particulièrement cys123 est substituée par de l'alanine.

3. Variant de DAO recombinée selon la revendication 1 ou 2, dans lequel le domaine de liaison de GAG est un domaine de liaison d'héparine/sulfate d'héparane.

4. Variant de DAO recombinée selon l'une quelconque des revendications 1 à 3, dans lequel la au moins une modification d'acide aminé dans le domaine de liaison de GAG est une substitution, une délétion, une insertion ou un couplage d'acide aminé avec un groupement chimique.

5. Variant de DAO recombinée selon l'une quelconque des revendications 1 à 4, comprenant 2, 3, 4, 5, 6, 7 ou 8 substitutions d'acide aminé dans le domaine de liaison de GAG.

6. Variant de DAO recombinée selon l'une quelconque des revendications 1 à 5, comprenant un domaine de liaison de GAG de la séquence d'acides aminés X1FX2X3X4LPX5, dans laquelle
X1 peut être un quelconque acide aminé, en particulier il représente A ou S, plus particulièrement il représente S ;
X2 peut être un quelconque acide aminé, en particulier il représente K ;
X3 peut être un quelconque acide aminé, en particulier il représente A ou T, plus particulièrement il représente T,
X4 peut être un quelconque acide aminé, en particulier il représente K, et
X5 peut être un quelconque acide aminé, en particulier il représente K ou T, plus particulièrement il représente T.

7. Variant de DAO recombinée selon l'une quelconque des revendications 1 à 6, comprenant la séquence d'acides aminés choisie dans le groupe constitué par SFKAKLPK (SEQ ID N° : 33), AFKAKLPT (SEQ ID N° : 34), AFKTKLPK (SEQ ID N° : 35), SFKTKLPK (SEQ ID N° 36), AFKTKLPT (SEQ ID N° : 37), SFKAKLPK (SEQ ID N° 38).

8. Variant de DAO recombinée selon l'une quelconque des revendications 1 à 7, comprenant en outre une substitution d'acide aminé au niveau de la position 168 par référence à la SEQ ID N° 1, en particulier Asn est remplacé par Gln.

9. Variant de DAO recombinée selon l'une quelconque des revendications 1 à 8, dans lequel ladite DAO présente une demi-vie plasmatique accrue par rapport à la DAO de type sauvage, en particulier ladite demi-vie est accrue d'un facteur d'au moins 1,5, en particulier d'un facteur d'au moins 2 par rapport à la DAO de type sauvage.

10. Variant de DAO recombinée selon l'une quelconque des revendications 1 à 9, dans lequel ladite DAO présente une aire sous la courbe (ASC) concentration plasmatique de médicament-temps accrue d'un facteur d'au moins 10 par rapport à la DAO de type sauvage.

11. Variant de DAO recombinée selon l'une quelconque des revendications 1 à 10, dans lequel l'internalisation par les cellules endothéliales est réduite d'au moins 10 %, 25 %, 50 %, 60 %, 70 %, 80 %, en particulier 90 % par rapport à la DAO de type sauvage.

12. Variant de DAO recombinée selon l'une quelconque des revendications 1 à 11, dans lequel l'affinité de liaison de GAG, en particulier l'affinité de liaison de l'héparine/sulfate d'héparane est réduite d'au moins 10 %, 25 %, 50 %, 60 %, 70 %, 80 %, en particulier 90 % par rapport à la DAO de type sauvage.

13. DAO recombinée selon l'une quelconque des revendications 2 à 12, comprenant les séquences d'acides aminés des SEQ ID N° : 9 à 16.

14. Polypeptide de fusion comprenant le variant de DAO recombinée selon l'une quelconque des revendications 1 à 12 et un domaine Fc d'IgG humaine ou de l'albumine de sérum humain (ASH), dans lequel le polypeptide de fusion conserve l'activité fonctionnelle de la DAO recombinée.

15. Séquence nucléotidique isolée codant le variant de DAO selon l'une quelconque des revendications 1 à 13, comprenant en particulier les séquences SEQ ID N° : 18 à 24 et 25 à 32.

16. Vecteur recombinant comprenant la séquence nucléotidique selon la revendication 15, en particulier le vecteur est un vecteur d'expression de bactérie, de levure, de baculovirus, de plante ou de mammifère.

17. Cassette d'expression comprenant la séquence nucléotidique selon la revendication 15, liée fonctionnellement à des éléments régulateurs.

18. Cellule hôte recombinée ou lignée de cellules hôtes comprenant le variant de DAO recombinée selon l'une quelconque des revendications 1 à 13, dans lesquelles les cellules hôtes sont choisies dans le groupe constitué par les cellules CHO, les cellules Vero, les cellules MDCK, les cellules de Pichia pastoris, les cellules SF9.

19. Système d'expression comprenant le vecteur selon la revendication 16 ou la cassette d'expression selon la revendication 17 et une cellule hôte ou une lignée de cellules hôtes selon la revendication 18.

20. Méthode de production du variant de DAO recombinée selon les revendications 1 à 12, ladite méthode comprenant les étapes de
i. clonage d'une séquence nucléotidique codant le variant de DAO selon l'une quelconque des revendications 1 à 13 dans un vecteur d'expression,
ii. transformation d'une cellule hôte avec ledit vecteur,
iii. culture de la cellule hôte transformée dans des conditions dans lesquelles le variant de DAO est exprimé,
iv. isolement du variant de DAO de la culture de cellules hôtes, éventuellement par désintégration des cellules hôtes, et éventuellement
v. purification du variant de DAO.

21. Composition pharmaceutique comprenant le variant de DAO recombinée selon l'une quelconque des revendications 1 à 13 et éventuellement un ou plusieurs excipients.

22. Variant de DAO recombinée selon l'une quelconque des revendications 1 à 13 destiné à être utilisé dans le traitement d'un état associé à un excès d'histamine, en particulier pour le traitement des maladies allergiques chroniques, plus particulièrement pour le traitement de l'anaphylaxie, d'un choc anaphylactique, de l'urticaire chronique, de l'urticaire aigue, de l'asthme, du rhume des foins, de la rhinite allergique, de la conjonctivite allergique, d'une intoxication par l'histamine, d'une céphalée, des maladies inflammatoires de dermatite atopique, de la mastocytose, du syndrome d'activation de mastocytes (MCAS), de la pré-éclampsie, de l'hyperémèse gravidique, du travail avant terme, des ulcères peptiques, du reflux acide, d'un prurit et de la septicémie.

23. Utilisation du variant de DAO recombinée selon l'une quelconque des revendications 1 à 13 pour la fabrication d'un médicament destiné au traitement d'un état associé un excès d'histamine, en particulier pour le traitement des maladies allergiques chroniques, plus particulièrement pour le traitement de l'anaphylaxie, d'un choc anaphylactique, de l'urticaire chronique, de l'urticaire aigue, de l'asthme, du rhume des foins, de la rhinite allergique, de la conjonctivite allergique, d'une intoxication par l'histamine, d'une céphalée, des maladies inflammatoires de dermatite atopique, de la mastocytose, des ulcères peptiques, du reflux acide, d'un prurit et de la septicémie.
